# EUROPEAN PATENT SPECIFICATION

(11) **EP 2 445 884 B1**
(45) Date of publication and mention of the grant of the patent: **13.02.2013**
(21) Application number: 10726558.9
(22) Date of filing: 21.06.2010
(51) Int. Cl.: C07D 213/79, C07D 401/12, C07D 407/12, C07D 409/12, A01N 43/40

(54) **4-AMINOPICOLINATES AND THEIR USE AS HERBICIDES**
4-AMINOPICOLINATE UND DEREN VERWENDUNG ALS HERBIZIDE
4-AMINOPICOLINATES ET UTILISATIONS DE CEUX-CI COMME HERBICIDES

(30) Priority: 22.06.2009 GB 0910766
(43) Date of publication of application: 02.05.2012
(73) Proprietor: Syngenta Limited, Guildford, Surrey GU2 7YH (GB)
(72) Inventor: WHITTINGHAM, William Guy, Berkshire RG42 6EY (GB); HACHISU, Shuji, Berkshire RG42 6EY (GB); HOTSON, Matthew Brian, Berkshire RG42 6EY (GB)
(74) Representative: Elliott, Kathryn
(86) International application number: PCT/GB2010/001213
(87) International publication number: WO 2010/149956

(56) References cited:
- WO-A1-01/51468
- WO-A1-2005/016887
- WO-A1-2006/062979

## Description

The present invention relates to certain substituted 4-aminopicolinate derivatives, to processes for their preparation, herbicidal compositions comprising them, and their use in controlling plants or inhibiting plant growth.

Herbicidal 4-aminopicolinates are disclosed in WO01/51468, WO03/011853, WO2004/089906, WO2005/016887 and WO2006/062979.

A problem that is unsolved is the provision of 4-aminopicolinates having enhanced selectivity compared with known compounds.

This invention seeks to provide compounds which solve this and other problems.

It has now been found that certain 4-amino-picolinic acid derivatives wherein the amine is substituted by a group comprising a 3 to 10 membered ring system display pre- and post- emergence herbicidal activity.

In a first aspect, the invention provides a compound having the formula (I): 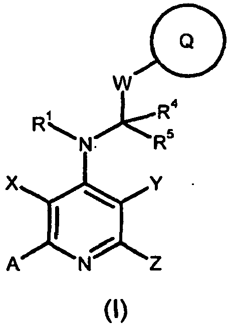 or a salt or N-oxide thereof,
wherein:
A is halogen, C1-C6 alkyl optionally substituted by 1 to 3 groups R², C1-C6 haloalkyl optionally substituted by 1 to 3 groups R², C2-C6 alkenyl optionally substituted by 1 to 3 groups R², C3-C8 cycloalkyl optionally substituted by 1 to 3 groups R², C1-C6 alkylthio optionally substituted by 1 to 3 groups R², C6-C10 aryl optionally substituted by 1 to 3 groups R³, a mono- or bicyclic heteroaryl group having 3 to 10 ring atoms and at least one ring atom which is nitrogen, oxygen or sulfur optionally substituted by 1 to 3 groups R³;
R¹ is hydrogen, C1-C6 alkyl optionally substituted by 1 to 3 groups R², C2-C6 alkenyl optionally substituted by 1 to 3 groups R², C2-C6 alkynyl optionally substituted by 1 to 3 groups R², C3-C8 cycloalkyl optionally substituted by 1 to 3 groups R², C1-C6 acyl optionally substituted by 1 to 3 groups R², C6-C10 aryl optionally substituted by 1 to 3 groups R³, a mono- or bicyclic heteroaryl group having 3 to 10 ring atoms and at least one ring atom which is nitrogen, oxygen or sulfur optionally substituted by 1 to 3 groups R³, C1-C6 alkylsulphonyl optionally substituted by 1 to 3 groups R², C2-C7 alkoxycarbonyl optionally substituted by 1 to 3 groups R², aminocarbonyl, C1-C6 alkylaminocarbonyl optionally substituted by 1 to 3 groups R², di C1-C6 alkylaminocarbonyl optionally substituted by 1 to 3 groups R²_{;}
R⁴ is hydrogen, cyano, nitro, C1-C6 alkyl optionally substituted by 1 to 3 groups R², C1-C6 haloalkyl optionally substituted by 1 to 3 groups R², C3-C6 cycloalkyl optionally substituted by 1 to 3 groups R², C2-C6 alkenyl optionally substituted by 1 to 3 groups R², C6-C10 aryl optionally substituted by 1 to 3 groups R³, a mono- or bicyclic heteroaryl group having 3 to 10 ring atoms and at least one ring atom which is nitrogen, oxygen or sulfur optionally substituted by 1 to 3 groups R³, C1-C6 acyl optionally substituted by 1 to 3 groups R², C1-C6 alkoxycarbonyl optionally substituted by 1 to 3 groups R², carboxy, aminocarbonyl, C1-C6 alkylaminocarbonyl optionally substituted by 1 to 3 groups R², di C1-C6 alkylaminocarbonyl optionally substituted by 1 to 3 groups R², or diC1-C6 alkylphosphonyl optionally substituted by 1 to 3 groups R²;
R⁵ is hydrogen, C1-C6 alkyl optionally substituted by 1 to 3 groups R², or C1-C6 haloalkyl;
W is a direct bond or a linker group of the formula -(C1-C3 alkylene)ₛ-L-(C1-C3 alkylene)ₜ- wherein each alkylene group is optionally substituted by up to 3 groups R², s and t may each be independently 0 or 1, and L is a direct single, double or triple bond, -S(O)ᵤ- wherein u is 0, 1 or 2, -N(R¹¹)- wherein R¹¹ is H or C1-C6 alkyl, -O- or -C(O)O-;
Q is a 3-10 membered ring system optionally containing up to 4 heteroatoms independently selected from O, N or S, the ring system optionally substituted by up to three substituents R³;
X is hydrogen, halogen, cyano, nitro, hydroxyl, C1-C6 alkyl optionally substituted by 1 to 3 groups R², C1-C6 haloalkyl optionally substituted by 1 to 3 groups R², C2-C6 alkenyl optionally substituted by 1 to 3 groups R², C2-C6 alkynyl optionally substituted by 1 to 3 groups R², C1-C6 haloalkoxy, C3-C8 cycloalkyl optionally substituted by 1 to 3 groups R², C6-C10 aryl optionally substituted by 1 to 3 groups R³, a mono- or bicyclic heteroaryl group having 3 to 10 ring atoms and at least one ring atom which is nitrogen, oxygen or sulfur optionally substituted by 1 to 3 groups R³, C1-C6 alkoxy optionally substituted by 1 to 3 groups R², amino, C1-C6 alkylamino optionally substituted by 1 to 3 groups R², di(C1-C6 alkyl)amino optionally substituted by 1 to 3 groups R², C1-C6 alkylthio optionally substituted by 1 to 3 groups R², C1-C6 alkylsulphinyl optionally substituted by 1 to 3 groups R², C1-C6 alkylsulphonyl optionally substituted by 1 to 3 groups R², di(C1-C6 alkyl) phosphonyl, tri(C1-C6 alkyl)silyl;
Y is halogen, cyano, nitro, hydroxyl, C1-C6 alkyl optionally substituted by 1 to 3 groups R², C2-C6 alkenyl optionally substituted by 1 to 3 groups R², C2-C6 alkynyl optionally substituted by 1 to 3 groups R², C1-C6 haloalkyl optionally substituted by 1 to 3 groups R², C1-C6 haloalkoxy, C1-C6 alkoxy optionally substituted by 1 to 3 groups R², C3-C8 cycloalkyl optionally substituted by 1 to 3 groups R², C3-C8 cycloalkoxy optionally substituted by 1 to 3 groups R², C6-C10 aryl optionally substituted by 1 to 3 groups R³, a mono- or bicyclic heteroaryl group having 3 to 10 ring atoms and at least one ring atom which is nitrogen, oxygen or sulfur optionally substituted by 1 to 3 groups R³, amino, C1-C6 alkylamino, di(C1-C6 alkyl)amino, C1-C6 alkylthio, C1-C6 alkylsulphinyl, C1-C6 alkylsulphonyl, di(C1-C6 alkyl) phosphonyl or tri(C1-C6 alkyl)silyl;
Z is C(O)R⁶, C(S)R⁶, or C(=NR⁷)R⁸;
each R² is independently halogen, hydroxyl, amino, C1-C3 alkylamino, di (C1-C3) alkylamino, carboxy, cyano, C1-C3 alkyl, C1-C3 haloalkyl, C3-C6 cycloalkyl, C1-C3 alkoxy, C1-C3 haloalkoxy, C1-C3 alkylthio, C1-C3 alkylsulphonyl, C2-C6 carboxyalkyl, C2-C6 alkoxycarbonyl, C2-C7 alkylcarbonyloxy, phenyl, or phenoxy;
each R³ is independently halogen, hydroxyl, nitro, amino, thiol, cyano, C1-C3 alkyl, C1-C3 haloalkyl, C1-C3 alkoxy, C1-C3 haloalkoxy, C1-C3 alkylthio, C1-C3 haloalkylthio, C2-C6 carboxyalkyl, C2-C6 alkoxycarbonyl, C2-C7 alkylcarbonyloxy, phenyl, phenoxy, C1-C3 alkylamino, or di(C1-C3 alkyl)amino;
R⁶ is hydrogen, hydroxyl, C1-C10 alkoxy optionally substituted by C1-C6 alkoxy or phenyl, C3-C8 cycloalkoxy optionally substituted by C1-C6 alkoxy or phenyl, C1-C6 haloalkoxy, C2-C6 alkenyloxy, C1-C6 alkylthio, amino, C1-C6 alkylamino, or di(C1-C6 alkyl)amino;
R⁷ is hydrogen, C1-C6 alkyl, C1-C6 alkoxy, C3-C8 cycloalkoxy, amino, C1-C6 alkylamino, or di(C1-C6 alkyl)amino;
R⁸ is hydrogen, C1-C6 alkoxy, C3-C8 cycloalkoxy, C1-C6 alkylthio, amino, C1-C6 alkylamino, or di(C1-C6 alkyl)amino.

In a second aspect, the invention relates to a herbicidal composition comprising a compound of formula (I) as defined above together with at least one agriculturally acceptable adjuvant or diluent.

In a third aspect, the invention relates to the use of a compound of formula (I) as defined above or composition as defined above as a herbicide.

In a fourth aspect, the invention relates to a method of controlling weeds in crops of useful plants, comprising applying to said weeds or to the locus of said weeds, or to said useful crop plants, a compound of formula (I) as defined above or composition as defined above.

In a fifth aspect, the invention relates to a process for the preparation of compounds of formula (I).

In a sixth aspect, the invention relates to intermediates useful in the preparation of compounds of formula (I).

### Tautomers

The compounds of formula (I) may exist as different geometric isomers, or in different tautomeric forms. This invention covers all such isomers and tautomers, and mixtures thereof in all proportions, as well as isotopic forms such as deuterated compounds. Zwitterionic forms are also covered. For example, compounds of formula (II) may exist in equilibrium with the zwitterionic forms (III) and (IV).

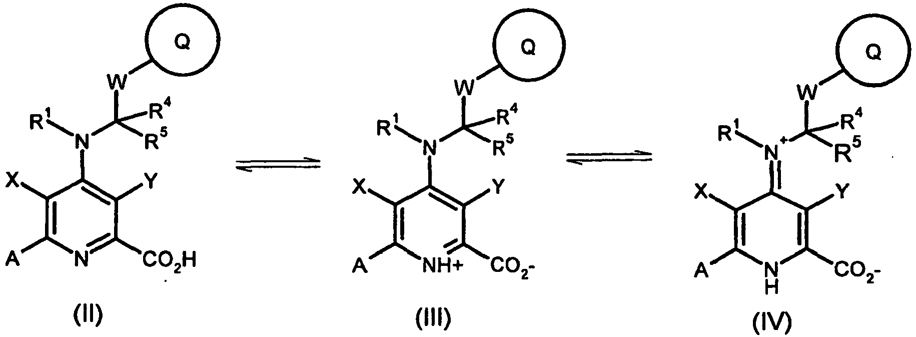

### Asymmetry

The compounds of this invention may contain an asymmetric carbon atom and some of the compounds of this invention may contain one or more asymmetric centers and may thus give rise to optical isomers and diastereomers. While shown without respect to stereochemistry, the present invention includes such optical isomers and diastereomers; as well as the racemic and resolved, enantiomerically pure R and S stereoisomers; as well as other mixtures of the R and S stereoisomers and agrochemically acceptable salts thereof. It is recognized that one optical isomer, including diastereomer and enantiomer, or stereoisomer may have favorable properties over the other. Thus when disclosing and claiming the invention, when one racemic mixture is disclosed, it is clearly contemplated that both optical isomers, including diastereomers and enantiomers, or stereoisomers substantially free of the other are disclosed and claimed as well.

### Alkyl

Alkyl, as used herein refers to an aliphatic hydrocarbon chain and includes straight and branched chains e. g. of 1 to 6 carbon atoms such as methyl, ethyl, n-propyl, isopropyl, n-butyl, isobutyl, sec-butyl, t-butyl, n-pentyl, isopentyl, neopentyl, n-hexyl, and isohexyl.

### Alkenyl

Alkenyl, as used herein, refers to an aliphatic hydrocarbon chain having at least one double bond, and preferably one double bond, and includes straight and branched chains e. g. of 2 to 6 carbon atoms such as ethenyl, propenyl, isopropenyl, but-1-enyl, but-2-enyl, but-3-enyl, 2-methypropenyl,

### Alkynyl

Alkynyl, as used herein, refers to an aliphatic hydrocarbon chain having at least one triple bond, and preferably one triple bond, and includes straight and branched chains e. g. of 2 to 6 carbon atoms such as ethynyl, propynyl, but-1-ynyl, but-2-ynyl and but-3-ynyl.

### Cycloalkyl

Cycloalkyl, as used herein, refers to a cyclic, saturated hydrocarbon group having from 3 to 8 ring carbon atoms. Examples of cycloalkyl groups are cyclopropyl, cyclobutyl, cyclopentyl, cyclohexyl, cycloheptyl and cyclooctyl.

### Alkoxy

Alkoxy as used herein refers to the group -O-alkyl, wherein alkyl is as defined above. Examples of alkoxy groups include methoxy, ethoxy, n-propoxy, isopropoxy, n-butoxy, isobutoxy, sec-butoxy, t-butoxy, n-pentoxy, isopentoxy, neo-pentoxy, n-hexyloxy, and isohexyloxy.

### Cycloalkoxy

Cycloalkoxy as used herein refers to the group -O-cycloalkyl, wherein cycloalkyl is as defined above. Examples of cycloalkoxy groups are cyclopropoxy, cyclobutoxy, cyclopentoxy, cyclohexyloxy, cycloheptyloxy and cyclooctyloxy.

### Alkylthio

Alkylthio as used herein refers to the group -S-alkyl, wherein alkyl is as defined above. Examples of thioalkyl groups are methylthio, ethylthio, n-propylthio, isopropylthio, n-butylthio, isobutylthio, sec-butylthio, t-butylthio, n-pentylthio, isopentylthio, neo-pentylthio, n-hexylthio, and isohexylthio.

### Alkylsulphinyl

Alkylsulphinyl refers to the group -S(O)-alkyl, wherein alkyl is as defined above.

### Alkylsulphonyl

Alkylsulphonyl refers to the group -S(O)₂-alkyl, wherein alkyl is as defined above.

### Halogen

Halogen, halide and halo refer to iodine, bromine, chlorine and fluorine.

### Haloalkyl

Haloalkyl as used herein refers to an alkyl group as defined above wherein at least one hydrogen atom has been replaced with a halogen atom as defined above. Examples of haloalkyl groups include chloromethyl, dichloromethyl, trichloromethyl, fluoromethyl, difluoromethyl and trifluoromethyl. Preferred haloalkyl groups are fluoroalkyl groups (i.e. haloalkyl groups, containing fluorine as the only halogen). More highly preferred haloalkyl groups are perfluoroalkyl groups, (i.e. alkyl groups wherein all the hydrogen atoms are replaced with fluorine atoms).

### Haloalkoxy

Haloalkoxy as used herein refers to an alkoxy group as defined above wherein at least one hydrogen atom has been replaced with a halogen atom as defined above.

### Acyl

As used herein, the term "acyl" refers to the group -C(O)-alkyl or -C(O)H, wherein the alkyl group is as defined above. Examples of acyl groups are formyl, acetyl, pivaloyl etc.

### Alkoxycarbonyl

As used herein, the term "alkoxycarbonyl" refers to the group -C(O)-O-alkyl, wherein the alkyl group is as defined above. Examples of alkoxycarbonyl groups include methoxycarbonyl, ethoxycarbonyl, i-propoxycarbonyl, n-propoxycarbonyl, n-butoxycarbonyl and s-butoxycarbonyl etc.

### Aminocarbonyl

As used herein, the term "aminocarbonyl" refers to the group -C(O)NH₂. Alkylamino

Alkylamino refers to the group -NH-alkyl, wherein alkyl is as defined above. Examples of alkylamino groups are methylamino, ethylamino, n-propylamino, i-propylamino etc.

### Alkylaminocarbonyl

As used herein, the term "alkylaminocarbonyl" refers to the group -C(O)NH-alkyl, wherein alkyl is as defined above.

### Dialkylamino

Dialkylamino refers to the group -N(alkyl)alkyl', wherein alkyl and alkyl' are both alkyl groups as defined above which may be the same or different. Examples of dialkylamino groups are dimethylamino, diethylamino, di-n-propylamino, methylethylamino, methyisopropylamino, etc.

### Dialkylaminocarbonyl

As used herein, the term "dialkylaminocarbonyl" refers to the group - C(O)N(alkyl)alkyl', wherein alkyl and alkyl' are both alkyl groups as defined above which may be the same or different.

### Dialkylphosphonyl

Dialkylphosphonyl refers to the group -P(O)(alkyl)(alkyl'), wherein alkyl and alkyl' are both alkyl groups as defined above which may be the same or different. Examples of dialkylphosphonyl groups are dimethylphosphonyl, diethylphosphonyl, ethyl methyl phosphonyl etc.

### Alkylcarbonyloxy

Alkylcarbonyloxy refers to the group -OC(O)-alkyl wherein alkyl is as defined above.

### Carboxyalkyl

Carboxyalkyl refers to the group -alkyl-C(O)OH, wherein alkyl is as defined above.

### Alkylene

The term "alkylene" is used as a branched or linear divalent hydrocarbon radical. Examples of alkylene are methylene, 1,1-ethylene, 1,2-ethylene, 1,1-propylene, 1,2-propylene, 1,3-propylene and 2,2-propylene etc.

### Aryl

As used herein, "aryl" refers to an unsaturated aromatic carbocyclic group of from 6 to 10 carbon atoms having a single ring (e. g., phenyl) or multiple condensed (fused) rings, at least one of which is aromatic (e.g., indanyl, naphthyl). Preferred aryl groups include phenyl, naphthyl and the like.

### Aryloxy

Aryloxy refers to the group -O-aryl, wherein aryl is as defined above. Preferred aryloxy groups include phenoxy, naphthyloxy and the like.

### Heteroaryl

The term "heteroaryl" refers to a ring system containing 3 to 10 ring atoms, and preferably 5 to 10 ring atoms, at least one ring heteroatom and consisting either of a single aromatic ring or of two or more fused rings, at least one of which is aromatic. Preferably, single rings will contain up to three and bicyclic systems up to four heteroatoms which will preferably be chosen from nitrogen, oxygen and sulfur. Examples of such groups include pyridyl, pyridazinyl, pyrimidinyl, pyrazinyl, triazinyl, furanyl, thiophenyl, oxazolyl, isoxazolyl, oxadiazolyl, thiazolyl, isothiazolyl, thiadiazolyl, pyrrolyl, pyrazolyl, imidazolyl, triazolyl and tetrazolyl. Examples of bicyclic groups are benzothiophenyl, benzimidazolyl, benzothiadiazolyl, quinolinyl, cinnolinyl, quinoxalinyl and pyrazolo[1,5-a]pyrimidinyl.

### Heteroaryloxy

The term "heteroaryloxy" refers to the group -O-heteroaryl, wherein heteroaryl is as defined above.

### Heterocyclyl

The term "heterocyclyl" refers to a non-aromatic ring system containing 3 to 10 ring atoms, at least one ring heteroatom and consisting either of a single ring or of two or more fused rings. Preferably, single rings will contain up to three and bicyclic systems up to four heteroatoms which will preferably be chosen from nitrogen, oxygen and sulfur. Examples of such groups include pyrrolidinyl, imidazolinyl, pyrazolidinyl, piperidyl, piperazinyl, quinuclidinyl, morpholinyl, together with unsaturated or partially unsaturated analogues such as 4,5,6,7-tetrahydro-benzothiophenyl, chromen-4-onyl, 9H-fluorenyl, 3,4-dihydro-2H-benzo-1,4-dioxepinyl, 2,3-dihydro-benzofuranyl, piperidinyl, 1,3-dioxolanyl, 1,3-dioxanyl, 4,5-dihydro-isoxazolyl, tetrahydrofuranyl and morpholinyl.

It is noted that, when the number of carbon atoms is specified for alkoxycarbonyl, carboxyalkyl and alkylcarbonyloxy groups, this number may include both the carbon atom associated with the carbonyl or carboxy group as well as the carbon atoms associated with the alkyl or alkoxy groups.

### Optional substitution

"Optionally substituted" as used herein means the group referred to can be substituted at one or more positions by any one or any combination of the radicals listed thereafter. For most groups, one or more hydrogen atoms are replaced by the radicals listed thereafter. For halogenated groups, for example, haloalkyl groups, one or more halogen atoms are replaced by the radicals listed thereafter.

### Salts

Suitable salts include those derived from alkali or alkaline earth metals and those derived from ammonia and amines. Preferred cations include sodium, potassium, magnesium, and ammonium cations of the formula N⁺(R⁹R¹⁰R¹¹R¹²) wherein R⁹, R¹⁰, R¹¹ and R¹² are independently selected from hydrogen, C1-C6 alkyl and C1-C6 hydroxyalkyl. Salts of the compounds of Formula I can be prepared by treatment of compounds of Formula I with a metal hydroxide, such as sodium hydroxide, or an amine, such as ammonia, trimethylamine, diethanolamine, 2-methylthiopropylamine, bisallylamine, 2-butoxyethylamine, morpholine, cyclododecylamine, or benzylamine. Amine salts are often preferred forms of the compounds of Formula I because they are water-soluble and lend themselves to the preparation of desirable aqueous based herbicidal compositions.

Acceptable salts can be formed from organic and inorganic acids, for example, acetic, propionic, lactic, citric, tartaric, succinic, fumaric, maleic, malonic, mandelic, malic, phthalic, hydrochloric, hydrobromic, phosphoric, nitric, sulfuric, methanesulfonic, naphthalenesulfonic, benzenesulfonic, toluenesulfonic, camphorsulfonic, and similarly known acceptable acids when a compound of this invention contains a basic moiety.

Preferred values of A, X, Y, Z, R¹ to R⁸, W and Q are set out below.

A is preferably halogen, C1-C6 alkyl, C1-C6 haloalkyl, C2-C6 alkenyl, C3-C8 cycloalkyl optionally substituted by 1 to 3 groups R², C6-C10 aryl optionally substituted by 1 to 3 groups R³, or a mono- or bicyclic heteroaryl group having 3 to 10 ring atoms and at least one ring atom which is nitrogen, oxygen or sulfur optionally substituted by 1 to 3 groups R³.

Examples of group A include 2,3,4-trichlorophenyl, 2,4-dichloro-3-fluorophenyl, 2,4-dichlorophenyl, 2-chloro-4-methylphenyl, 3,4-dichloro-2-fluorophenyl, 3,4-dichlorophenyl, 4,5-dichloro-2-fluorophenyl, 4-bromophenyl, 4-chloro-2,3-difluorophenyl, 4-chloro-2,5-difluorophenyl, 4-chloro-2-fluoro-3-methoxyphenyl, 4-chloro-2-fluorophenyl, 4-chloro-3-dimethylamino-2-fluorophenyl, 4-chloro-3-fluorophenyl, 4-chlorophenyl, 4-methylphenyl, 4-trifluoromethylphenyl, 5-chlorothiophen-2-yl, 6-chloropyridin-3-yl, chloro, cyclopropyl and isopropyl.

More preferably, A is halogen, phenyl optionally substituted by 1 to 3 groups R³, or C3-C6 cycloalkyl optionally substituted by 1 to 3 groups R².

Very preferably, A is chlorine. In an alternative, very preferred embodiment, A is unsubstituted cyclopropyl.

In an alternative, very preferred embodiment, A is trisubstituted phenyl, wherein the substituents are independently R³. More preferably, A is 2,3,4-trisubstituted phenyl, wherein the substituents are independently R³. More preferably, A is 4-chloro-2-fluoro-3-methoxyphenyl, or 4-chloro-3-dimethylamino-2-fluorophenyl.

Preferably, R¹ is hydrogen, C1-C6 alkyl optionally substituted by 1 or 2 groups R³, or C2-C6 alkenyl.

Examples of R¹ are hydrogen, methyl, ethyl and isopropyl.

More preferably, R¹ is hydrogen or C1-C6 alkyl. Most preferably, R¹ is hydrogen.

Preferably, X is hydrogen, halogen, C1-C6 alkyl, C1-C6 haloalkyl, C10C3 alkoxy(C1-C6)alkyl or C3-C6 cycloalkyl.

More preferably, X is hydrogen, halogen, C1-C2 alkyl, C1-C2 haloalkyl, C1-C2 alkoxy(C1-C2)alkyl, or C3-C6 cycloalkyl.

Examples of X are hydrogen, fluoro, chloro, bromo, methyl and cyclopropyl.

Most preferably, X is hydrogen, fluoro or chloro.

Preferably, Y is halogen, C1-C3 alkyl, C1-C3 haloalkyl, C1-C2-alkoxy(C1-C2) alkyl, cyclylpropyl, C2-C4 alkenyl or C2-C4 alkynyl.

More preferably, Y is halogen, C1-C2 alkyl, C1-C2 haloalkyl, C1-C2 alkoxy(C1-C2)alkyl, cyclopropyl, C2-C4 alkenyl or C2-C4 alkynyl.

Examples of Y are chlorine, bromine, methyl, vinyl, cyclopropyl and 1-propenyl.

More preferably, Y is chlorine, vinyl or cyclopropyl. Most preferably, Y is chlorine or vinyl.

Preferably, Z is C(O)R⁶, wherein R⁶ is hydroxyl, C1-C6 alkoxy, phenyl(C1-C2)alkoxy, or (C1-C3)alkoxy(C1-C6)alkoxy.

Examples of group Z are CO₂CH₂CH₂OEt, CO₂CH₂Ph, CO₂Et, CO₂Me and CO₂H.

More preferably, Z is C(O)R⁶ wherein R⁶ is hydroxyl, C1-C6 alkoxy or phenyl(C1-C2)alkoxy. More preferably, Z is CO₂H or CO₂Me.

Preferably, R⁴ is hydrogen, C1-C6 alkyl, C1-C6 haloalkyl, C1-C6 hydroxyalkyl, C1-C3 alkoxy(C1-C6)alkyl, C2-C6 alkoxycarbonyl or carboxyl. Most preferably, R⁴ is hydrogen.

Examples of R⁴ are hydrogen, methyl, CH₂OH, CO₂Et CO₂Me, CO₂H.

Preferably, R⁵ is hydrogen or C1-C6 alkyl. More preferably, R⁵ is hydrogen.

An example of R⁵ is hydrogen.

Preferably, W is a direct bond or a methylene group. More preferably, W is a direct bond.

Examples of W are CH(OH), -CH(OH)CH₂O-, CH₂, and a direct bond.

In one preferred embodiment, Q is C6-C10 aryl optionally substituted by 1 to 3 groups independently selected from R³. More preferably, Q is phenyl or napthyl optionally substituted by 1 to 3 groups independently selected from R³. More preferably, Q is phenyl optionally substituted by 1 or 2 groups independently selected from R³.

In another preferred embodiment, Q is a heteroaryl ring system containing 3 to 10 ring atoms, with up to four ring heteroatoms independently selected from N, O or S, consisting either of a single aromatic ring or of two or more fused rings, at least one of which is aromatic, optionally substituted by 1 to 3 groups independently selected from R³. Preferably, Q is a monocyclic heteroaryl ring with 5 to 7 ring members comprising 1 to 3 ring heteroatoms independently selected from N, O or S optionally substituted by 1 to 3 groups independently selected from R³. More preferably, Q is a monocyclic heteroaryl ring with 5 or 6 ring members comprising 1 to 3 ring heteroatoms independently selected from N, O or S optionally substituted by 1 to 3 groups independently selected from R³. More preferably, Q is pyridyl, furyl, thiophenyl, oxazolyl, or thiazolyl, optionally substituted by 1-2 groups R³. More preferably Q is furyl optionally substituted by 1-2 groups R³. More preferably, Q is unsubstituted furyl. More preferably, Q is 2-furyl optionally substituted by 1-2 groups R³. More preferably, Q is unsubstituted 2-furyl.

In another preferred embodiment, Q is C3-C8 cycloalkyl optionally substituted by 1 or 2 groups R³.

Examples of Q are phenyl, naphthyl, pyridyl, pyrimidinyl, furanyl, benzofuranyl, thiophenyl, benzothiophenyl, pyrrolyl, indolyl, oxazolyl, oxadiazolyl, benzoxazolyl, pyrazolyl, thiazolyl, pyrrolidinyl, 1,3-dioxolanyl, morpholinyl, imidazolidinyl, 3,4-dihydro-2H-pyran-2-yl, benzothiazolyl, benzofuranyl, indanyl, tetrahydropyranyl, tetrahydrofuranyl, benzo[1,3]dioxolyl each optionally substituted by 1 or 2 groups R³, C3-8 cycloalkyl optionally substituted by 1 or 2 groups R³, C3-8 cycloalkenyl optionally substituted by 1 or 2 groups R³;
wherein
each R³ is independently halogen, hydroxyl, nitro, cyano, C1-C2 alkyl, C1-C2 haloalkyl, C1-C2 alkoxy, C1-C2 haloalkoxy, C1-C3 alkoxycarbonyl, amino or di(C1-C2alkyl)amino.

R³ is preferably halogen, hydroxyl, nitro, C1-C2 alkyl, C1-C2 alkoxy, di(C1-C2)alkylamino.

In a preferred embodiment, A is halogen, R¹ is hydrogen, X is hydrogen or halogen, Y is halogen, methyl or vinyl, Z is C(O)R⁶, wherein R⁶ is hydroxyl or C1-C6 alkoxy, R⁴ and R⁵ are both hydrogen, W is a direct bond and Q is a phenyl,furanyl, pyridyl or cyclopropyl ring optionally substituted by up to three substituents R³, wherein each R³ is independently halogen, hydroxyl, nitro, amino, C1-C3 alkyl, C1-C3 haloalkyl, C1-C3 alkoxy, C1-C3 haloalkoxy, C2-C6 alkoxycarbonyl, C2-C7 alkylcarbonyloxy, C1-C3 alkylamino, or di(C1-C3 alkyl)amino.

The compounds described below are illustrative of novel compounds of the invention. Table 1 below provides 467 compounds designated compounds 1-1 to 1-467 respectively, of formula (I) wherein R¹ is H, R⁴ is H, R⁵ is H, W is a direct bond and Q is cyclopropyl.

**TABLE 1**

| Compound Number | Substituent Values | | | |
|---|---|---|---|---|
| | A | X | Y | Z |
| 1-1 | Cl | H | Cl | CO2H |
| 1-2 | Cl | H | Cl | CO2Me |
| 1-3 | Cl | H | Cl | CO2Et |
| 1-4 | Cl | H | Cl | CO2CH2CHOEt |
| 1-5 | Cl | H | Cl | CO2CH2Ph |
| 1-6 | Cl | H | cyclopropyl | CO2H |
| 1-7 | Cl | H | cyclopropyl | CO2Me |
| 1-8 | Cl | H | cyclopropyl | CO2Et |
| 1-9 | Cl | H | cyclopropyl | CO2CH2CH2OEt |
| 1-10 | Cl | H | cyclopropyl | CO2CH2Ph |
| 1-11 | Cl | H | vinyl | CO2H |
| 1-12 | Cl | H | vinyl | CO2Me |
| 1-13 | Cl | H | vinyl | CO2Et |
| 1-14 | Cl | H | vinyl | CO2CH2CH2OEt |
| 1-15 | Cl | H | vinyl | CO2CH2Ph |
| 1-16 | Cl | H | Br | CO2H |
| 1-17 | Cl | H | Br | CO2Me |
| 1-18 | Cl | H | Me | CO2H |
| 1-19 | Cl | H | Me | CO2Me |
| 1-20 | Cl | H | 1-propenyl | CO2H |
| 1-21 | Cl | H | 1-propenyl | CO2Me |
| 1-22 | Cl | F | Cl | CO2H |
| 1-23 | Cl | F | Cl | CO2Me |
| 1-24 | Cl | F | cyclopropyl | CO2H |
| 1-25 | Cl | F | cyclopropyl | CO2Me |
| 1-26 | Cl | F | vinyl | CO2H |
| 1-27 | Cl | F | vinyl | CO2Me |
| 1-28 | Cl | F | Br | CO2H |
| 1-29 | Cl | F | Br | CO2Me |
| 1-30 | Cl | F | Me | CO2H |
| 1-31 | Cl | F | Me | CO2Me |
| 1-32 | Cl | F | 1-propenyl | CO2H |
| 1-33 | Cl | F | 1-propenyl | CO2Me |
| 1-34 | Cl | Cl | Cl | CO2H |
| 1-35 | Cl | Cl | Cl | CO2Me |
| 1-36 | Cl | Cl | cyclopropyl | CO2H |
| 1-37 | Cl | Cl | cyclopropyl | CO2Me |
| 1-38 | Cl | Cl | vinyl | CO2H |
| 1-39 | Cl | Cl | vinyl | CO2Me |
| 1-40 | Cl | Cl | Br | CO2H |
| 1-41 | Cl | Cl | Br | CO2Me |
| 1-42 | Cl | Cl | Me | CO2H |
| 1-43 | Cl | Cl | Me | CO2Me |
| 1-44 | Cl | Cl | 1-propenyl | CO2H |
| 1-45 | Cl | Cl | 1-propenyl | CO2Me |
| 1-46 | Cl | Br | Cl | CO2H |
| 1-47 | Cl | Br | Cl | CO2Me |
| 1-48 | Cl | Br | cyclopropyl | CO2H |
| 1-49 | Cl | Br | cyclopropyl | CO2Me |
| 1-50 | Cl | Br | vinyl | CO2H |
| 1-51 | Cl | Br | vinyl | CO2Me |
| 1-52 | Cl | Br | Br | CO2H |
| 1-53 | Cl | Br | Br | CO2Me |
| 1-54 | Cl | Br | Me | CO2H |
| 1-55 | Cl | Br | Me | CO2Me |
| 1-56 | Cl | Br | 1-propenyl | CO2H |
| 1-57 | Cl | Br | 1-propenyl | CO2Me |
| 1-58 | Cl | Me | Cl | CO2H |
| 1-59 | Cl | Me | Cl | CO2Me |
| 1-60 | Cl | Me | cyclopropyl | CO2H |
| 1-61 | Cl | Me | cyclopropyl | CO2Me |
| 1-62 | Cl | Me | vinyl | CO2H |
| 1-63 | Cl | Me | vinyl | CO2Me |
| 1-64 | Cl | Me | Br | CO2H |
| 1-65 | Cl | Me | Br | CO2Me |
| 1-66 | Cl | Me | Me | CO2H |
| 1-67 | Cl | Me | Me | CO2Me |
| 1-68 | Cl | Me | 1-propenyl | CO2H |
| 1-69 | Cl | Me | 1-propenyl | CO2Me |
| 1-70 | Cl | cyclopropyl | Cl | CO2H |
| 1-71 | Cl | cyclopropyl | Cl | CO2Me |
| 1-72 | Cl | cyclopropyl | cyclopropyl | CO2H |
| 1-73 | Cl | cyclopropyl | cyclopropyl | CO2Me |
| 1-74 | Cl | cyclopropyl | vinyl | CO2H |
| 1-75 | Cl | cyclopropyl | vinyl | CO2Me |
| 1-76 | Cl | cyclopropyl | Br | CO2H |
| 1-77 | Cl | cyclopropyl | Br | CO2Me |
| 1-78 | Cl | cyclopropyl | Me | CO2H |
| 1-79 | Cl | cyclopropyl | Me | CO2Me |
| 1-80 | Cl | cyclopropyl | 1-propenyl | CO2H |
| 1-81 | Cl | cyclopropyl | 1-propenyl | CO2Me |
| 1-82 | cyclopropyl | H | Cl | CO2H |
| 1-83 | cyclopropyl | H | Cl | CO2Me |
| 1-84 | cyclopropyl | H | Cl | CO2Et |
| 1-85 | cyclopropyl | H | Cl | CO2CH2CH2OEt |
| 1-86 | cyclopropyl | H | Cl | CO2CH2Ph |
| 1-87 | cyclopropyl | H | cyclopropyl | CO2H |
| 1-88 | cyclopropyl | H | cyclopropyl | CO2Me |
| 1-89 | cyclopropyl | H | cyclopropyl | CO2Et |
| 1-90 | cyclopropyl | H | cyclopropyl | CO2CH2CH2OEt |
| 1-91 | cyclopropyl | H | cyclopropyl | CO2CH2Ph |
| 1-92 | cyclopropyl | H | vinyl | CO2H |
| 1-93 | cyclopropyl | H | vinyl | CO2Me |
| 1-94 | cyclopropyl | H | vinyl | CO2Et |
| 1-95 | cyclopropyl | H | vinyl | CO2CH2CH2OEt |
| 1-96 | cyclopropyl | H | vinyl | CO2CH2Ph |
| 1-97 | cyclopropyl | H | Br | CO2H |
| 1-98 | cyclopropyl | H | Br | CO2Me |
| 1-99 | cyclopropyl | H | Me | CO2H |
| 1-100 | cyclopropyl | H | Me | CO2Me |
| 1-101 | cyclopropyl | H | 1-propenyl | CO2H |
| 1-102 | cyclopropyl | H | 1-propenyl | CO2Me |
| 1-103 | cyclopropyl | F | Cl | CO2H |
| 1-104 | cyclopropyl | F | Cl | CO2Me |
| 1-105 | cyclopropyl | F | cyclopropyl | CO2H |
| 1-106 | cyclopropyl | F | cyclopropyl | CO2Me |
| 1-107 | cyclopropyl | F | vinyl | CO2H |
| 1-108 | cyclopropyl | F | vinyl | CO2Me |
| 1-109 | cyclopropyl | F | Br | CO2H |
| 1-110 | cyclopropyl | F | Br | CO2Me |
| 1-111 | cyclopropyl | F | Me | CO2H |
| 1-112 | cyclopropyl | F | Me | CO2Me |
| 1-113 | cyclopropyl | F | 1-propenyl | CO2H |
| 1-114 | cyclopropyl | F | 1-propenyl | CO2Me |
| 1-115 | cyclopropyl | Cl | Cl | CO2H |
| 1-116 | cyclopropyl | Cl | Cl | CO2Me |
| 1-117 | cyclopropyl | Cl | cyclopropyl | CO2H |
| 1-118 | cyclopropyl | Cl | cyclopropyl | CO2Me |
| 1-119 | cyclopropyl | Cl | vinyl | CO2H |
| 1-120 | cyclopropyl | Cl | vinyl | CO2Me |
| 1-121 | cyclopropyl | Cl | Br | CO2H |
| 1-122 | cyclopropyl | Cl | Br | CO2Me |
| 1-123 | cyclopropyl | Cl | Me | CO2H |
| 1-124 | cyclopropyl | Cl | Me | CO2Me |
| 1-125 | cyclopropyl | Cl | 1-propenyl | CO2H |
| 1-126 | cyclopropyl | Cl | 1-propenyl | CO2Me |
| 1-127 | cyclopropyl | Br | Cl | CO2H |
| 1-128 | cyclopropyl | Br | Cl | CO2Me |
| 1-129 | cyclopropyl | Br | cyclopropyl | CO2H |
| 1-130 | cyclopropyl | Br | cyclopropyl | CO2Me |
| 1-131 | cyclopropyl | Br | vinyl | CO2H |
| 1-132 | cyclopropyl | Br | vinyl | CO2Me |
| 1-133 | cyclopropyl | Br | Br | CO2H |
| 1-134 | cyclopropyl | Br | Br | CO2Me |
| 1-135 | cyclopropyl | Br | Me | CO2H |
| 1-136 | cyclopropyl | Br | Me | CO2Me |
| 1-137 | cyclopropyl | Br | 1-propenyl | CO2H |
| 1-138 | cyclopropyl | Br | 1-propenyl | CO2Me |
| 1-139 | cyclopropyl | Me | Cl | CO2H |
| 1-140 | cyclopropyl | Me | Cl | CO2Me |
| 1-141 | cyclopropyl | Me | cyclopropyl | CO2H |
| 1-142 | cyclopropyl | Me | cyclopropyl | CO2Me |
| 1-143 | cyclopropyl | Me | vinyl | CO2H |
| 1-144 | cyclopropyl | Me | vinyl | CO2Me |
| 1-145 | cyclopropyl | Me | Br | CO2H |
| 1-146 | cyclopropyl | Me | Br | CO2Me |
| 1-147 | cyclopropyl | Me | Me | CO2H |
| 1-148 | cyclopropyl | Me | Me | CO2Me |
| 1-149 | cyclopropyl | Me | 1-propenyl | CO2H |
| 1-150 | cyclopropyl | Me | 1-propenyl | CO2Me |
| 1-151 | cyclopropyl | cyclopropyl | Cl | CO2H |
| 1-152 | cyclopropyl | cyclopropyl | Cl | CO2Me |
| 1-153 | cyclopropyl | cyclopropyl | cyclopropyl | CO2H |
| 1-154 | cyclopropyl | cyclopropyl | cyclopropyl | CO2Me |
| 1-155 | cyclopropyl | cyclopropyl | vinyl | CO2H |
| 1-156 | cyclopropyl | cyclopropyl | vinyl | CO2Me |
| 1-157 | cyclopropyl | cyclopropyl | Br | CO2H |
| 1-158 | cyclopropyl | cyclopropyl | Br | CO2Me |
| 1-159 | cyclopropyl | cyclopropyl | Me | CO2H |
| 1-160 | cyclopropyl | cyclopropyl | Me | CO2Me |
| 1-161 | cyclopropyl | cyclopropyl | 1-propenyl | CO2H |
| 1-162 | cyclopropyl | cyclopropyl | 1-propenyl | CO2Me |
| 1-163 | 4-chloro-2-fluoro-3-methoxyphenyl | H | Cl | CO2H |
| 1-164 | 4-chloro-2-fluoro-3-methoxyphenyl | H | Cl | CO2Me |
| 1-165 | 4-chloro-2-fluoro-3-methoxyphenyl | H | Cl | CO2Et |
| 1-166 | 4-chloro-2-fluoro-3-methoxyphenyl | H | Cl | CO2CH2CH2OEt |
| 1-167 | 4-chloro-2-fluoro-3-methoxyphenyl | H | Cl | CO2CH2Ph |
| 1-168 | 4-chloro-2-fluoro-3-methoxyphenyl | H | cyclopropyl | CO2H |
| 1-169 | 4-chloro-2-fluoro-3-methoxyphenyl | H | cyclopropyl | CO2Me |
| 1-170 | 4-chloro-2-fluoro-3-methoxyphenyl | H | cyclopropyl | CO2Et |
| 1-171 | 4-chloro-2-fluoro-3-methoxyphenyl | H | cyclopropyl | CO2CH2CH2OEt |
| 1-172 | 4-chloro-2-fluoro-3-methoxyphenyl | H | cyclopropyl | CO2CH2Ph |
| 1-173 | 4-chloro-2-fluoro-3-methoxyphenyl | H | vinyl | CO2H |
| 1-174 | 4-chloro-2-fluoro-3-methoxyphenyl | H | vinyl | CO2Me |
| 1-175 | 4-chloro-2-fluoro-3-methoxyphenyl | H | vinyl | CO2Et |
| 1-176 | 4-chloro-2-fluoro-3-methoxyphenyl | H | vinyl | CO2CH2CH2OEt |
| 1-177 | 4-chloro-2-fluoro-3-methoxyphenyl | H | vinyl | CO2CH2Ph |
| 1-178 | 4-chloro-2-fluoro-3-methoxyphenyl | H | Br | CO2H |
| 1-179 | 4-chloro-2-fluoro-3-methoxyphenyl | H | Br | CO2Me |
| 1-180 | 4-chloro-2-fluoro-3-methoxyphenyl | H | Me | CO2H |
| 1-181 | 4-chloro-2-fluoro-3-methoxyphenyl | H | Me | CO2Me |
| 1-182 | 4-chloro-2-fluoro-3-methoxyphenyl | H | 1-propenyl | CO2H |
| 1-183 | 4-chloro-2-fluoro-3-methoxyphenyl | H | 1-propenyl | CO2Me |
| 1-184 | 4-chloro-2-fluoro-3-methoxyphenyl | F | Cl | CO2H |
| 1-185 | 4-chloro-2-fluoro-3-methoxyphenyl | F | Cl | CO2Me |
| 1-186 | 4-chloro-2-fluoro-3-methoxyphenyl | F | cyclopropyl | CO2H |
| 1-187 | 4-chloro-2-fluoro-3-methoxyphenyl | F | cyclopropyl | CO2Me |
| 1-188 | 4-chloro-2-fluoro-3-methoxyphenyl | F | vinyl | CO2H |
| 1-189 | 4-chloro-2-fluoro-3-methoxyphenyl | F | vinyl | CO2Me |
| 1-190 | 4-chloro-2-fluoro-3-methoxyphenyl | F | Br | CO2H |
| 1-191 | 4-chloro-2-fluoro-3-methoxyphenyl | F | Br | CO2Me |
| 1-192 | 4-chloro-2-fluoro-3-methoxyphenyl | F | Me | CO2H |
| 1-193 | 4-chloro-2-fluoro-3-methoxyphenyl | F | Me | CO2Me |
| 1-194 | 4-chloro-2-fluoro-3-methoxyphenyl | F | 1-propenyl | CO2H |
| 1-195 | 4-chloro-2-fluoro-3-methoxyphenyl | F | 1-propenyl | CO2Me |
| 1-196 | 4-chloro-2-fluoro-3-methoxyphenyl | Cl | Cl | CO2H |
| 1-197 | 4-chloro-2-fluoro-3-methoxyphenyl | Cl | Cl | CO2Me |
| 1-198 | 4-chloro-2-fluoro-3-methoxyphenyl | Cl | cyclopropyl | CO2H |
| 1-199 | 4-chloro-2-fluoro-3-methoxyphenyl | Cl | cyclopropyl | CO2Me |
| 1-200 | 4-chloro-2-fluoro-3-methoxyphenyl | Cl | vinyl | CO2H |
| 1-201 | 4-chloro-2-fluoro-3-methoxyphenyl | Cl | vinyl | CO2Me |
| 1-202 | 4-chloro-2-fluoro-3-methoxyphenyl | Cl | Br | CO2H |
| 1-203 | 4-chloro-2-fluoro-3-methoxyphenyl | Cl | Br | CO2Me |
| 1-204 | 4-chloro-2-fluoro-3-methoxyphenyl | Cl | Me | CO2H |
| 1-205 | 4-chloro-2-fluoro-3-methoxyphenyl | Cl | Me | CO2Me |
| 1-206 | 4-chloro-2-fluoro-3-methoxyphenyl | Cl | 1-propenyl | CO2H |
| 1-207 | 4-chloro-2-fluoro-3-methoxyphenyl | Cl | 1-propenyl | CO2Me |
| 1-208 | 4-chloro-2-fluoro-3-methoxyphenyl | Br | Cl | CO2H |
| 1-209 | 4-chloro-2-fluoro-3-methoxyphenyl | Br | Cl | CO2Me |
| 1-210 | 4-chloro-2-fluoro-3-methoxyphenyl | Br | cyclopropyl | CO2H |
| 1-211 | 4-chloro-2-fluoro-3-methoxyphenyl | Br | cyclopropyl | CO2Me |
| 1-212 | 4-chloro-2-fluoro-3-methoxyphenyl | Br | vinyl | CO2H |
| 1-213 | 4-chloro-2-fluoro-3-methoxyphenyl | Br | vinyl | CO2Me |
| 1-214 | 4-chloro-2-fluoro-3-methoxyphenyl | Br | Br | CO2H |
| 1-215 | 4-chloro-2-fluoro-3-methoxyphenyl | Br | Br | CO2Me |
| 1-216 | 4-chloro-2-fluoro-3-methoxyphenyl | Br | Me | CO2H |
| 1-217 | 4-chloro-2-fluoro-3-methoxyphenyl | Br | Me | CO2Me |
| 1-218 | 4-chloro-2-fluoro-3-methoxyphenyl | Br | 1-propenyl | CO2H |
| 1-219 | 4-chloro-2-fluoro-3-methoxyphenyl | Br | 1-propenyl | CO2Me |
| 1-220 | 4-chloro-2-fluoro-3-methoxyphenyl | Me | Cl | CO2H |
| 1-221 | 4-chloro-2-fluoro-3-methoxyphenyl | Me | Cl | CO2Me |
| 1-222 | 4-chloro-2-fluoro-3-methoxyphenyl | Me | cyclopropyl | CO2H |
| 1-223 | 4-chloro-2-fluoro-3-methoxyphenyl | Me | cyclopropyl | CO2Me |
| 1-224 | 4-chloro-2-fluoro-3-methoxyphenyl | Me | vinyl | CO2H |
| 1-225 | 4-chloro-2-fluoro-3-methoxyphenyl | Me | vinyl | CO2Me |
| 1-226 | 4-chloro-2-fluoro-3-methoxyphenyl | Me | Br | CO2H |
| 1-227 | 4-chloro-2-fluoro-3-methoxyphenyl | Me | Br | CO2Me |
| 1-228 | 4-chloro-2-fluoro-3-methoxyphenyl | Me | Me | CO2H |
| 1-229 | 4-chloro-2-fluoro-3-methoxyphenyl | Me | Me | CO2Me |
| 1-230 | 4-chloro-2-fluoro-3-methoxyphenyl | Me | 1-propenyl | CO2H |
| 1-231 | 4-chloro-2-fluoro-3-methoxyphenyl | Me | 1-propenyl | CO2Me |
| 1-232 | 4-chloro-2-fluoro-3-methoxyphenyl | cyclopropyl | Cl | CO2H |
| 1-233 | 4-chloro-2-fluoro-3-methoxyphenyl | cyclopropyl | Cl | CO2Me |
| 1-234 | 4-chloro-2-fluoro-3-methoxyphenyl | cyclopropyl | cyclopropyl | CO2H |
| 1-235 | 4-chloro-2-fluoro-3-methoxyphenyl | cyclopropyl | cyclopropyl | CO2Me |
| 1-236 | 4-chloro-2-fluoro-3-methoxyphenyl | cyclopropyl | vinyl | CO2H |
| 1-237 | 4-chloro-2-fluoro-3-methoxyphenyl | cyclopropyl | vinyl | CO2Me |
| 1-238 | 4-chloro-2-fluoro-3-methoxyphenyl | cyclopropyl | Br | CO2H |
| 1-239 | 4-chloro-2-fluoro-3-methoxyphenyl | cyclopropyl | Br | CO2Me |
| 1-240 | 4-chloro-2-fluoro-3-methoxyphenyl | cyclopropyl | Me | CO2H |
| 1-241 | 4-chloro-2-fluoro-3-methoxyphenyl | cyclopropyl | Me | CO2Me |
| 1-242 | 4-chloro-2-fluoro-3-methoxyphenyl | cyclopropyl | 1-propenyl | CO2H |
| 1-243 | 4-chloro-2-fluoro-3-methoxyphenyl | cyclopropyl | 1-propenyl | CO2Me |
| 1-244 | 4-chloro-3-dimethylamino-2-fluorophenyl | H | Cl | CO2H |
| 1-245 | 4-chloro-3-dimethylamino-2-fluorophenyl | H | Cl | CO2Me |
| 1-246 | 4-chloro-3-dimethylamino-2-fluorophenyl | H | Cl | CO2Et |
| 1-247 | 4-chloro-3-dimethylamino-2-fluorophenyl | H | Cl | CO2CH2CH2OEt |
| 1-248 | 4-chloro-3-dimethylamino-2-fluorophenyl | H | Cl | CO2CH2Ph |
| 1-249 | 4-chloro-3-dimethylamino-2-fluorophenyl | H | cyclopropyl | CO2H |
| 1-250 | 4-chloro-3-dimethylamino-2-fluorophenyl | H | cyclopropyl | CO2Me |
| 1-251 | 4-chloro-3-dimethylamino-2-fluorophenyl | H | cyclopropyl | CO2Et |
| 1-252 | 4-chloro-3-dimethylamino-2-fluorophenyl | H | cyclopropyl | CO2CH2CH2OEt |
| 1-253 | 4-chloro-3-dimethylamino-2-fluorophenyl | H | cyclopropyl | CO2CH2Ph |
| 1-254 | 4-chloro-3-dimethylamino-2-fluorophenyl | H | vinyl | CO2H |
| 1-255 | 4-chloro-3-dimethylamino-2-fluorophenyl | H | vinyl | CO2Me |
| 1-256 | 4-chloro-3-dimethylamino-2-fluorophenyl | H | vinyl | CO2Et |
| 1-257 | 4-chloro-3-dimethylamino-2-fluorophenyl | H | vinyl | CO2CH2CH2OEt |
| 1-258 | 4-chloro-3-dimethylamino-2-fluorophenyl | H | vinyl | CO2CH2Ph |
| 1-259 | 4-chloro-3-dimethylamino-2-fluorophenyl | H | Br | CO2H |
| 1-260 | 4-chloro-3-dimethylamino-2-fluorophenyl | H | Br | CO2Me |
| 1-261 | 4-chloro-3-dimethylamino-2-fluorophenyl | H | Me | CO2H |
| 1-262 | 4-chloro-3-dimethylamino-2-fluorophenyl | H | Me | CO2Me |
| 1-263 | 4-chloro-3-dimethylamino-2-fluorophenyl | H | 1-propenyl | CO2H |
| 1-264 | 4-chloro-3-dimethylamino-2-fluorophenyl | H | 1-propenyl | CO2Me |
| 1-265 | 4-chloro-3-dimethylamino-2-fluorophenyl | F | Cl | CO2H |
| 1-266 | 4-chloro-3-dimethylamino-2-fluorophenyl | F | Cl | CO2Me |
| 1-267 | 4-chloro-3-dimethylamino-2-fluorophenyl | F | cyclopropyl | CO2H |
| 1-268 | 4-chloro-3-dimethylamino-2-fluorophenyl | F | cyclopropyl | CO2Me |
| 1-269 | 4-chloro-3-dimethylamino-2-fluorophenyl | F | vinyl | CO2H |
| 1-270 | 4-chloro-3-dimethylamino-2-fluorophenyl | F | vinyl | CO2Me |
| 1-271 | 4-chloro-3-dimethylamino-2-fluorophenyl | F | Br | CO2H |
| 1-272 | 4-chloro-3-dimethylamino-2-fluorophenyl | F | Br | CO2Me |
| 1-273 | 4-chloro-3-dimethylamino-2-fluorophenyl | F | Me | CO2H |
| 1-274 | 4-chloro-3-dimethylamino-2-fluorophenyl | F | Me | CO2Me |
| 1-275 | 4-chloro-3-dimethylamino-2-fluorophenyl | F | 1-propenyl | CO2H |
| 1-276 | 4-chloro-3-dimethylamino-2-fluorophenyl | F | 1-propenyl | CO2Me |
| 1-277 | 4-chloro-3-dimethylamino-2-fluorophenyl | Cl | Cl | CO2H |
| 1-278 | 4-chloro-3-dimethylamino-2-fluorophenyl | Cl | Cl | CO2Me |
| 1-279 | 4-chloro-3-dimethylamino-2-fluorophenyl | Cl | cyclopropyl | CO2H |
| 1-280 | 4-chloro-3-dimethylamino-2-fluorophenyl | Cl | cyclopropyl | CO2Me |
| 1-281 | 4-chloro-3-dimethylamino-2-fluorophenyl | Cl | vinyl | CO2H |
| 1-282 | 4-chloro-3-dimethylamino-2-fluorophenyl | Cl | vinyl | CO2Me |
| 1-283 | 4-chloro-3-dimethylamino-2-fluorophenyl | Cl | Br | CO2H |
| 1-284 | 4-chloro-3-dimethylamino-2-fluorophenyl | Cl | Br | CO2Me |
| 1-285 | 4-chloro-3-dimethylamino-2-fluorophenyl | Cl | Me | CO2H |
| 1-286 | 4-chloro-3-dimethylamino-2-fluorophenyl | Cl | Me | CO2Me |
| 1-287 | 4-chloro-3-dimethylamino-2-fluorophenyl | Cl | 1-propenyl | CO2H |
| 1-288 | 4-chloro-3-dimethylamino-2-fluorophenyl | Cl | 1-propenyl | CO2Me |
| 1-289 | 4-chloro-3-dimethylamino-2-fluorophenyl | Br | Cl | CO2H |
| 1-290 | 4-chloro-3-dimethylamino-2-fluorophenyl | Br | Cl | CO2Me |
| 1-291 | 4-chloro-3-dimethylamino-2-fluorophenyl | Br | cyclopropyl | CO2H |
| 1-292 | 4-chloro-3-dimethylamino-2-fluorophenyl | Br | cyclopropyl | CO2Me |
| 1-293 | 4-chloro-3-dimethylamino-2-fluorophenyl | Br | vinyl | CO2H |
| 1-294 | 4-chloro-3-dimethylamino-2-fluorophenyl | Br | vinyl | CO2Me |
| 1-295 | 4-chloro-3-dimethylamino-2-fluorophenyl | Br | Br | CO2H |
| 1-296 | 4-chloro-3-dimethylamino-2-fluorophenyl | Br | Br | CO2Me |
| 1-297 | 4-chloro-3-dimethylamino-2-fluorophenyl | Br | Me | CO2H |
| 1-298 | 4-chloro-3-dimethylamino-2-fluorophenyl | Br | Me | CO2Me |
| 1-299 | 4-chloro-3-dimethylamino-2-fluorophenyl | Br | 1-propenyl | CO2H |
| 1-300 | 4-chloro-3-dimethylamino-2-fluorophenyl | Br | 1-propenyl | CO2Me |
| 1-301 | 4-chloro-3-dimethylamino-2-fluorophenyl | Me | Cl | CO2H |
| 1-302 | 4-chloro-3-dimethylamino-2-fluorophenyl | Me | Cl | CO2Me |
| 1-303 | 4-chloro-3-dimethylamino-2-fluorophenyl | Me | cyclopropyl | CO2H |
| 1-304 | 4-chloro-3-dimethylamino-2-fluorophenyl | Me | cyclopropyl | CO2Me |
| 1-305 | 4-chloro-3-dimethylamino-2-fluorophenyl | Me | vinyl | CO2H |
| 1-306 | 4-chloro-3-dimethylamino-2-fluorophenyl | Me | vinyl | CO2Me |
| 1-307 | 4-chloro-3-dimethylamino-2-fluorophenyl | Me | Br | CO2H |
| 1-308 | 4-chloro-3-dimethylamino-2-fluorophenyl | Me | Br | CO2Me |
| 1-309 | 4-chloro-3-dimethylamino-2-fluorophenyl | Me | Me | CO2H |
| 1-310 | 4-chloro-3-dimethylamino-2-fluorophenyl | Me | Me | CO2Me |
| 1-311 | 4-chloro-3-dimethylamino-2-fluorophenyl | Me | 1-propenyl | CO2H |
| 1-312 | 4-chloro-3-dimethylamino-2-fluorophenyl | Me | 1-propenyl | CO2Me |
| 1-313 | 4-chloro-3-dimethylamino-2-fluorophenyl | cyclopropyl | Cl | CO2H |
| 1-314 | 4-chloro-3-dimethylamino-2-fluorophenyl | cyclopropyl | Cl | CO2Me |
| 1-315 | 4-chloro-3-dimethylamino-2-fluorophenyl | cyclopropyl | cyclopropyl | CO2H |
| 1-316 | 4-chloro-3-dimethylamino-2-fluorophenyl | cyclopropyl | cyclopropyl | CO2Me |
| 1-317 | 4-chloro-3-dimethylamino-2-fluorophenyl | cyclopropyl | vinyl | CO2H |
| 1-318 | 4-chloro-3-dimethylamino-2-fluorophenyl | cyclopropyl | vinyl | CO2Me |
| 1-319 | 4-chloro-3-dimethylamino-2-fluorophenyl | cyclopropyl | Br | CO2H |
| 1-320 | 4-chloro-3-dimethylamino-2-fluorophenyl | cyclopropyl | Br | CO2Me |
| 1-321 | 4-chloro-3-dimethylamino-2-fluorophenyl | cyclopropyl | Me | CO2H |
| 1-322 | 4-chloro-3-dimethylamino-2-fluorophenyl | cyclopropyl | Me | CO2Me |
| 1-323 | 4-chloro-3-dimethylamino-2-fluorophenyl | cyclopropyl | 1-propenyl | CO2H |
| 1-324 | 4-chloro-3-dimethylamino-2-fluorophenyl | cyclopropyl | 1-propenyl | CO2Me |
| 1-325 | isopropyl | H | Cl | CO2H |
| 1-326 | isopropyl | H | Cl | CO2Me |
| 1-327 | isopropyl | H | cyclopropyl | CO2H |
| 1-328 | isopropyl | H | cyclopropyl | CO2Me |
| 1-329 | isopropyl | H | vinyl | CO2H |
| 1-330 | isopropyl | H | vinyl | CO2Me |
| 1-331 | 4-chlorophenyl | H | Cl | CO2H |
| 1-332 | 4-chlorophenyl | H | Cl | CO2Me |
| 1-333 | 4-chlorophenyl | H | cyclopropyl | CO2H |
| 1-334 | 4-chlorophenyl | H | cyclopropyl | CO2Me |
| 1-335 | 4-chlorophenyl | H | vinyl | CO2H |
| 1-336 | 4-chlorophenyl | H | vinyl | CO2Me |
| 1-337 | 4-bromophenyl | H | Cl | CO2H |
| 1-338 | 4-bromophenyl | H | Cl | CO2Me |
| 1-339 | 4-bromophenyl | H | cyclopropyl | CO2H |
| 1-340 | 4-bromophenyl | H | cyclopropyl | CO2Me |
| 1-341 | 4-bromophenyl | H | vinyl | CO2H |
| 1-342 | 4-bromophenyl | H | vinyl | CO2Me |
| 1-343 | 2,4-dichlorophenyl | H | Cl | CO2H |
| 1-344 | 2,4-dichlorophenyl | H | Cl | CO2Me |
| 1-345 | 2,4-dichlorophenyl | H | cyclopropyl | CO2H |
| 1-346 | 2,4-dichlorophenyl | H | cyclopropyl | CO2Me |
| 1-347 | 2,4-dichlorophenyl | H | vinyl | CO2H |
| 1-348 | 2,4-dichlorophenyl | H | vinyl | CO2Me |
| 1-349 | 3,4-dichlorophenyl | H | Cl | CO2H |
| 1-350 | 3,4-dichlorophenyl | H | Cl | CO2Me |
| 1-351 | 3,4-dichlorophenyl | H | cyclopropyl | CO2H |
| 1-352 | 3,4-dichlorophenyl | H | cyclopropyl | CO2Me |
| 1-353 | 3,4-dichlorophenyl | H | vinyl | CO2H |
| 1-354 | 3,4-dichlorophenyl | H | vinyl | CO2Me |
| 1-355 | 4-chloro-2-fluorophenyl | H | Cl | CO2H |
| 1-356 | 4-chloro-2-fluorophenyl | H | Cl | CO2Me |
| 1-357 | 4-chloro-2-fluorophenyl | H | cyclopropyl | CO2H |
| 1-358 | 4-chloro-2-fluorophenyl | H | cyclopropyl | CO2Me |
| 1-359 | 4-chloro-2-fluorophenyl | H | vinyl | CO2H |
| 1-360 | 4-chloro-2-fluorophenyl | H | vinyl | CO2Me |
| 1-361 | 4-chloro-2-fluorophenyl | H | Cl | CO2H |
| 1-362 | 4-chloro-2-fluorophenyl | H | Cl | CO2Me |
| 1-363 | 4-chloro-2-fluorophenyl | H | cyclopropyl | CO2H |
| 1-364 | 4-chloro-2-fluorophenyl | H | cyclopropyl | CO2Me |
| 1-365 | 4-chloro-3-fluorophenyl | H | vinyl | CO2H |
| 1-366 | 4-chloro-2-fluorophenyl | H | vinyl | CO2Me |
| 1-367 | 2,4-dichloro-3-fluorophenyl | H | Cl | CO2H |
| 1-368 | 2,4-dichloro-3-fluorophenyl | H | Cl | CO2Me |
| 1-369 | 2,4-dichloro-3-fluorophenyl | H | cyclopropyl | CO2H |
| 1-370 | 2,4-dichloro-3-fluorophenyl | H | cyclopropyl | CO2Me |
| 1-371 | 2,4-dichloro-3-fluorophenyl | H | vinyl | CO2H |
| 1-372 | 2,4-dichloro-3-fluorophenyl | H | vinyl | CO2Me |
| 1-373 | 4-chloro-2,3-difluorophenyl | H | Cl | ùCO2H |
| 1-374 | 4-chloro-2,3-difluorophenyl | H | Cl | CO2Me |
| 1-375 | 4-chloro-2,3-difluorophenyl | H | cyclopropyl | CO2H |
| 1-376 | 4-chloro-2,3-difluorophenyl | H | cyclopropyl | CO2Me |
| 1-377 | 4-chloro-2,3-difluorophenyl | H | vinyl | CO2H |
| 1-378 | 4-chloro-2,3-difluorophenyl | H | vinyl | CO2Me |
| 1-379 | 3,4-dichloro-2-fluorophenyl | H | Cl | CO2H |
| 1-380 | 3,4-dichloro-2-fluorophenyl | H | Cl | CO2Me |
| 1-381 | 3,4-dichloro-2-fluorophenyl | H | cyclopropyl | CO2H |
| 1-382 | 3,4-dichloro-2-fluorophenyl | H | cyclopropyl | CO2Me |
| 1-383 | 3,4-dichloro-2-fluorophenyl | H | vinyl | CO2H |
| 1-384 | 3,4-dichloro-2-fluorophenyl | H | vinyl | CO2Me |
| 1-385 | 2,3,4-trichlorophenyl | H | Cl | CO2H |
| 1-386 | 2,3,4-trichlorophenyl | H | Cl | CO2Me |
| 1-387 | 2,3,4-trichlorophenyl | H | cyclopropyl | CO2H |
| 1-388 | 2,3,4-trichlorophenyl | H | cyclopropyl | CO2Me |
| 1-389 | 2,3,4-trichlorophenyl | H | vinyl | CO2H |
| 1-390 | 2,3,4-trichlorophenyl | H | vinyl | CO2Me |
| 1-391 | 4-trifluoromethylphenyl | H | Cl | CO2H |
| 1-392 | 4-trifluoromethylphenyl | H | Cl | CO2Me |
| 1-393 | 4-trifluoromethylphenyl | H | cyclopropyl | CO2H |
| 1-394 | 4-trifluoromethylphenyl | H | cyclopropyl | CO2Me |
| 1-395 | 4-trifluoromethylphenyl | H | vinyl | CO2H |
| 1-396 | 4-trifluoromethylphenyl | H | vinyl | CO2Me |
| 1-397 | 4-methylphenyl | H | Cl | CO2H |
| 1-398 | 4-methylphenyl | H | Cl | CO2Me |
| 1-399 | 4-methylphenyl | H | cyclopropyl | CO2H |
| 1-400 | 4-methylphenyl | H | cyclopropyl | CO2Me |
| 1-401 | 4-methylphenyl | H | vinyl | CO2H |
| 1-402 | 4-methylphenyl | H | vinyl | CO2Me |
| 1-403 | 2-chloro-4-methylphenyl | H | Cl | CO2H |
| 1-404 | 2-chloro-4-methylphenyl | H | Cl | CO2Me |
| 1-405 | 2-chloro-4-methylphenyl | H | cyclopropyl | CO2H |
| 1-406 | 2-chloro-4-methylphenyl | H | cyclopropyl | CO2Me |
| 1-407 | 2-chloro-4-methylphenyl | H | vinyl | CO2H |
| 1-408 | 2-chloro-4-methylphenyl | H | vinyl | CO2Me |
| 1-409 | 4,5-dichloro-2-fluorophenyl | H | Cl | CO2H |
| 1-410 | 4,5-dichloro-2-fluorophenyl | H | Cl | CO2Me |
| 1-411 | 4,5-dichloro-2-fluorophenyl | H | cyclopropyl | CO2H |
| 1-412 | 4,5-dichloro-2-fluorophenyl | H | cyclopropyl | CO2Me |
| 1-413 | 4,5-dichloro-2-fluorophenyl | H | vinyl | CO2H |
| 1-414 | 4,5-dichloro-2-fluorophenyl | H | vinyl | CO2Me |
| 1-415 | 4-chloro-2,5-difluorophenyl | H | Cl | CO2H |
| 1-416 | 4-chloro-2,5-difluorophenyl | H | Cl | CO2Me |
| 1-417 | 4-chloro-2,5-difluorophenyl | H | cyclopropyl | CO2H |
| 1-418 | 4-chloro-2,5-difluorophenyl | H | cyclopropyl | CO2Me |
| 1-419 | 4-chloro-2,5-difluorophenyl | H | vinyl | CO2H |
| 1-420 | 4-chloro-2,5-difluorophenyl | H | vinyl | CO2Me |
| 1-421 | 5-chlorothiophen-2-yl | H | Cl | CO2H |
| 1-422 | 5-chlorothiophen-2-yl | H | Cl | CO2Me |
| 1-423 | 5-chlorothiophen-2-yl | H | cyclopropyl | CO2H |
| 1-424 | 5-chlorothiophen-2-yl | H | cyclopropyl | CO2Me |
| 1-425 | 5-chlorothiophen-2-yl | H | vinyl | CO2H |
| 1-426 | 5-chlorothiophen-2-yl | H | vinyl | CO2Me |
| 1-427 | 6-chloropyridin-3-yl | H | Cl | CO2H |
| 1-428 | 6-chloropyridin-3-yl | H | Cl | CO2Me |
| 1-429 | 6-chloropyridin-3-yl | H | cyclopropyl | CO2H |
| 1-430 | 6-chloropyridin-3-yl | H | cyclopropyl | CO2Me |
| 1-431 | 6-chloropyridin-3-yl | H | vinyl | CO2H |
| 1-432 | 6-chloropyridin-3-yl | H | vinyl | CO2Me |
| 1-433 | methylthio | H | Cl | CO2H |
| 1-434 | methylthio | H | Cl | CO2Me |
| 1-435 | methylthio | H | cyclopropyl | CO2H |
| 1-436 | methylthio | H | cyclopropyl | CO2Me |
| 1-437 | methylthio | H | vinyl | CO2H |
| 1-438 | methylthio | H | vinyl | CO2Me |
| 1-439 | Cl | H | CF3 | CO2H |
| 1-440 | Cl | H | CF3 | CO2Me |
| 1-441 | Cl | F | CF3 | CO2H |
| 1-442 | Cl | F | CF3 | CO2Me |
| 1-443 | Cl | Cl | CF3 | CO2H |
| 1-444 | Cl | Cl | CF3 | CO2Me |
| 1-445 | Cl | H | ethynyl | CO2H |
| 1-446 | Cl | H | ethynyl | CO2Me |
| 1-447 | Cl | F | ethynyl | CO2H |
| 1-448 | Cl | F | ethynyl | CO2Me |
| 1-449 | Cl | Cl | ethynyl | CO2H |
| 1-450 | Cl | Cl | ethynyl | CO2Me |
| 1-451 | Cl | H | 1-propynyl | CO2H |
| 1-452 | Cl | H | 1-propynyl | CO2Me |
| 1-453 | Cl | F | 1-propynyl | CO2H |
| 1-454 | Cl | F | 1-propynyl | CO2Me |
| 1-455 | Cl | Cl | 1-propynyl | CO2H |
| 1-456 | Cl | Cl | 1-propynyl | CO2Me |
| 1-457 | Cl | H | phenyl | CO2H |
| 1-458 | Cl | H | phenyl | CO2Me |
| 1-459 | Cl | F | phenyl | CO2H |
| 1-460 | Cl | F | phenyl | CO2Me |
| 1-461 | Cl | Cl | phenyl | CO2H |
| 1-462 | Cl | Cl | phenyl | CO2Me |
| 1-463 | 4-chloro-2-fluoro-3-methoxyphenyl | Cl | 2-ethoxyvinyl | CO2Me |
| 1-464 | Me | Cl | Me | CO2H |
| 1-465 | Me | Cl | Me | CO2Me |
| 1-466 | Vinyl | F | Vinyl | CO2H |
| 1-467 | vinyl | F | vinyl | CO2Me |

467 compounds are described, designated compounds 2-1 to 2-467 respectively, of formula (I) wherein R¹ is H, R⁴ is H, R⁵ is H, W is a direct bond and Q is phenyl, and the values of A, X, Y and Z are as defined in Table 1.

467 compounds are described, designated compounds 3-1 to 3-467 respectively, of formula (I) wherein R¹ is H, R⁴ is H, R⁵ is H, W is a direct bond and Q is 2-chlorophenyl, and the values of A, X, Y and Z are as defined in Table 1.

467 compounds are described, designated compounds 4-1 to 4-467 respectively, of formula (I) wherein R¹ is H, R⁴ is H, R⁵ is H, W is a direct bond and Q is 3-chlorophenyl, and the values of A, X, Y and Z are as defined in Table 1.

467 compounds are described, designated compounds 5-1 to 5-467 respectively, of formula (I) wherein R¹ is H, R⁴ is H, R⁵ is H, W is a direct bond and Q is 4-chlorophenyl, and the values of A, X, Y and Z are as defined in Table 1.

467 compounds are described, designated compounds 6-1 to 6-467 respectively, of formula (I) wherein R¹ is H, R⁴ is H, R⁵ is H, W is a direct bond and Q is 2-fluorophenyl, and the values of A, X, Y and Z are as defined in Table 1.

467 compounds are described, designated compounds 7-1 to 7-467 respectively, of formula (I) wherein R¹ is H, R⁴ is H, R⁵ is H, W is a direct bond and Q is 3-fluorophenyl, and the values of A, X, Y and Z are as defined in Table 1.

467 compounds are described, designated compounds 8-1 to 8-467 respectively, of formula (I) wherein R¹ is H, R⁴ is H, R⁵ is H, W is a direct bond and Q is 4-fluorophenyl, and the values of A, X, Y and Z are as defined in Table 1.

467 compounds are described, designated compounds 9-1 to 9-467 respectively, of formula (I) wherein R¹ is H, R⁴ is H, R⁵ is H, W is a direct bond and Q is 2-methylphenyl, and the values of A, X, Y and Z are as defined in Table 1.

467 compounds are described, designated compounds 10-1 to 10-467 respectively, of formula (I) wherein R¹ is H, R⁴ is H, R⁵ is H, W is a direct bond and Q is 4-methylphenyl, and the values of A, X, Y and Z are as defined in Table 1.

467 compounds are described, designated compounds 11-1 to 11-467 respectively, of formula (I) wherein R¹ is H, R⁴ is H, R⁵ is H, W is a direct bond and Q is 3-trifluoromethylphenyl, and the values of A, X, Y and Z are as defined in Table 1.

467 compounds are described, designated compounds 12-1 to 12-467 respectively, of formula (I) wherein R¹ is H, R⁴ is H, R⁵ is H, W is a direct bond and Q is 4-trifluoromethylphenyl, and the values of A, X, Y and Z are as defined in Table 1.

467 compounds are described, designated compounds 13-1 to 13-467 respectively, of formula (I) wherein R¹ is H, R⁴ is H, R⁵ is H, W is a direct bond and Q is 4-aminophenyl, and the values of A, X, Y and Z are as defined in Table 1.

467 compounds are described, designated compounds 14-1 to 14-467 respectively, of formula (I) wherein R¹ is H, R⁴ is H, R⁵ is H, W is a direct bond and Q is 4-dimethylaminophenyl, and the values of A, X, Y and Z are as defined in Table 1.

467 compounds are described, designated compounds 15-1 to 15-467 respectively, of formula (I) wherein R¹ is H, R⁴ is H, R⁵ is H, W is a direct bond and Q is 2-nitrophenyl, and the values of A, X, Y and Z are as defined in Table 1.

467 compounds are described, designated compounds 16-1 to 16-467 respectively, of formula (I) wherein R¹ is H, R⁴ is H, R⁵ is H, W is a direct bond and Q is 3-nitrophenyl, and the values of A, X, Y and Z are as defined in Table 1.

467 compounds are described, designated compounds 17-1 to 17-467 respectively, of formula (I) wherein R¹ is H, R⁴ is H, R⁵ is H, W is a direct bond and Q is 4-nitrophenyl, and the values of A, X, Y and Z are as defined in Table 1.

467 compounds are described, designated compounds 18-1 to 18-467 respectively, of formula (I) wherein R¹ is H, R⁴ is H, R⁵ is H, W is a direct bond and Q is 2,5-difluorophenyl, and the values of A, X, Y and Z are as defined in Table 1.

467 compounds are described, designated compounds 19-1 to 19-467 respectively, of formula (I) wherein R¹ is H, R⁴ is H, R⁵ is H, W is a direct bond and Q is 2,6-difluorophenyl, and the values of A, X, Y and Z are as defined in Table 1.

467 compounds are described, designated compounds 20-1 to 20-467 respectively, of formula (I) wherein R¹ is H, R⁴ is H, R⁵ is H, W is a direct bond and Q is 3,5-difluorophenyl, and the values of A, X, Y and Z are as defined in Table 1.

467 compounds are described, designated compounds 21-1 to 21-467 respectively, of formula (I) wherein R¹ is H, R⁴ is H, R⁵ is H, W is a direct bond and Q is 2,6-dichlorophenyl, and the values of A, X, Y and Z are as defined in Table 1.

467 compounds are described, designated compounds 22-1 to 22-467 respectively, of formula (I) wherein R¹ is H, R⁴ is H, R⁵ is H, W is a direct bond and Q is 2,4-dimethoxyphenyl, and the values of A, X, Y and Z are as defined in Table 1.

467 compounds are described, designated compounds 23-1 to 23-467 respectively, of formula (I) wherein R¹ is H, R⁴ is H, R⁵ is H, W is a direct bond and Q is 3,4-dimethoxyphenyl, and the values of A, X, Y and Z are as defined in Table 1.

467 compounds are described, designated compounds 24-1 to 24-467 respectively, of formula (I) wherein R¹ is H, R⁴ is H, R⁵ is H, W is a direct bond and Q is benzo[1,3]dioxol-5-yl, and the values of A, X, Y and Z are as defined in Table 1.

467 compounds are described, designated compounds 25-1 to 25-467 respectively, of formula (I) wherein R¹ is H, R⁴ is H, R⁵ is H, W is a direct bond and Q is 4-chloro-3-methoxyphenyl, and the values of A, X, Y and Z are as defined in Table 1.

467 compounds are described, designated compounds 26-1 to 26-467 respectively, of formula (I) wherein R¹ is H, R⁴ is H, R⁵ is H, W is a direct bond and Q is 2-furanyl, and the values of A, X, Y and Z are as defined in Table 1.

467 compounds are described, designated compounds 27-1 to 27-467 respectively, of formula (I) wherein R¹ is H, R⁴ is H, R⁵ is H, W is a direct bond and Q is 3-bromofuran-2-yl, and the values of A, X, Y and Z are as defined in Table 1.

467 compounds are described, designated compounds 28-1 to 28-467 respectively, of formula (I) wherein R¹ is H, R⁴ is H, R⁵ is H, W is a direct bond and Q is 5-bromofuran-2-yl, and the values of A, X, Y and Z are as defined in Table 1.

467 compounds are described, designated compounds 29-1 to 29-467 respectively, of formula (I) wherein R¹ is H, R⁴ is H, R⁵ is H, W is a direct bond and Q is 5-methylfuran-2-yl, and the values of A, X, Y and Z are as defined in Table 1.

467 compounds are described, designated compounds 30-1 to 30-467 respectively, of formula (I) wherein R¹ is H, R⁴ is H, R⁵ is H, W is a direct bond and Q is 5-bromothiophen-2-yl, and the values of A, X, Y and Z are as defined in Table 1.

467 compounds are described, designated compounds 31-1 to 31-467 respectively, of formula (I) wherein R¹ is H, R⁴ is H, R⁵ is H, W is a direct bond and Q is 2-pyridyl, and the values of A, X, Y and Z are as defined in Table 1.

467 compounds are described, designated compounds 32-1 to 32-467 respectively, of formula (I) wherein R¹ is H, R⁴ is H, R⁵ is H, W is a direct bond and Q is 3-chloropyrid-2-yl, and the values of A, X, Y and Z are as defined in Table 1.

467 compounds are described, designated compounds 33-1 to 33-467 respectively, of formula (I) wherein R¹ is H, R⁴ is H, R⁵ is H, W is a direct bond and Q is 3-methylpyrid-2-yl, and the values of A, X, Y and Z are as defined in Table 1.

467 compounds are described, designated compounds 34-1 to 34-467 respectively, of formula (I) wherein R¹ is H, R⁴ is methyl, R⁵ is H, W is a direct bond and Q is cyclopropyl, and the values of A, X, Y and Z are as defined in Table 1.

467 compounds are described, designated compounds 35-1 to 35-467 respectively, of formula (I) wherein R¹ is H, R⁴ is methyl, R⁵ is H, W is a direct bond and Q is 4-phenyl, and the values of A, X, Y and Z are as defined in Table 1.

467 compounds are described, designated compounds 36-1 to 36-467 respectively, of formula (I) wherein R¹ is H, R⁴ is methyl, R⁵ is H, W is a direct bond and Q is 4-fluorophenyl, and the values of A, X, Y and Z are as defined in Table 1.

467 compounds are described, designated compounds 37-1 to 37-467 respectively, of formula (I) wherein R¹ is H, R⁴ is methyl, R⁵ is H, W is a direct bond and Q is 2-furanyl, and the values of A, X, Y and Z are as defined in Table 1.

467 compounds are described, designated compounds 38-1 to 38-467 respectively, of formula (I) wherein R¹ is H, R⁴ is methyl, R⁵ is methyl, W is a direct bond and Q is phenyl, and the values of A, X, Y and Z are as defined in Table 1.

467 compounds are described, designated compounds 39-1 to 39-467 respectively, of formula (I) wherein R¹ is H, R⁴ is H, R⁵ is H, W is CH₂ and Q is phenyl, and the values of A, X, Y and Z are as defined in Table 1.

467 compounds are described, designated compounds 40-1 to 40-467 respectively, of formula (I) wherein R¹ is H, R⁴ is H, R⁵ is H, W is CH(OH) and Q is phenyl, and the values of A, X, Y and Z are as defined in Table 1.

467 compounds are described, designated compounds 41-1 to 41-467 respectively, of formula (I) wherein R¹ is H, R⁴ is H, R⁵ is H, W is CH(OH)CH₂O and Q is phenyl, and the values of A, X, Y and Z are as defined in Table 1.

467 compounds are described, designated compounds 42-1 to 42-467 respectively, of formula (I) wherein R¹ is H, R⁴ is methyl, R⁵ is H, W is CH(OH) and Q is phenyl, and the values of A, X, Y and Z are as defined in Table 1.

467 compounds are described, designated compounds 43-1 to 43-467 respectively, of formula (I) wherein R¹ is H, R⁴ is CH₂OH, R⁵ is H, W is CH(OH) and Q is phenyl, and the values of A, X, Y and Z are as defined in Table 1.

467 compounds are described, designated compounds 44-1 to 44-467 respectively, of formula (I) wherein R¹ is H, R⁴ is CO₂H, R⁵ is H, W is CH₂OCH₂ and Q is phenyl, and the values of A, X, Y and Z are as defined in Table 1.

467 compounds are described, designated compounds 45-1 to 45-467 respectively, of formula (I) wherein R¹ is H, R⁴ is CO₂Me, R⁵ is H, W is CH(OH) and Q is phenyl, and the values of A, X, Y and Z are as defined in Table 1.

467 compounds are described, designated compounds 46-1 to 46-467 respectively, of formula (I) wherein R¹ is H, R⁴ is CO₂Et, R⁵ is H, W is a direct bond and Q is phenyl, and the values of A, X, Y and Z are as defined in Table 1.

467 compounds are described, designated compounds 47-1 to 47-467 respectively, of formula (I) wherein R¹ is methyl, R⁴ is H, R⁵ is H, W is a direct bond and Q is phenyl, and the values of A, X, Y and Z are as defined in Table 1.

467 compounds are described, designated compounds 48-1 to 48-467 respectively, of formula (I) wherein R¹ is methyl, R⁴ is H, R⁵ is H, W is a direct bond and Q is 3-chlorophenyl, and the values of A, X, Y and Z are as defined in Table 1.

467 compounds are described, designated compounds 49-1 to 49-467 respectively, of formula (I) wherein R¹ is methyl, R⁴ is H, R⁵ is H, W is a direct bond and Q is 2-fluorophenyl, and the values of A, X, Y and Z are as defined in Table 1.

467 compounds are described, designated compounds 50-1 to 50-467 respectively, of formula (I) wherein R¹ is methyl, R⁴ is H, R⁵ is H, W is a direct bond and Q is 3-fluorophenyl, and the values of A, X, Y and Z are as defined in Table 1.

467 compounds are described, designated compounds 51-1 to 51-467 respectively, of formula (I) wherein R¹ is methyl, R⁴ is H, R⁵ is H, W is a direct bond and Q is 4-fluorophenyl, and the values of A, X, Y and Z are as defined in Table 1.

467 compounds are described, designated compounds 52-1 to 52-467 respectively, of formula (I) wherein R¹ is methyl, R⁴ is H, R⁵ is H, W is a direct bond and Q is 4-aminophenyl, and the values of A, X, Y and Z are as defined in Table 1.

467 compounds are described, designated compounds 53-1 to 53-467 respectively, of formula (I) wherein R¹ is methyl, R⁴ is H, R⁵ is H, W is a direct bond and Q is 2-nitrophenyl, and the values of A, X, Y and Z are as defined in Table 1.

467 compounds are described, designated compounds 54-1 to 54-467 respectively, of formula (I) wherein R¹ is methyl, R⁴ is H, R⁵ is H, W is a direct bond and Q is 4-nitrophenyl, and the values of A, X, Y and Z are as defined in Table 1.

467 compounds are described, designated compounds 55-1 to 55-467 respectively, of formula (I) wherein R¹ is methyl, R⁴ is H, R⁵ is H, W is a direct bond and Q is 2-furanyl, and the values of A, X, Y and Z are as defined in Table 1.

467 compounds are described, designated compounds 56-1 to 56-467 respectively, of formula (I) wherein R¹ is methyl, R⁴ is H, R⁵ is H, W is a direct bond and Q is 5-methylfuran-2-yl, and the values of A, X, Y and Z are as defined in Table 1.

467 compounds are described, designated compounds 57-1 to 57-467 respectively, of formula (I) wherein R¹ is methyl, R⁴ is H, R⁵ is H, W is a direct bond and Q is 5-bromothiophen-2-yl, and the values of A, X, Y and Z are as defined in Table 1.

467 compounds are described, designated compounds 58-1 to 58-467 respectively, of formula (I) wherein R¹ is methyl, R⁴ is H, R⁵ is H, W is a direct bond and Q is 4-pyridyl, and the values of A, X, Y and Z are as defined in Table 1.

467 compounds are described, designated compounds 59-1 to 59-467 respectively, of formula (I) wherein R¹ is ethyl, R⁴ is H, R⁵ is H, W is a direct bond and Q is phenyl, and the values of A, X, Y and Z are as defined in Table 1.

467 compounds are described, designated compounds 60-1 to 60-467 respectively, of formula (I) wherein R¹ is ethyl, R⁴ is H, R⁵ is H, W is a direct bond and Q is 2-fluorophenyl, and the values of A, X, Y and Z are as defined in Table 1.

467 compounds are described, designated compounds 61-1 to 61-467 respectively, of formula (I) wherein R¹ is ethyl, R⁴ is H, R⁵ is H, W is a direct bond and Q is 3-fluorophenyl, and the values of A, X, Y and Z are as defined in Table 1.

467 compounds are described, designated compounds 62-1 to 62-467 respectively, of formula (I) wherein R¹ is ethyl, R⁴ is H, R⁵ is H, W is a direct bond and Q is 4-fluorophenyl, and the values of A, X, Y and Z are as defined in Table 1.

467 compounds are described, designated compounds 63-1 to 63-467 respectively, of formula (I) wherein R¹ is ethyl, R⁴ is H, R⁵ is H, W is a direct bond and Q is 2-furanyl, and the values of A, X, Y and Z are as defined in Table 1.

467 compounds are described, designated compounds 64-1 to 64-467 respectively, of formula (I) wherein R¹ is ethyl, R⁴ is H, R⁵ is H, W is a direct bond and Q is 5-methylfuran-2-yl, and the values of A, X, Y and Z are as defined in Table 1.

467 compounds are described, designated compounds 65-1 to 65-467 respectively, of formula (I) wherein R¹ is isopropyl, R⁴ is H, R⁵ is H, W is a direct bond and Q is 2-furanyl, and the values of A, X, Y and Z are as defined in Table 1.

467 compounds are described, designated compounds 66-1 to 66-467 respectively, of formula (I) wherein R¹ is 2-hydroxyethyl, R⁴ is H, R⁵ is H, W is a direct bond and Q is 2-furanyl, and the values of A, X, Y and Z are as defined in Table 1.

467 compounds are described, designated compounds 67-1 to 67-467 respectively, of formula (I) wherein R¹ is H, R⁴ is H, R⁵ is H, W is a direct bond and Q is cyclohexyl, and the values of A, X, Y and Z are as defined in Table 1.

467 compounds are described, designated compounds 68-1 to 68-467 respectively, of formula (I) wherein R¹ is H, R⁴ is H, R⁵ is H, W is a direct bond and Q is 2-hydroxycyclohexyl, and the values of A, X, Y and Z are as defined in Table 1.

467 compounds are described, designated compounds 69-1 to 69-467 respectively, of formula (I) wherein R¹ is H, R⁴ is H, R⁵ is H, W is a direct bond and Q is cyclooctyl, and the values of A, X, Y and Z are as defined in Table 1.

467 compounds are described, designated compounds 70-1 to 70-467 respectively, of formula (I) wherein R¹ is H, R⁴ is H, R⁵ is H, W is a direct bond and Q is 2-tetrahydrofuranyl, and the values of A, X, Y and Z are as defined in Table 1.

467 compounds are described, designated compounds 71-1 to 71-467 respectively, of formula (I) wherein R¹ is H, R⁴ is H, R⁵ is H, W is a direct bond and Q is *N*-ethyl-pyrrolidin-2-yl, and the values of A, X, Y and Z are as defined in Table 1.

467 compounds are described, designated compounds 72-1 to 72-467 respectively, of formula (I) wherein R¹ is H, R⁴ is H, R⁵ is H, W is a direct bond and Q is 2-bromophenyl, and the values of A, X, Y and Z are as defined in Table 1.

467 compounds are described, designated compounds 73-1 to 73-467 respectively, of formula (I) wherein R¹ is H, R⁴ is H, R⁵ is H, W is a direct bond and Q is 2-methoxyphenyl, and the values of A, X, Y and Z are as defined in Table 1.

467 compounds are described, designated compounds 74-1 to 74-467 respectively, of formula (I) wherein R¹ is H, R⁴ is H, R⁵ is H, W is a direct bond and Q is 2-trifluoromethylphenyl, and the values of A, X, Y and Z are as defined in Table 1.

467 compounds are described, designated compounds 75-1 to 75-467 respectively, of formula (I) wherein R¹ is H, R⁴ is H, R⁵ is H, W is a direct bond and Q is thiophen-2-yl, and the values of A, X, Y and Z are as defined in Table 1.

467 compounds are described, designated compounds 76-1 to 76-467 respectively, of formula (I) wherein R¹ is H, R⁴ is H, R⁵ is H, W is a direct bond and Q is 2-bromothiophen-3-yl, and the values of A, X, Y and Z are as defined in Table 1.

467 compounds are described, designated compounds 77-1 to 77-467 respectively, of formula (I) wherein R¹ is H, R⁴ is H, R⁵ is H, W is a direct bond and Q is 1,3-dimethyl-pyrazol-5-yl, and the values of A, X, Y and Z are as defined in Table 1.

467 compounds are described, designated compounds 78-1 to 78-467 respectively, of formula (I) wherein R¹ is H, R⁴ is H, R⁵ is H, W is a direct bond and Q is 3-pyridyl, and the values of A, X, Y and Z are as defined in Table 1.

467 compounds are described, designated compounds 79-1 to 79-467 respectively, of formula (I) wherein R¹ is H, R⁴ is H, R⁵ is H, W is a direct bond and Q is 6-trifluoromethylpyrid-3-yl, and the values of A, X, Y and Z are as defined in Table 1.

467 compounds are described, designated compounds 80-1 to 80-467 respectively, of formula (I) wherein R¹ is H, R⁴ is H, R⁵ is H, W is a direct bond and Q is 4-pyridyl, and the values of A, X, Y and Z are as defined in Table 1.

467 compounds are described, designated compounds 81-1 to 81-467 respectively, of Formula (I) wherein R¹ is H, R⁴ is H, R⁵ is H, W is a direct bond and Q is 5-methylpyrazin-2-yl, and the values of A, X, Y and Z are as defined in Table 1.

General methods for the production of compounds of formula (I) are described below. Unless otherwise stated in the text, the substitutents A, R¹, R⁴, R⁵, W, Q, X, Y and Z are as defined hereinbefore. The abbreviation LG as used herein refers to any suitable leaving group. Preferred leaving groups are halogen, sulphonate (preferably tosylate), and sulphone groups.

Compounds of formula (I) may be prepared from compounds of formula (A) as shown in reaction scheme 1.

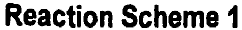

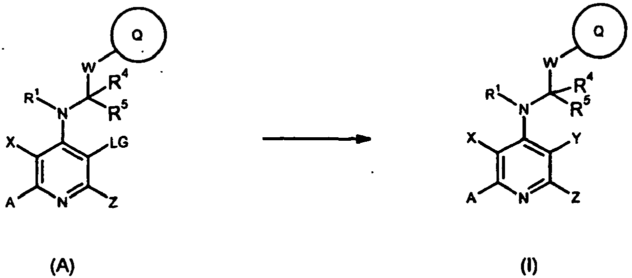

For example (see reaction scheme 2) a compound of formula (I), in which Y is a group attached through a carbon atom, may be prepared by reacting a suitable metal or metalloid derivative Y-M (e.g. a boronic acid or ester, a trialkyltin derivative, a zinc derivative or a Grignard reagent) with a compound of formula (A) in the presence of a suitable base (e.g. an inorganic base, such as potassium phosphate or caesium fluoride), a metal source (e.g. a palladium source, such as Pd(OAc)₂) and, optionally, a ligand for the metal (e.g. a phosphine ligand, such as PCy₃.HBF₄) in a suitable solvent (e.g. a single solvent, such as dimethylformamide, or a mixed solvent system, such as a mixture of dimethoxyethane and water or toluene and water). The metal catalyst and ligands may also be added as a single, pre-formed, complex (e.g. a palladium/phosphine complex, such as bis(triphenylphosphine)palladium dichloride or [1,1'-bis(diphenylphosphino)ferrocene] palladium dichloride dichloromethane adduct).

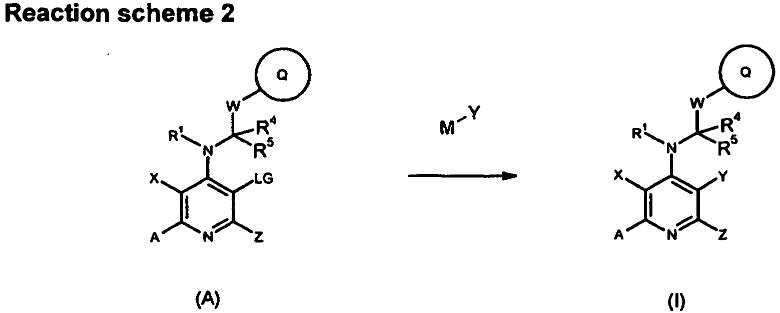

In a second example (see reaction scheme 3) a compound of formula (I), in which Y is a cyano group, may be prepared by reacting a metal cyanide (e.g. copper(I) cyanide) with a compound of formula (A) in a suitable solvent (e.g. dimethylformamide or N-methylpyrrolidone). This transformation may also be performed in the presence of a suitable metal (e.g. palladium) catalyst, optionally complexed by any suitable ligands (e.g. phosphine ligands, such as 1,1'-bis(diphenylphosphino)ferrocene).

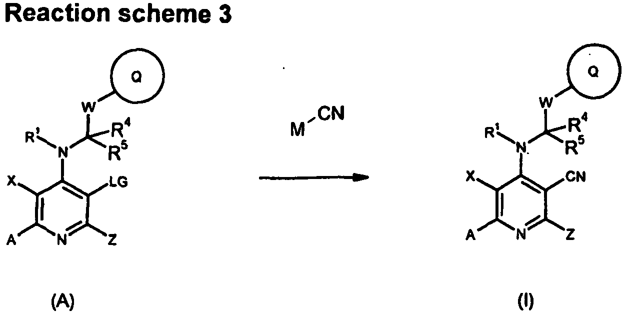

In a third example (see reaction scheme 4) a compound of formula (I), in which Y is a group attached through a nitrogen atom, may be prepared by reacting a suitable amine (e.g. propylamine) with a compound of formula (A) in the presence of a suitable base (e.g. an inorganic base, such as sodium tert-butoxide), a metal source (e.g. a palladium source, such as Pd₂(dba)₃) and, optionally, a ligand for the metal (e.g. a phosphine ligand, such as JohnPhos) in a suitable solvent (e.g. toluene).

In a fourth example (see reaction scheme 4) a compound of formula (I), in which Y is a group attached through a phosphorous atom, may be prepared by reacting a suitable phosphine or phosphonate (e.g. diphenylphosphine or diethylphosphite) with a compound of formula (A) in the presence of a suitable base (e.g. triethylamine), and a metal source (e.g. a palladium source, such as Pd(OAc)₂) and, optionally, a ligand for the metal (e.g. a phosphine ligand, such as triphenylphosphine) in a suitable solvent (e.g. toluene).

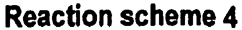

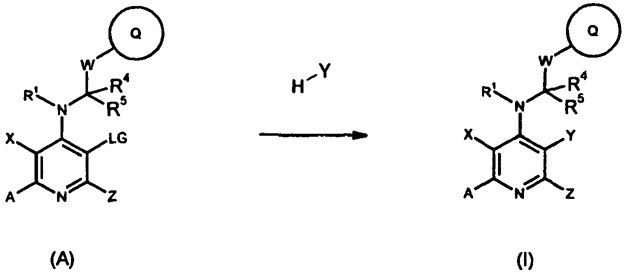

In a fifth example (see reaction scheme 5) a compound of formula (I), in which Y is a hydroxyl group, may be prepared by reacting a suitable organometallic reagent (e.g. n-butyllithium) with a compound of formula (A) in a suitable solvent (e.g. tetrahydrofuran) followed by a treatment with an alkylborane (e.g. trisisopropoxyborane), and ultimately exposed to an oxidative work up (e.g. hydrogen peroxide in aqueous base, such as sodium hydroxide).

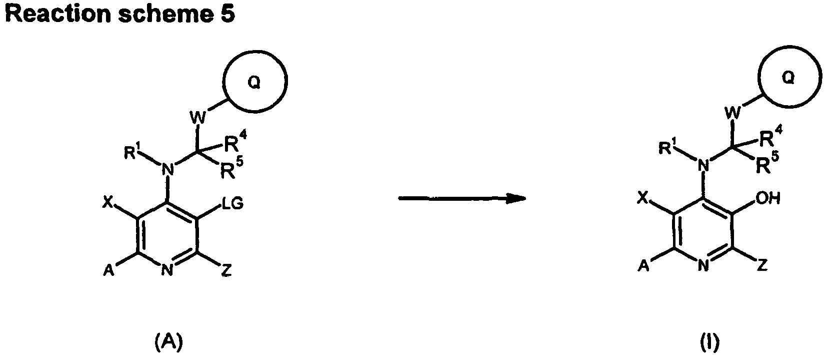

In a sixth example (see reaction scheme 6) a compound of formula (I) in which Y is an alkylthio group may be prepared by reacting a compound of formula (A) with a metal alkylthiolate (e.g. sodium methanethiolate) in a suitable solvent (e.g. methanol).

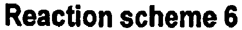

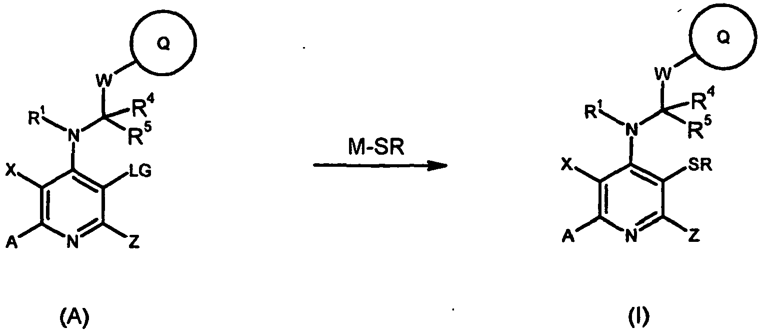

Compounds of formula (I) may be prepared from compounds of formula (B) as shown in reaction scheme 7.

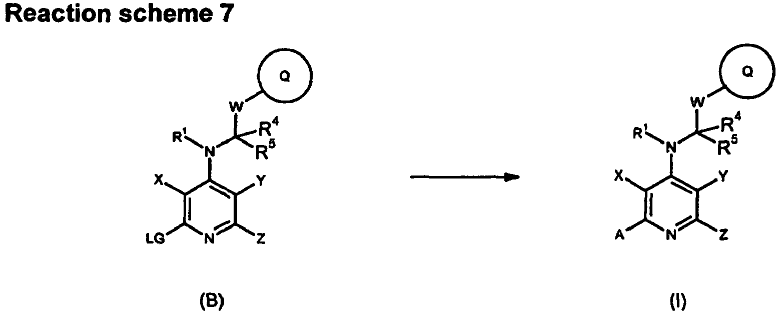

For example (see reaction scheme 8) a compound of formula (I), in which A is a group attached through a carbon atom, may be prepared by reacting a suitable metal or metalloid derivative A-M (e.g. a boronic acid or ester, a trialkyltin derivative, a zinc derivative or a Grignard reagent) with a compound of formula (B) in the presence of a suitable base (e.g. an inorganic base, such as potassium phosphate or caesium fluoride), a metal source (e.g. a palladium source, such as Pd(OAc)₂) and, optionally, a ligand for the metal (e.g. a phosphine ligand, such as PCy₃.HBF₄) in a suitable solvent (e.g. a single solvent, such as dimethylformamide, or a mixed solvent system, such as a mixture of dimethoxyethane and water or toluene and water). The metal catalyst and ligands may also be added as a single, pre-formed, complex (e.g. a palladium/phosphine complex, such as bis(triphenylphosphine)palladium dichloride or [1,1'-bis(diphenylphosphino)ferrocene] palladium dichloride dichloromethane adduct).

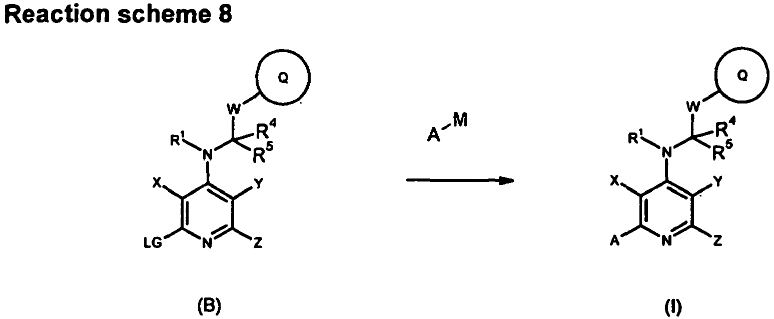

In a second example (see reaction scheme 9) a compound of formula (I), in which A is an alkylthio group, may be prepared by reacting a metal alkylthiolate (e.g. sodium methanethiolate) with a compound of formula (B) in a suitable solvent (e.g. methanol).

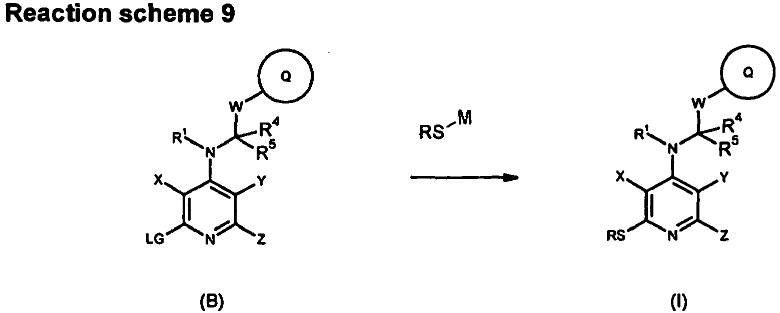

In a third example (see reaction scheme 10) a compound of formula (I), in which A is a halogen, may be prepared by reacting a metal halide or metalloid derivative A-M (e.g. potassium fluoride, sodium iodide or bromotrimethylsilane) with a compound of formula (C) in a suitable solvent (e.g. acetonitrile or dimethyl sulfoxide).

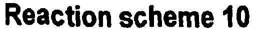

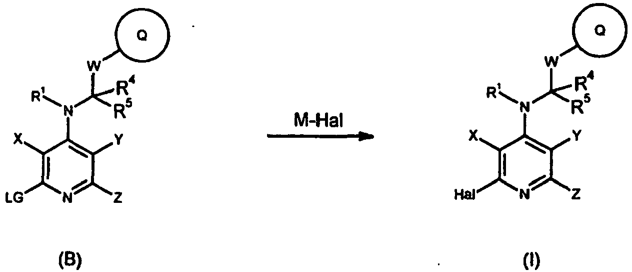

Compounds of formula (I) may be prepared from compounds of formula (C) as shown in reaction scheme 11.

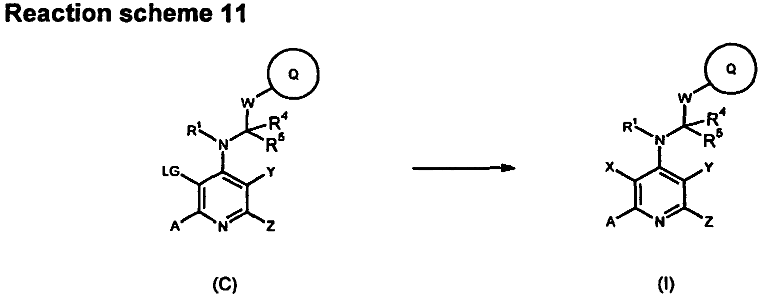

For example (see reaction scheme 12) a compound of formula (I), in which X is a group attached through a carbon atom, may be prepared by reacting a suitable metal or metalloid derivative X-M (e.g. a boronic acid or ester, a trialkyltin derivative, a zinc derivative or a Grignard reagent) with a compound of formula (C) in the presence of a suitable base (e.g. an inorganic base, such as potassium phosphate or caesium fluoride), a metal source (e.g. a palladium source, such as Pd(OAc)₂) and, optionally, a ligand for the metal (e.g. a phosphine ligand, such as PCy₃.HBF₄) in a suitable solvent (e.g. a single solvent, such as dimethylformamide, or a mixed solvent system, such as a mixture of dimethoxyethane and water or toluene and water). The metal catalyst and ligands may also be added as a single, pre-formed, complex (e.g. a palladium/phosphine complex, such as bis(triphenylphosphine)palladium dichloride or [1,1'-bis(diphenylphosphino)ferrocene] palladium dichloride dichloromethane adduct).

In a second example (see reaction scheme 12) a compound of formula (I), in which X is a cyano group, may be prepared by reacting a metal cyanide (e.g. copper(I) cyanide) with a compound of formula (C) in a suitable solvent (e.g. dimethylformamide or N-methylpyrrolidone). This transformation may also be performed in the presence of a suitable metal (e.g. palladium) catalyst, optionally complexed by any suitable ligands (e.g. phosphine ligands, such as 1,1'-bis(diphenylphosphino)ferrocene).

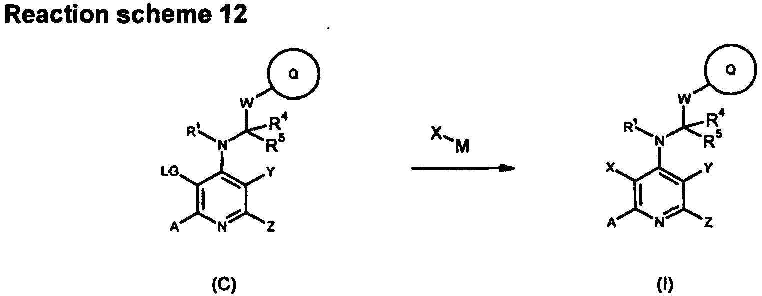

In a third example (see reaction scheme 13) a compound of formula (I), in which X is a group attached through a nitrogen atom, may be prepared by reacting a suitable amine (e.g. propylamine) with a compound of formula (C) in the presence of a suitable base (e.g. an inorganic base, such as sodium tert-butoxide), a metal source (e.g. a palladium source, such as Pd₂(dba)₃) and, optionally, a ligand for the metal (e.g. a phosphine ligand, such as JohnPhos) in a suitable solvent (e.g. toluene).

In a fourth example (see reaction scheme 13) a compound of formula (I), in which X is a group attached through a phosphorous atom, may be prepared by reacting a suitable phosphine or phosphonate (e.g. diphenylphosphine or diethylphosphite) with a compound of formula (C) in the presence of a suitable base (e.g. triethylamine), and a metal source (e.g. a palladium source, such as Pd(OAc)₂) and, optionally, a ligand for the metal (e.g. a phosphine ligand, such as triphenylphosphine) in a suitable solvent (e.g. toluene).

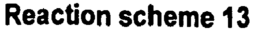

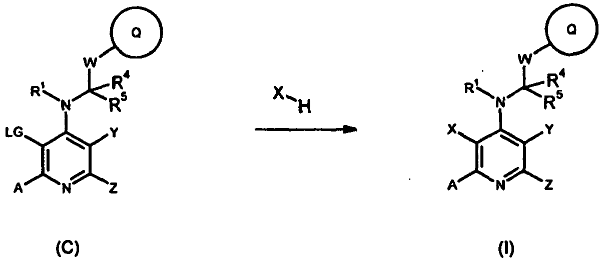

In a fifth example (see reaction scheme 14) a compound of formula (I), in which X is a hydroxyl group, may be prepared by reacting a suitable organometallic reagent (e.g. n-butyllithium) with a compound of formula (C) in a suitable solvent (e.g. tetrahydrofuran) followed by a treatment with an alkylborane (e.g. trisisopropoxyborane), and ultimately exposed to an oxidative work up (e.g. hydrogen peroxide in aqueous base, such as sodium hydroxide).

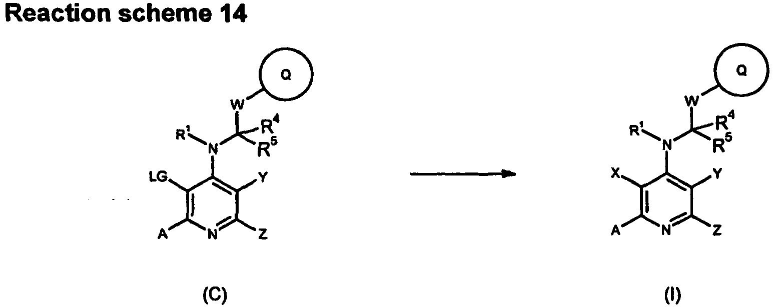

Compounds of formula (C) may be prepared from compounds of formula (D) as shown in reaction scheme 15.

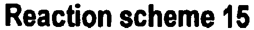

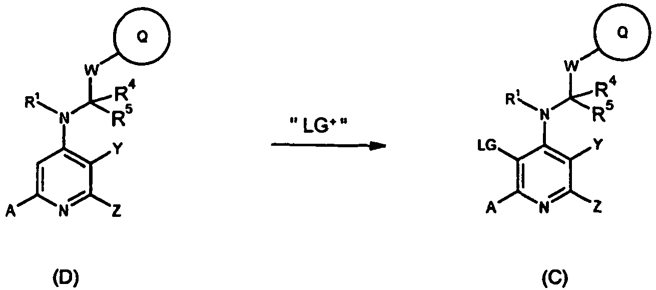

For example a compound of formula (C) in which LG is a halogen may be prepared from a compound of formula (D) by reaction with a halogenating agent (e.g. Selectfluor®, an *N-*halosuccinimide such as N-chlorosuccinimide or N-iodosuccinimide, or an elemental halogen such as bromine) in a suitable solvent (e.g. acetonitrile). This transformation may also be performed by first deprotonating compound of formula (D) with a suitable base (e.g. LDA or ZnTMP), followed by a reaction with a halogenating agent (e.g. Selectfluor®, an N-halosuccinimide such as *N*-chlorosuccinimide or *N*-iodosuccinimide, or an elemental halogen such as bromine).

Compounds of formula (I) may be prepared from compounds of formula (E) as shown in reaction scheme 16.

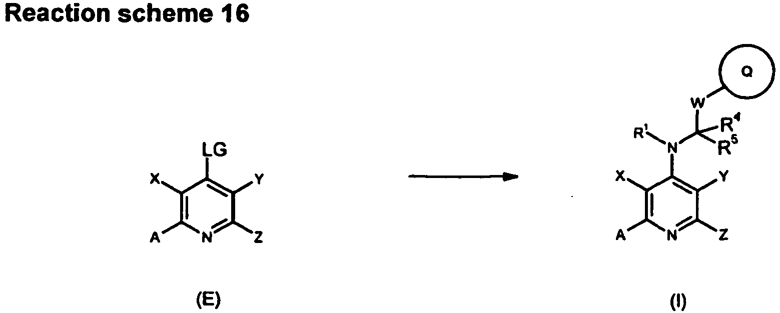

For example, a compound of formula (I) may be prepared from a compound of formula (E) by reaction with a reagent R¹(QWCR⁴R⁵)N-H or its salt (e.g. a hydrogen halide salt, such as R¹(QWCR⁴R⁵)NH₂Cl) in the presence of a suitable base (e.g. an organic base, such as triethylamine), in a suitable solvent (e.g. an organic solvent, such as dimethylformamide).

One skilled in the art will realise that it is often possible to alter the order in which the transformations described above are conducted, or to combine them in alternative ways to prepare a wide range of compounds of formula (I). All such variations are contemplated within the scope of the invention.

The skilled man will also be aware that some reagents will be incompatible with certain values or combinations of the substituents A, R¹, R⁴, R⁵, W, Q, X, Y and Z as defined herein, and any additional steps, such as protection and/or deprotection steps, which are necessary to achieve the desired transformation will be clear to the skilled man.

Compounds of formula (I) may be used in unmodified form, i.e. as obtainable from synthesis, but preferably are formulated in any suitable manner using formulation adjuvants, such as carriers, solvents and surface-active substances, for example, as described hereinafter.

The formulations can be in various physical forms, e.g. in the form of dusting powders, gels, wettable powders, water-dispersible granules, water-dispersible tablets, effervescent pellets, emulsifiable concentrates, microemulsifiable concentrates, oil-in-water emulsions, oil-flowables, aqueous dispersions, oily dispersions, suspo-emulsions, capsule suspensions, suspension concentrates, emulsifiable granules, soluble liquids, water-soluble concentrates (with water or a water-miscible organic solvent as carrier), impregnated polymer films or in other forms known e.g. from the Manual on Development and Use of FAO Specifications for Plant Protection Products, 5th Edition, 1999. The formulations can be in the form of concentrates which are diluted prior to use, although ready-to-use formulations can also be made. The dilutions can be made, for example, with water, liquid fertilisers, micronutrients, biological organisms, oil or solvents.

The formulations can be prepared e.g. by mixing the active ingredient with the formulation adjuvants in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof. The active ingredients can also be contained in very fine microcapsules consisting of a polymer. Microcapsules usually have a diameter of from 0.1 to 500 microns. Typically, they will contain active ingredients in an amount of about from 25 to 95% by weight of the capsule weight. The active ingredients can be in the form of a monolithic solid, in the form of fine particles in solid or liquid dispersion or in the form of a suitable solution. The encapsulating membranes comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other known polymers. Alternatively, very fine microcapsules can be formed in which the active ingredient is contained in the form of finely divided particles in a solid matrix of base substance, but the microcapsules are not themselves encapsulated.

The formulation adjuvants that are suitable for the preparation of compositions according to the invention are known *per se.* As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, N,N-dimethylformamide, dimethyl sulfoxide, 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alpha-pinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxypropanol, methyl isoamyl ketone, methyl isobutyl ketone, methyl laurate, methyl octanoate, methyl oleate, methylene chloride, m-xylene, *n*-hexane, n-octylamine, octadecanoic acid, octylamine acetate, oleic acid, oleylamine, o-xylene, phenol, polyethylene glycol (PEG), propionic acid, propyl lactate, propylene carbonate, propylene glycol, propylene glycol methyl ether, p-xylene, toluene, triethyl phosphate, triethylene glycol, xylenesulfonic acid, paraffin, mineral oil, trichloroethylene, perchloroethylene, amyl acetate, butyl acetate, propylene glycol methyl ether, methanol, ethanol, isopropanol, and alcohols of higher molecular weight, such as amyl alcohol, tetrahydrofurfuryl alcohol, hexanol, octanol, N-methyl-2-pyrrolidone and the like. Water is generally the carrier of choice for diluting the concentrates. Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances, as described, for example, in CFR 180.1001. (c) & (d).

A large number of surface-active substances may advantageously be used in the formulations, especially in those formulations designed to be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate; salts of alkylarylsulfonates, such as calcium dodecylbenzenesulfonate; alkylphenol/alkylene oxide addition products, such as nonylphenol ethoxylate; alcohol/alkylene oxide addition products, such as tridecylalcohol ethoxylate; soaps, such as sodium stearate; salts of alkylnaphthalenesulfonates, such as sodium dibutylnaphthalenesulfonate; dialkyl esters of sulfosuccinate salts, such as sodium di(2-ethylhexyl)sulfosuccinate; sorbitol esters, such as sorbitol oleate; quaternary amines, such as lauryltrimethylammonium chloride, polyethylene glycol esters of fatty acids, such as polyethylene glycol stearate; block copolymers of ethylene oxide and propylene oxide; and salts of mono- and di- alkylphosphate esters; and also further substances described e.g. in "McCutcheon's Detergents and Emulsifiers Annual" MC Publishing Corp., Ridgewood New Jersey, 1981.

Further adjuvants that can usually be used in pesticidal formulations include crystallisation inhibitors, viscosity modifiers, suspending agents, dyes, antioxidants, foaming agents, light absorbers, mixing auxiliaries, antifoams, complexing agents, neutralising or pH-modifying substances and buffers, corrosion inhibitors, fragrances, wetting agents, take-up enhancers, micronutrients, plasticisers, glidants, lubricants, dispersants, thickeners, antifreezes, microbicides, and also liquid and solid fertilisers.

The compositions according to the invention can additionally include an additive comprising an oil of vegetable or animal origin, a mineral oil, alkyl esters of such oils or mixtures of such oils and oil derivatives. The amount of oil additive in the composition according to the invention is generally from 0.01 to 10%, based on the spray mixture. For example, the oil additive can be added to the spray tank in the desired concentration after the spray mixture has been prepared. Preferred oil additives comprise mineral oils or an oil of vegetable origin, for example rapeseed oil, olive oil or sunflower oil, emulsified vegetable oil, such as AMIGO® (Rhône-Poulenc Canada Inc.), alkyl esters of oils of vegetable origin, for example the methyl derivatives, or an oil of animal origin, such as fish oil or beef tallow. A preferred additive contains, for example, as active components essentially 80% by weight alkyl esters of fish oils and 15% by weight methylated rapeseed oil, and also 5% by weight of customary emulsifiers and pH modifiers. Especially preferred oil additives comprise alkyl esters of C₈₋₂₂ fatty acids, especially the methyl derivatives of C₁₂₋₁₈ fatty acids, for example the methyl esters of lauric acid, palmitic acid and oleic acid, being of importance. Those esters are known as methyl laurate (CAS-111-82-0), methyl palmitate (CAS-112-39-0) and methyl oleate (CAS-112-62-9). A preferred fatty acid methyl ester derivative is Emery® 2230 and 2231 (Cognis GmbH). Those and other oil derivatives are also known from the Compendium of Herbicide Adjuvants, 5th Edition, Southern Illinois University, 2000. Another preferred adjuvant is Adigor® (Syngenta AG) which is a methylated rapeseed oil-based adjuvant.

The application and action of the oil additives can be further improved by combination with surface-active substances, such as non-ionic, anionic or cationic surfactants. Examples of suitable anionic, non-ionic and cationic surfactants are listed on pages 7 and 8 of WO97/34485. Preferred surface-active substances are anionic surfactants of the dodecylbenzylsulfonate type, especially the calcium salts thereof, and also non-ionic surfactants of the fatty alcohol ethoxylate type. Special preference is given to ethoxylated C₁₂₋₂₂ fatty alcohols having a degree of ethoxylation of from 5 to 40. Examples of commercially available surfactants are the Genapol types (Clariant AG). Also preferred are silicone surfactants, especially polyalkyl-oxide-modified heptamethyltriloxanes which are commercially available e.g. as Silwet L-77®, and also perfluorinated surfactants. The concentration of the surface-active substances in relation to the total additive is generally from 1 to 30% by weight. Examples of oil additives consisting of mixtures of oil or mineral oils or derivatives thereof with surfactants are Edenor ME SU®, Turbocharge® (Syngenta AG, CH) or ActipronC (BP Oil UK Limited, GB).

If desired, it is also possible for the mentioned surface-active substances to be used in the formulations on their own, that is to say without oil additives.

Furthermore, the addition of an organic solvent to the oil additive/surfactant mixture may contribute to an additional enhancement of action. Suitable solvents are, for example, Solvesso® (ESSO) or Aromatic Solvent® (Exxon Corporation). The concentration of such solvents can be from 10 to 80% by weight of the total weight. Oil additives that are present in admixture with solvents are described, for example, in US-A-4,834,908. A commercially available oil additive disclosed therein is known by the name MERGE® (BASF Corporation). A further oil additive that is preferred according to the invention is SCORE®(Syngenta Crop Protection Canada).

In addition to the oil additives listed above, for the purpose of enhancing the action of the compositions according to the invention it is also possible for formulations of alkylpyrrolidones (e.g. Agrimax®) to be added to the spray mixture. Formulations of synthetic lattices, e.g. polyacrylamide, polyvinyl compounds or poly-1-p-menthene (e.g. Bond®, Courier® or Emerald®) may also be used. It is also possible for solutions that contain propionic acid, for example Eurogkem Pen-e-trate®, to be added to the spray mixture as action-enhancing agent.

Herbicidal compositions of the invention generally comprise from 0.1 to 99% by weight, especially from 0.1 to 95% by weight, compounds of formula (I) and from 1 to 99.9% by weight of a formulation adjuvant which preferably includes from 0 to 25% by weight of a surface-active substance. Whereas commercial products will preferably be formulated as concentrates, the end user will normally employ dilute formulations.

Examples of preferred formulation types and their typical compositions are given below (% is percent by weight). Wettable powders as described herein are one particularly preferred type of formulation for use in the invention. In other preferred embodiments, in particular where the compound/composition/formulation of the invention is intended for use on turf, granular (inert or fertiliser) formulations as described herein are particularly suitable.

### Emulsifiable concentrates:

| | |
|---|---|
| active ingredient: | 1 to 95%, preferably 60 to 90% |
| surface-active agent: | 1 to 30%, preferably 5 to 20% |
| liquid carrier: | 1 to 80%, preferably 1 to 35% |

Dusts:

| | |
|---|---|
| active ingredient: | 0.1 to 10%, preferably 0.1 to 5% |
| solid carrier: | 99.9 to 90%, preferably 99.9 to 99% |

### Suspension concentrates:

| | |
|---|---|
| active ingredient: | 5 to 75%, preferably 10 to 50% |
| water: | 94 to 24%, preferably 88 to 30% |
| surface-active agent: | 1 to 40%, preferably 2 to 30% |

### Wettable powders:

| | |
|---|---|
| active ingredient: | 0.5 to 90%, preferably 1 to 80% |
| surface-active agent: | 0.5 to 20%, preferably 1 to 15% |
| solid carrier: | 5 to 95%, preferably 15 to 90% |

### Granules:

| | |
|---|---|
| active ingredient: | 0.1 to 30%, preferably 0.1 to 15% |
| solid carrier: | 99.5 to 70%, preferably 97 to 85% |

The following Examples further illustrate, but do not limit, the invention.

### Formulation Examples for herbicides of formula (I) (% =% by weight)

| F1. Emulsifiable concentrates | a) | b) | c) | d) |
|---|---|---|---|---|
| active ingredient | 5% | 10% | 25% | 50% |
| calcium dodecylbenzenesulfonate | 6% | 8% | 6% | 8% |
| castor oil polyglycol ether | 4% | - | 4% | 4% |
| (36 mol of ethylene oxide) | | | | |
| octylphenol polyglycol ether | - | 4% | - | 2% |
| (7-8 mol of ethylene oxide) | | | | |
| N-methyl pyrrolidone | - | - | 10% | 20% |
| arom. hydrocarbon mixture | 85% | 78% | 55% | 16% |
| (C₉-C₁₂) | | | | |

Emulsions of any desired concentration can be obtained from such concentrates by dilution with water.

| F2. Solutions | a) | b) | c) | d) |
|---|---|---|---|---|
| active ingredient | 5% | 10% | 50% | 90% |
| 1-methoxy-3-(3-methoxy- | | | | |
| propoxy)-propane | - | 20% | 20% | - |
| polyethylene glycol MW 400 | 20% | 10% | - | - |
| NMP | - | - | 30% | 10% |
| arom. hydrocarbon mixture | 75% | 60% | - | - |
| (C₉-C₁₂) | | | | |

The solutions are suitable for use in the form of microdrops.

| F3. Wettable powders | a) | b) | c) | d) |
|---|---|---|---|---|
| active ingredient | 5% | 25% | 50% | 80% |
| sodium lignosulfonate | 4% | | 3% | - |
| sodium lauryl sulphate | 2% | 3% | - | 4% |
| sodium diisobutylnaphthalene- | | | | |
| sulfonate | - | 6% | 5% | 6% |
| octylphenol polyglycol ether | - | 1% | 2% | - |
| (7-8 mol of ethylene oxide) | | | | |
| highly dispersed silicic acid | 1% | 3% | 5% | 10% |
| kaolin | 88% | 62% | 35% | - |

The active ingredient is mixed thoroughly with the adjuvants and the mixture is thoroughly ground in a suitable mill, affording wettable powders which can be diluted with water to give suspensions of any desired concentration.

| F4. Coated granules | a) | b) | c) |
|---|---|---|---|
| active ingredient | 0.1 % | 5% | 15% |
| highly dispersed silicic acid | 0.9% | 2% | 2% |
| inorganic carrier | 99.0% | 93% | 83% |
| (diameter 0.1 - 1 mm) | | | |
| e.g. CaCO₃ or SiO₂ | | | |

The active ingredient is dissolved in methylene chloride and applied to the carrier by spraying, and the solvent is then evaporated off *in vacuo.*

| F5. Coated granules | a) | b) | c) |
|---|---|---|---|
| active ingredient | 0.1 % | 5% | 15% |
| polyethylene glycol MW 200 | 1.0% | 2% | 3% |
| highly dispersed silicic acid | 0.9% | 1% | 2% |
| inorganic carrier | 98.0% | 92% | 80% |
| (diameter 0.1 - 1 mm) | | | |
| e.g. CaCO₃ or SiO₂ | | | |

The finely ground active ingredient is uniformly applied, in a mixer, to the carrier moistened with polyethylene glycol. Non-dusty coated granules are obtained in this manner.

| F6. Extruder granules | a) | b) | c) | d) |
|---|---|---|---|---|
| active ingredient | 0.1 % | 3% | 5% | 15% |
| sodium lignosulfonate | 1.5% | 2% | 3% | 4% |
| carboxymethylcellulose | 1.4% | 2% | 2% | 2% |
| kaolin | 97.0% | 93% | 90% | 79% |

The active ingredient is mixed and ground with the adjuvants, and the mixture is moistened with water. The mixture is extruded and then dried in a stream of air.

| F7. Dusts | a) | b) | c) |
|---|---|---|---|
| active ingredient | 0.1% | 1% | 5% |
| talcum | 39.9% | 49% | 35% |
| kaolin | 60.0% | 50% | 60% |

Ready-to-use dusts are obtained by mixing the active ingredient with the carriers and grinding the mixture in a suitable mill.

| F8. Suspension concentrates | a) | b) | c) | d) |
|---|---|---|---|---|
| active ingredient | 3% | 10% | 25% | 50% |
| ethylene glycol | 5% | 5% | 5% | 5% |
| nonylphenol polyglycol ether | | | | |
| (15 mol of ethylene oxide) | - | 1% | 2% | - |
| sodium lignosulfonate | 3% | 3% | 4% | 5% |
| carboxymethylcellulose | 1% | 1% | 1% | 1% |
| 37% aqueous formaldehyde | | | | |
| solution | 0.2 % | 0.2% | 0.2% | 0.2% |
| silicone oil emulsion | 0.8% | 0.8% | 0.8% | 0.8 % |
| water | 87% | 79% | 62% | 38% |

The finely ground active ingredient is intimately mixed with the adjuvants, giving a suspension concentrate from which suspensions of any desired concentration can be obtained by dilution with water.

Compounds of the invention (as well as mixtures and/or formulations containing the same) find utility as herbicides, and may thus be employed in methods of controlling plant growth. Such methods involve applying to the plants or to the locus thereof a herbicidally effective amount of said compound, or composition comprising the same (or mixture as described hereinafter). The invention thus also relates to a method of inhibiting plant growth which comprises applying to the plants or to the locus thereof a herbicidally effective amount of a compound of formula (I), composition, or mixture of the invention. In particular the invention provides a method of controlling weeds in crops of useful plants, which comprising applying to said weeds or the locus of said weeds, or to said crop of useful plants, a compound of formula I or a composition or mixture containing the same.

The term "locus" as used herein includes not only areas where weeds may already be growing, but also areas where weeds have yet to emerge, and also to areas under cultivation with respect to crops of useful plants. Areas under cultivation include land on which the crop plants are already growing and land intended for cultivation with such crop plants.

A compound, composition, and/or mixture of the invention may be used in a pre-emergence application and/or in a post-emergence application in order to mediate its effect.

Crops of useful plants in which compounds of formula (I), as well as formulations and/or mixtures containing the same, may be used according to the invention include perennial crops, such as citrus fruit, grapevines, nuts, oil palms, olives, pome fruit, stone fruit and rubber, and annual arable crops, such as cereals, for example barley and wheat, cotton, oilseed rape, maize, rice, soy beans, sugar beet, sugar cane, sunflowers, ornamentals and vegetables, especially cereals and maize.

Compounds of formula (I), formulations and/or mixtures containing the same may also be used on turf, pasture, rangeland, rights of way etc. In particular they may be used on golf-courses, lawns, parks, sports-fields, race-courses and the like.

Crops are to be understood as also including those crops which have been rendered tolerant to herbicides or classes of herbicides (e.g. ALS-, GS-, EPSPS-, PPO- and HPPD-inhibitors and synthetic auxins) by conventional methods of breeding or by genetic engineering. An example of a crop that has been rendered tolerant to imidazolinones, e.g. imazamox, by conventional methods of breeding is Clearfield® summer rape (canola). Examples of crops that have been rendered tolerant to herbicides by genetic engineering methods include e.g. glyphosate- and glufosinate-resistant maize varieties commercially available under the trade names RoundupReady® and LibertyLink®.

Crops are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK® (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut® (maize), Yield Gard® (maize), NuCOTIN33B® (cotton), Bollgard® (cotton), NewLeaf® (potatoes), NatureGard® and Protexcta®. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crops are also to be understood as being those which are obtained by conventional methods of breeding or genetic engineering and contain so-called output traits (e.g. improved storage stability, higher nutritional value and improved flavour).

The term "weeds" as used herein means any undesired plant, and thus includes not only agronomically important weeds as described below, but also volunteer crop plants.

Compounds of formula (I) may be used against a large number of agronomically important weeds. The weeds that may be controlled include both monocotyledonous and dicotyledonous weeds, such as, for example, *Alisma spp, Leptochloa chinensis, Stellaria, Nasturtium, Agrostis, Digitaria, Avena, Setaria, Sinapis, Lolium, Solanum, Echinochloa, Scirpus, Monochoria, Sagittaria, Bromus, Alopecurus, Sorghum, Rottboellia, Cyperus and especially Cyperus iria, Abutilon, Sida, Xanthium, Amaranthus, Chenopodium, Ipomoea, Chrysanthemum, Galium, Viola, Veronica,* and *Ischaemum* spp.

The rates of application of compounds of formula (I) may vary within wide limits and depend on the nature of the soil, the method of application (pre- or post-emergence; seed dressing; application to the seed furrow; no tillage application etc.), the crop plant, the weed to be controlled, the prevailing climatic conditions, and other factors governed by the method of application, the time of application and the target crop. The compounds of formula I according to the invention are generally applied at a rate of from 10 to 2000 g/ha, especially from 25 to 1000 g/ha.

Any method of application to weeds/crop of useful plant, or locus thereof, which is routinely used in agriculture may be used, for example application by spray or broadcast method typically after suitable dilution of a compound of formula (I) (whether said compound is formulated and/or in combination with one or more further active ingredients and/or safeners, as described herein).

The compounds of formula (I) according to the invention can also be used in combination with other active ingredients, e.g. other herbicides, and/or insecticides, and/or acaricides, and/or nematocides, and/or molluscicides, and/or fungicides, and/or plant growth regulators. Such mixtures, and the use of such mixtures to control weeds and/or undesired plant growth form yet further aspects of the invention. For the avoidance of doubt, mixtures of invention also include mixtures of two or more different compounds of formula (I).

Where a compound of formula (I) is combined with at least one additional herbicide, the following mixtures of the compound of formula (I) are particularly preferred. Compound of formula (I) + acetochlor, compound of formula (I) + acifluorfen, compound of formula (I) + acifluorfen-sodium, compound of formula (I) + aclonifen, compound of formula (I) + acrolein, compound of formula (I) + alachlor, compound of formula (I) + alloxydim, compound of formula (I) + allyl alcohol, compound of formula (I) + ametryn, compound of formula (I) + amicarbaione, compound of formula (I) + amidosulfuron, compound of formula (I) + aminocyclopyrachlor, compound of formula (I) + aminopyralid, compound of formula (I) + amitrole, compound of formula (I) + ammonium sulfamate, compound of formula (I) + anilofos, compound of formula (I) + asulam, compound of formula (I) + atrazine, formula (I) + aviglycine, formula (I) + azafenidin, compound of formula (I) + azimsulfuron, compound of formula (I) + BCPC, compound of formula (I) + beflubutamid, compound of formula (I) + benazolin, formula (I) + bencarbazone, compound of formula (I) + benfluralin, compound of formula (I) + benfuresate, compound of formula (I) + bensulfuron, compound of formula (I) + bensulfuron-methyl, compound of formula (I) + bensulide, compound of formula (I) + bentazone, compound of formula (I) + benzfendizone, compound of formula (I) + benzobicyclon, compound of formula (I) + benzofenap, compound of formula (I) + bifenox, compound of formula (I) + bilanafos, compound of formula (I) + bispyribac, compound of formula (I) + bispyribac-sodium, compound of formula (I) + borax, compound of formula (I) + bromacil, compound of formula (I) + bromobutide, formula (I) + bromophenoxim, compound of formula (I) + bromoxynil, compound of formula (I) + butachlor, compound of formula (I) + butafenacil, compound of formula (I) + butamifos, compound of formula (I) + butralin, compound of formula (I) + butroxydim, compound of formula (I) + butylate, compound of formula (I) + cacodylic acid, compound of formula (I) + calcium chlorate, compound of formula (I) + cafenstrole, compound of formula (I) + carbetamide, compound of formula (I) + carfentrazone, compound of formula (I) + carfentrazone-ethyl, compound of formula (I) + CDEA, compound of formula (I) + CEPC, compound of formula (I) + chlorflurenol, compound of formula (I) + chlorflurenol-methyl, compound of formula (I) + chloridazon, compound of formula (I) + chlorimuron, compound of formula (I) + chlorimuron-ethyl, compound of formula (I) + chloroacetic acid, compound of formula (I) + chlorotoluron, compound of formula (I) + chlorpropham, compound of formula (I) + chlorsulfuron, compound of formula (I) + chlorthal, compound of formula (I) + chlorthal-dimethyl, compound of formula (I) + cinidon-ethyl, compound of formula (I) + cinmethylin, compound of formula (I) + cinosulfuron, compound of formula (I) + cisanilide, compound of formula (I) + clethodim, compound of formula (I) + clodinafop, compound of formula (I) + clodinafop-propargyl, compound of formula (I) + clomazone, compound of formula (I) + clomeprop, compound of formula (I) + clopyralid, compound of formula (I) + cloransulam, compound of formula (I) + cloransulam-methyl, compound of formula (I) + CMA, compound of formula (I) + 4-CPB, compound of formula (I) + CPMF, compound of formula (I) + 4-CPP, compound of formula (I) + CPPC, compound of formula (I) + cresol, compound of formula (I) + cumyluron, compound of formula (I) + cyanamide, compound of formula (I) + cyanazine, compound of formula (I) + cycloate, compound of formula (I) + cyclosulfamuron, compound of formula (I) + cycloxydim, compound of formula (I) + cyhalofop, compound of formula (I) + cyhalofop-butyl, compound of formula (I) + 2,4-D, compound of formula (I) + 3,4-DA, compound of formula (I) + daimuron, compound of formula (I) + dalapon, compound of formula (I) + dazomet, compound of formula (I) + 2,4-DB, compound of formula (I) + 3,4-DB, compound of formula (I) + 2,4-DEB, compound of.formula (I) + desmedipham, formula (I) + desmetryn, compound of formula (I) + dicamba, compound of formula (I) + dichlobenil, compound of formula (I) + ortho-dichlorobenzene, compound of formula (I) + para-dichlorobenzene, compound of formula (I) + dichlorprop, compound of formula (I) + dichlorprop-P, compound of formula (I) + diclofop, compound of formula (I) + diclofop-methyl, compound of formula (I) + diclosulam, compound of formula (I) + difenzoquat, compound of formula (I) + difenzoquat metilsulfate, compound of formula (I) + diflufenican, compound of formula (I) + diflufenzopyr, compound of formula (I) + dimefuron, compound of formula (I) + dimepiperate, compound of formula (I) + dimethachlor, compound of formula (I) + dimethametryn, compound of formula (I) + dimethenamid, compound of formula (I) + dimethenamid-P, compound of formula (I) + dimethipin, compound of formula (I) + dimethylarsinic acid, compound of formula (I) + dinitramine, compound of formula (I) + dinoterb, compound of formula (I) + diphenamid, formula (I) + dipropetryn, compound of formula (I) + diquat, compound of formula (I) + diquat dibromide, compound of formula (I) + dithiopyr, compound of formula (I) + diuron, compound of formula (I) + DNOC, compound of formula (I) + 3,4-DP, compound of formula (I) + DSMA, compound of formula (I) + EBEP, compound of formula (I) + endothal, compound of formula (I) + EPTC, compound of formula (I) + esprocarb, compound of formula (I) + ethalfluralin, compound of formula (I) + ethametsulfuron, compound of formula (I) + ethametsulfuron-methyl, formula (I) + ethephon, compound of formula (I) + ethofumesate, compound of formula (I) + ethoxyfen, compound of formula (I) + ethoxysulfuron, compound of formula (I) + etobenzanid, compound of formual (I) + fenoxaprop, compound of formula (I) + fenoxaprop-P, compound of formula (I) + fenoxaprop-ethyl, compound of formula (I) + fenoxaprop-P-ethyl, compound of formula (I) + fentrazamide, compound of formula (I) + ferrous sulfate, compound of formula (I) + flamprop-M, compound of formula (I) + flazasulfuron, compound of formula (I) + florasulam, compound of formula (I) + fluazifop, compound of formula (I) + fluazifop-butyl, compound of formula (I) + fluazifop-P, compound of formula (I) + fluazifop-P-butyl, formula (I) + fluazolate, compound of formula (I) + flucarbazone, compound of formula (I) + flucarbazone-sodium, compound of formula (I) + flucetosulfuron, compound of formula (I) + fluchloralin, compound of formula (I) + flufenacet, compound of formula (I) + flufenpyr, compound of formula (I) + flufenpyr-ethyl, formula (I) + flumetralin, compound of formula (I) + flumetsulam, compound of formula (I) + flumiclorac, compound of formula (I) + flumiclorac-pentyl, compound of formula (I) + flumioxazin, formula (I) + flumipropin, compound of formula (I) + fluometuron, compound of formula (I) + fluoroglycofen, compound of formula (I) + fluoroglycofen-ethyl, formula (I) + fluoxaprop, formula (I) + flupoxam, formula (I) + flupropacil, compound of formula (I) + flupropanate, compound of formula (I) + flupyrsulfuron, compound of formula (I) + flupyrsulfuron-methyl-sodium, compound of formula (I) + flurenol, compound of formula (I) + fluridone, compound of formula (I) + flurochloridone, compound of formula (I) + fluroxypyr, compound of formula (I) + flurtamone, compound of formula (I) + fluthiacet, compound of formula (I) + fluthiacet-methyl, compound of formula (I) + fomesafen, compound of formula (I) + foramsulfuron, compound of formula (I) + fosamine, compound of formula (I) + glufosinate, compound of formula (I) + glufosinate-ammonium, compound of formula (I) + glyphosate, compound of formula (I) + halosulfuron, compound of formula (I) + halosulfuron-methyl, compound of formula (I) + haloxyfop, compound of formula (I) + haloxyfop-P, compound of formula (I) + HC-252, compound of formula (I) + hexazinone, compound of formula (I) + imazamethabenz, compound of formula (I) + imazamethabenz-methyl, compound of formula (I) + imazamox, compound of formula (I) + imazapic, compound of formula (I) + imazapyr, compound of formula (I) + imazaquin, compound of formula (I) + imazethapyr, compound of formula (I) + imazosulfuron, compound of formula (I) + indanofan, compound of formula (I) + iodomethane, compound of formula (I) + iodosulfuron, compound of formula (I) + iodosulfuron-methyl-sodium, compound of formula (I) + ioxynil, compound of formula (I) + isoproturon, compound of formula (I) + isouron, compound of formula (I) + isoxaben, compound of formula (I) + isoxachlortole, compound of formula (I) + isoxaflutole, formula (I) + isoxapyrifop, compound of formula (I) + karbutilate, compound of formula (I) + lactofen, compound of formula (I) + lenacil, compound of formula (I) + linuron, compound of formula (I) + MAA, compound of formula (I) + MAMA, compound of formula (I) + MCPA, compound of formula (I) + MCPA-thioethyl, compound of formula (I) + MCPB, compound of formula (I) + mecoprop, compound of formula (I) + mecoprop-P, compound of formula (I) + mefenacet, compound of formula (I) + mefluidide, compound of formula (I) + mesosulfuron, compound of formula (I) + mesosulfuron-methyl, compound of formula (I) + mesotrione, compound of formula (I) + metam, compound of formula (I) + metamifop, compound of formula (I) + metamitron, compound of formula (I) + metazachlor, compound of formula (I) + methabenzthiazuron, formula (I) + methazole, compound of formula (I) + methylarsonic acid, compound of formula (I) + methyldymron, compound of formula (I) + methyl isothiocyanate, compound of formula (I) + metobenzuron, formula (I) + metobromuron, compound of formula (I) + metolachlor, compound of formula (I) + S-metolachlor, compound of formula (I) + metosulam, compound of formula (I) + metoxuron, compound of formula (I) + metribuzin, compound of formula (I) + metsulfuron, compound of formula (I) + metsutfuron-methyl, compound of formula (I) + MK-616, compound of formula (I) + molinate, compound of formula (I) + monolinuron, compound of formula (I) + MSMA, compound of formula (I) + naproanilide, compound of formula (I) + napropamide, compound of formula (I) + naptalam, formula (I) + NDA-402989, compound of formula (I) + neburon, compound of formula (I) + nicosulfuron, formula (I) + nipyraclofen, formula (I) + n-methyl glyphosate, compound of formula (I) + nonanoic acid, compound of formula (I) + norflurazon, compound of formula (I) + oleic acid (fatty acids), compound of formula (I) + orbencarb, compound of formula (I) + orthosulfamuron, compound of formula (I) + oryzalin, compound of formula (I) + oxadiargyl, compound of formula (I) + oxadiazon, compound of formula (I) + oxasulfuron, compound of formula (I) + oxaziclomefone, compound of formula (I) + oxyfluorfen, compound of formula (I) + paraquat, compound of formula (I) + paraquat dichloride, compound of formula (I) + pebulate, compound of formula (I) + pendimethalin, compound of formula (I) + penoxsulam, compound of formula (I) + pentachlorophenol, compound of formula (I) + pentanochlor, compound of formula (I) + pentoxazone, compound of formula (I) + pethoxamid, compound of formula (I) + petrolium oils, compound of formula (I) + phenmedipham, compound of formula (I) + phenmedipham-ethyl, compound of formula (I) + picloram, compound of formula (I) + picolinafen, compound of formula (I) + pinoxaden, compound of formula (I) + piperophos, compound of formula (I) + potassium arsenite, compound of formula (I) + potassium azide, compound of formula (I) + pretilachlor, compound of formula (I) + primisulfuron, compound of formula (I) + primisulfuron-methyl, compound of formula (I) + prodiamine, compound of formula (I) + profluazol, compound of formula (I) + profoxydim, formula (I) + prohexadione-calcium, compound of formula (I) + prometon, compound of formula (I) + prometryn, compound of formula (I) + propachlor, compound of formula (I) + propanil, compound of formula (I) + propaquizafop, compound of formula (I) + propazine, compound of formula (I) + propham, compound of formula (I) + propisochlor, compound of formula (I) + propoxycarbazone, compound of formula (I) + propoxycarbazone-sodium, compound of formula (I) + propyzamide, compound of formula (I) + prosulfocarb, compound of formula (I) + prosulfuron, compound of formula (I) + pyraclonil, compound of formula (I) + pyraflufen, compound of formula (I) + pyraflufen-ethyl, formula (I) + pyrasulfotole, compound of formula (I) + pyrazolynate, compound of formula (I) + pyrazosulfuron, compound of formula (I) + pyrazosulfuron-ethyl, compound of formula (I) + pyrazoxyfen, compound of formula (I) + pyribenzoxim, compound of formula (I) + pyributicarb, compound of formula (I) + pyridafol, compound of formula (I) + pyridate, compound of formula (I) + pyriftalid, compound of formula (I) + pyriminobac, compound of formula (I) + pyriminobacmethyl, compound of formula (I) + pyrimisulfan, compound of formula (I) + pyrithiobac, compound of formula (I) + pyrithiobac-sodium, formula (I) + pyroxasulfone, formula (I) + pyroxulam, compound of formula (I) + quinclorac, compound of formula (I) + quinmerac, compound of formula (I) + quinoclamine, compound of formula (I) + quizalofop, compound of formula (I) + quizalofop-P, compound of formula (I) + quizalofop-ethyl, compound of formula (I) + quizalofop-P-ethyl, compound of formula (I) + rimsulfuron, compound of formula (I) + saflufenacil, compound of formula (I) + sethoxydim, compound of formula (I) + siduron, compound of formula (I) + simazine, compound of formula (I) + simetryn, compound of formula (I) + SMA, compound of formula (I) + sodium arsenite, compound of formula (I) + sodium azide, compound of formula (I) + sodium chlorate, compound of formula (I) + sulcotrione, compound of formula (I) + sulfentrazone, compound of formula (I) + sulfometuron, compound of formula (I) + sulfometuron-methyl, compound of formula (I) + sulfosate, compound of formula (I) + sulfosulfuron, compound of formula (I) + sulfuric acid, compound of formula (I) + tar oils, compound of formula (I) + 2,3,6-TBA, compound of formula (I) + TCA, compound of formula (I) + TCA-sodium, formula (I) + tebutam, compound of formula (I) + tebuthiuron, formula (I) + tefuryltrione, compound of formula 1 + tembotrione, compound of formula (I) + tepraloxydim, compound of formula (I) + terbacil, compound of formula (I) + terbumeton, compound of formula (I) + terbuthylazine, compound of formula (I) + terbutryn, compound of formula (I) + thenylchlor, compound of formula (I) + thiazafluron, compound of formula (I) + thiazopyr, compound of formula (I) + thifensulfuron, compound of formula (I) + thiencarbazone, compound of formula (I) + thifensulfuron-methyl, compound of formula (I) + thiobencarb, compound of formula (I) + tiocarbazil, compound of formula (I) + topramezone, compound of formula (I) + tralkoxydim, compound of formula (I) + tri-allate, compound of formula (I) + triasulfuron, compound of formula (I) + triaziflam, compound of formula (I) + tribenuron, compound of formula (I) + tribenuron-methyl, compound of formula (I) + tricamba, compound of formula (I) + triclopyr, compound of formula (I) + trietazine, compound of formula (I) + trifloxysulfuron, compound of formula (I) + trifloxysulfuron-sodium, compound of formula (I) + trifluralin, compound of formula (I) + triflusulfuron, compound of formula (I) + triflusulfuron-methyl, compound of formula (I) + trifop, compound of formula (I) + trifop-methyl, compound of formula (I) + trihydroxytriazine, compound of formula (I) + trinexapac-ethyl, compound of formula (I) + tritosulfuron, compound of formula (I) + [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetic acid ethyl ester (CAS RN 353292-31-6), compound of formula (I) + 4-hydroxy-3-[[2-[(2-methoxyethoxy)methyl]-6-(trifluoromethyl)-3-pyridinyl]carbonyl]-bicyclo[3.2.1]oct-3-en-2-one (CAS RN 352010-68-5), compound of formula (I) + 4-amino-3-chloro-6-(4-chloro-2-fluoro-3-methoxyphenyl)-2-pyridinecarboxylic acid (CAS RN 943832-60-8), and compound of formula (I) + 4-hydroxy-3-[[2-(3-methoxypropyl)-6-(difluoromethyl)-3-pyridinyl]carbonyl]-bicyclo[3.2.1]oct-3-en-2-one.

Whilst two-way mixtures of a compound of formula (I) and another herbicide are explicitly disclosed above, the skilled man will appreciate that the invention extends to three-way, and further multiple combinations comprising the above two-way mixtures.

In preferred embodiments a compound of formula (I) is combined with an acetolactate synthase inhibitor, (e.g. one or more of florasulam, metsulfuron, nicosulfuron, prosulfuron, thifensulfuron, tribenuron, triasulfuron, flucarbazone, flupyrsulfuron, iodosulfuron, mesosulfuron, propoxicarbazone, sulfosulfuron, pyroxsulam and tritosulfuron, as well as salts or esters thereof), a synthetic auxin herbicide (e.g. one or more of aminocyclopyrachlor, aminopyralid, clopyralid, 2,4-D, 2,4-DB, dicamba, dichlorprop, fluroxypyr, MCPA, MCPB, mecoprop, mecoprop-P and 4-amino-3-chloro-6-(4-chloro-2-fluoro-3-methoxyphenyl)-2-pyridinecarboxylic acid (CAS RN 943832-60-8)), an ACCase-inhibiting herbicide (e.g. one or more of phenylpyrazolin; pinoxaden; an aryloxyphenoxypropionic herbicide such as clodinafop, cyhalofop, diclofop, fenoxaprop, fluazifop, haloxyfop, quizalofop, trifop and mixtures thereof, as well as the isomers thereof, for example, fenoxaprop-P, fluazifop-P, haloxyfop-P, quizalofop-P; and a cyclohexanedione herbicide such as alloxydim; butroxydim, clethodim, cycloxydim, profoxydim, sethoxydim, tepraloxydim and tralkoxydim, as well as salts or esters thereof), an auxin transport inhibitor such as semicarbazone (e.g. diflufenzopyr, in particular the sodium salt), an HPPD inhibiting herbicide (e.g. mesotrione, tembotrione, topramezone) or phthalamate compound (e.g. naptalam), and/or an EPSPS inhibitor such as glyphosate.

Particularly preferred mixture partners for compounds of formula (I) are: florasulam, iodosulfuron-methyl-sodium, mesosulfuron-methyl, metsulfuronmethyl, nicosulfuron, prosulfuron, thifensulfuron, triasulfuron, tribenuron-methyl or pyroxsulam; dicamba, fluroxypyr, MCPA, mecoprop or mecoprop-P; clodinafop-propargyl, cyhalofop-butyl, diclofop-methyl, fenoxaprop-ethyl, fenoxaprop-P-ethyl, fluazifop-butyl, fluazifop-P-butyl, haloxyfop-methyl, haloxyfop-P-methyl, pinoxaden, propaquizafop, quizalofop-ethyl, quizalofop-P-ethyl, tralkoxydim, trifop-methyl, diflufenzopyr-Na, mesotrione, topramezone, naptalam, and glyphosate.

For the avoidance of doubt, even if not explicitly stated above, the mixing partners of the compound of formula (I) may also be in the form of any suitable agrochemically acceptable ester or salt, as mentioned e.g. in The Pesticide Manual, Thirteenth Edition, British Crop Protection Council, 2003.

The mixing ratio of the compound of formula (I) to the mixing partner is preferably from 1: 100 to 1000:1.

The mixtures can advantageously be used in the above-mentioned formulations (in which case "active ingredient" relates to the respective mixture of compound of formula (I) with the mixing partner).

The compounds of formula (I) according to the invention can also be used in combination with one or more safeners. Likewise, mixtures of a compound of formula (I) according to the invention with one or more further active ingredients, in particular with one or more further herbicides, can also be used in combination with one or more safeners. Suitable safeners for use in combination with compounds of formula (I) include AD 67 (MON 4660), benoxacor, cloquintocet-mexyl, cyometrinil and the corresponding (Z) isomer, cyprosulfamide (CAS RN 221667-31-8), dichlormid, fenchlorazole-ethyl, fenclorim, flurazole, fluxofenim, furilazole and the corresponding R isomer, isoxadifen-ethyl, mefenpyr-diethyl, oxabetrinil, naphthalic anhydride (CAS RN 81-84-5), N-isopropyl-4-(2-methoxybenzoylsulfamoyl)-benzamide (CAS RN 221668-34-4) and N-(2-methoxybenzoyl)-4-[(methylaminocarbonyl)amino]benzenesulfonamide. Particularly preferred safeners for use in the invention are cloquintocet-mexyl, cyprosulfamide, fenchlorazole-ethyl, mefenpyr-diethyl and N-(2-methoxybenzoyl)-4-[(methylaminocarbonyl)amino]benzenesulfonamide. The safeners of the compound of formula (I) may also be in the form of esters or salts, as mentioned e.g. in The Pesticide Manual, 13^{th} Edition *supra.* The reference to cloquintocet-mexyl also applies to a lithium, sodium, potassium, calcium, magnesium, aluminium, iron, ammonium, quaternary ammonium, sulfonium or phosphonium salt thereof as disclosed in WO02/34048, and the reference to fenchlorazole-ethyl also applies to fenchlorazole, etc.

Preferably the mixing ratio of compound of formula (I) to safener is from 100:1 to 1:10, especially from 20:1 to 1:1.

The mixtures can advantageously be used in the above-mentioned formulations (in which case "active ingredient" relates to the respective mixture of compound of formula (I) with the safener).

Preferred mixtures of a compound of formula (I) with further herbicides and safeners include: a compound of formula (I) + pinoxaden + cloquinctocet-mexyl, a compound of formula (I) + clodinafop + cloquintocet-mexyl, and a compound of formula (I) + clodinafop-propargyl + cloquintocet-mexyl.

Various aspects and embodiments of the present invention will now be illustrated in more detail by way of example. It will be appreciated that modification of detail may be made without departing from the scope of the invention.

### EXAMPLES

### EXAMPLE 1

### Synthesis of 3-bromo-6-chloro-pyridine-2-carboxylic acid methyl ester.

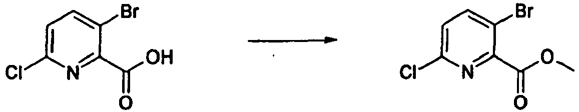

3-Bromo-6-chloro-pyridine-2-carboxylic acid (50.00 g, 211.5 mmol) was dissolved in methanol (235 mL), and concentrated sulphuric acid (5.6 mL) was added. The resulting reaction mixture was heated at reflux for 28 h. The reaction mixture was cooled to room temperature, and the resulting precipitate was recrystallized from methanol to give 3-bromo-6-chloro-pyridine-2-carboxylic acid methyl ester (54.38 g, quantitative) as a solid. Characterising data for the compound are as follow: ¹H NMR (400 MHz, CDCl₃) δ 7.94 (d, 1H), 7.34 (d, 1H) and 4.01 (s, 3H) ppm.

Further examples of compounds that were prepared using this method are listed below in Table 2.

**TABLE 2 Compounds made according to the method described in Example 1 above.**

| **Name** | **Structure** | **¹H NMR (400 MHz, CDCl₃) δ ppm** |
|---|---|---|
| 3,6-Dichloropyridine-2-carboxylic acid methyl ester | 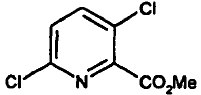 | 7.80 (d, 1H), 7.40 (d, 1H), 4.00 (s, 3H) ppm |

### EXAMPLE 2

**Synthesis of 3-bromo-4,6-dichloro-pyridine-2-carboxylic acid methyl ester.**

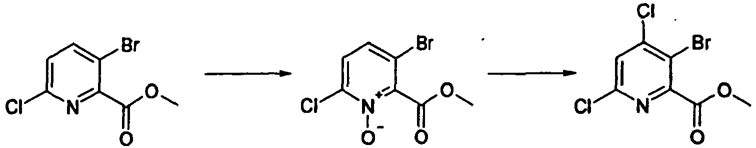

Urea hydrogen peroxide (65.32 g, 347.2 mmol) was added portionwise to a solution of trifluoroacetic anhydride (145.8 g, 694.4 mmol, 96.5 mL) in dichloromethane (577 mL) at 0 °C. 3-Bromo-6-chloro-pyridine-2-carboxylic acid methyl ester (prepared as described in Example 1) (27.18 g, 108.5 mmol) was added to the mixture portionwise and the reaction mixture was stirred at room temperature for 19 h. The reaction was quenched by the addition of water, the phases separated and the organic layer washed with water and saturated aqueous potassium carbonate. The organic layer was dried over magnesium sulphate, filtered and concentrated under reduced pressure to give 3-bromo-6-chloro-pyridine-2-carboxylic acid N-oxide as a yellow oil. Characterising data for the compound are as follows: ¹H NMR (400 MHz, CD₃OD) δ 7.77-7.75 (m, 2H) and 4.01 (s, 3H) ppm.

3-Bromo-6-chloro-pyridine-2-carboxylic acid *N*-oxide was dissolved in phosphorus oxychloride (166 g, 1.085 mol, 101 mL) and stirred at room temperature for 2 h, then at reflux for 3 h. The reaction mixture was cooled, concentrated under reduced pressure, and purified by silica gel chromatography (gradient elution: 0-100% ethyl acetate in iso-hexane) followed by reverse phase silica gel chromatography (gradient elution: 0-100% methanol in water) to give 3-bromo-4,6-dichloro-pyridine-2-carboxylic acid methyl ester (9.45 g, 31%) as a solid. Characterising data for the compound are as follows: ¹H NMR (400 MHz, CDCl₃) δ 7.57 (s, 1H) and 4.01 (s, 3H) ppm.

Further examples of compounds that were prepared using this method are listed below in Table 3.

**TABLE 3 Compounds made according to the method described in Example 2 above.**

| **Name** | **Structure** | **¹H NMR (400 MHz, CDCl₃) δ ppm** |
|---|---|---|
| 3,4,6-Trichloro-pyridine-2-carboxylic acid methyl ester | 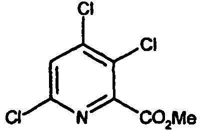 | 7.58 (s, 1H), 3.99 (s, 3H) |

### EXAMPLE 3

### Synthesis of 3,6-dichloro-4-[(furan-2-ylmethyl)-amino]-pyridine-2-carboxylic acid methyl ester (Compound 26-2).

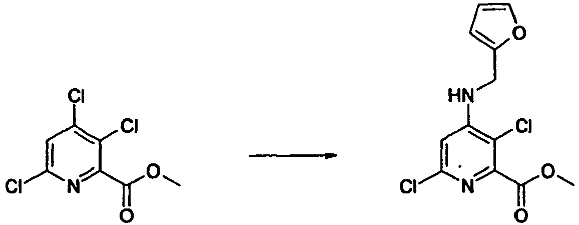

3,4,6-Trichloro-pyridine-2-carboxylic acid methyl ester (prepared as described in Example 2) (0.500 g, 2.08 mmol) was dissolved in dimethylformamide (3 mL), and triethylamine (0.58 mL, 4.17 mmol) was added. Furfurylamine (0.202 g, 2.08 mmol) dissolved in dimethylformamide (2 mL) was added to the solution, and the resulting reaction mixture was heated to 100 °C for 3 h. The reaction mixture was cooled to room temperature, poured into water and the aqueous layer was extracted with diethyl ether. The combined organic layers were washed with brine, dried over magnesium sulphate and evaporated under reduced pressure. The resulting residue was purified by silica gel chromatography (gradient elution: 0 - 40% ethyl acetate in *iso*-hexane) to give 3,6-dichloro-4-[(furan-2-ylmethyl)-amino]-pyridine-2-carboxylic acid methyl ester (221 mg, 35%) as a solid.
Characterising data for the compound are as follows:
¹H NMR (400 MHz, CDCl₃) δ 7.42 (m, 1H), 6.72 (s, 1H), 6.37 (m, 1H), 6.32 (m, 1H), 5.55 (br. s, 1H), 4.44 (d, 2H), 3.96 (s, 3H) ppm.

Further examples of compounds that were prepared using this method are listed below in Table 4. Characteristic data provided is either ¹H-nmr data (400 MHz, CDCl₃) δ_{H} ppm or LCMS [retention time (RT, recorded in minutes) and the molecular ion, typically the cation M+H⁺]. Compounds characterised by HPLC-MS used a Waters 2777 injector with a 1525 micro pump HPLC equipped with a Waters Atlantis dC18 IS column (column length 20 mm, internal diameter of column 3 mm, particle size 3 micron), Waters 2996 photodiode array, Waters 2420 ELSD and Micromass ZQ2000. The analysis was conducted using a three minute run time, according to the following gradient table:

| Time (minutes) | Solvent A (%) | Solvent B (%) | Flow (ml / minute) |
|---|---|---|---|
| 0.00 | 95 | 5 | 1.3 |
| 2.50 | 0 | 100 | 1.3 |
| 2.80 | 0 | 100 | 1.3 |
| 2.90 | 95 | 5 | 1.3 |

| | | | |
|---|---|---|---|
| Solvent A: H₂O with 0.05% TFA Solvent B: CH₃CN with 0.05% TFA | | | |

**TABLE 4 Compounds made according to the method described in Example 3 above.**

| **Compound Number** | **Name** | **Structure** | **Characteristic data** |
|---|---|---|---|
| 33-2 | 3,6-Dichloro-4-[(3-methyl-pyridin-2-ylmethyl)-amino]-pyridine-2-carboxylic acid methyl ester | 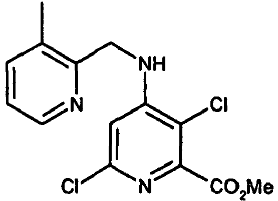 | 8.49 (d, 1H), 7.64 (br. s, 1H), 7.53 (d, 1H), 7.22 (m, 1H), 6.69 (s, 1H), 4.35 (d, 2H), 3.98 (s, 3H), 2.36 (s, 3H) |
| 1-2 | 4-Cyclopropylmethy lamino-3,6-dichloro-pyridine-2-carboxylic acid methyl ester | 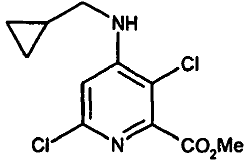 | 6.50 (s, 1H), 5.31 (br. s, 1H), 3.89 (s, 3H), 3.00 (m, 2H), 1.08 (m, 1H), 0.58 (m, 2H), 0.25 (m, 2H) |
| 2-2 | 3,6-Dichloro-4-phenylmethylami no-pyridine-2-carboxylic acid methyl ester | 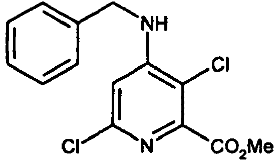 | 7.40-7.30 (m, 5H), 6.56 (s, 1H), 5.76 (m, 1H), 4.44 (d, 2H), 3.94 (s, 3H) |
| 5-2 | 4-[(4-Chlorophenylmet hyl)-amino]-3,6-dichloro-pyridine-2-carboxylic acid methyl ester | 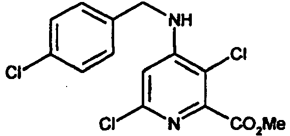 | 7.34 (d, 2H), 7.24 (d, 2H), 6.50 (s, 1H), 6.02 (m, 1H), 4.44 (d, 2H), 3.95 (s, 3H) |
| 6-2 | 3,6-Dichloro-4-[(2-fluorophenylmeth yl)-amino]-pyridine-2-carboxylic acid methyl ester | 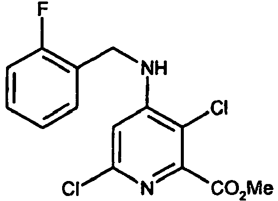 | 7.37-7.24 (m, 2H), 7.17-7.08 (m, 2H), 6.61 (s, 1H), 5.73 (br. s, 1H), 4.51 (d, 2H), 3.95 (s, 3H) |
| 7-2 | 3,6-Dichloro-4-[(3-fluorophenylmeth yl)-amino]-pyridine-2-carboxylic acid methyl ester | 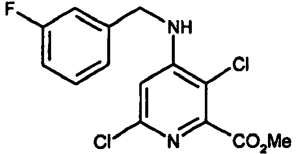 | 7.35 (m, 1H), 7.09 (d, 1H), 7.00 (m, 2H), 6.52 (s, 1H), 5.87 (br. s, 1H), 4.47 (d, 2H), 3.95 (s, 3H) |
| 9-2 | 3,6-Dichloro-4-[(2-methylphenylmet hyl)-amino]-pyridine-2-carboxylic acid methyl ester | 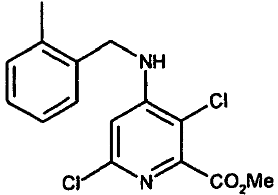 | 7.31-7.21 (m, 4H), 6.63 (s, 1H), 5.36 (m, 1H), 4.38 (d, 2H), 3.97 (s, 3H), 2.37 (s, 3H) |
| 15-2 | 3,6-Dichloro-4-[(2-nitrophenylmethyl )-amino]-pyridine-2-carboxylic acid methyl ester | 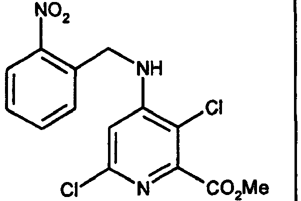 | 6.84 (s, 1H), 6.78 (m, 2H), 6.62 (s, 1H), 5.91 (s, 2H), 4.36 (s, 2H), 3.82 (s, 3H) |
| 19-2 | 3,6-Dichloro-4-[(2,6-difluorophenylmet hyl)-amino]-pyridine-2-carboxylic acid methyl ester | 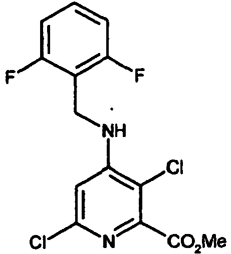 | RT 1.66, MH⁺ 347, 349 |
| 22-2 | 3,6-Dichloro-4-[(2,4-dimethoxyphenyl methyl)-amino]-pyridine-2-carboxylic acid methyl ester | 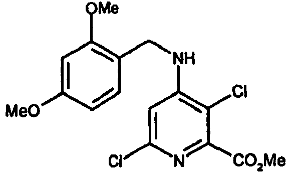 | Used without purification |
| 24-2 | 3,6-Dichloro-4-[(3,4-methylenedioxyp henylmethyl)-amino]-pyridine-2-carboxylic acid methyl ester | 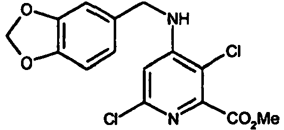 | 6.84 (s, 1H), 6.78 (m, 2H), 6.62 (s, 1H), 5.91 (s, 2H), 4.36 (s, 2H), 3.82 (s, 3H) (NH not observed) |
| 31-2 | 3,6-Dichloro-4-(pyridin-2-ylmethylamino)-pyridine-2-carboxylic acid methyl ester | 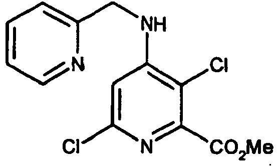 | 8.61 (m, 1H), 7.72 (m, 1H), 7.28 (m, 2H), 6.86 (m, 1H), 6.61 (s, 1H), 4.50 (d, 2H), 3.96 (s, 3H) |
| 66-2 | 3,6-Dichloro-4-[ *N*-(furan-2-ylmethyl)-N-(2-hydroxyethyl)-amino]-pyridine-2-carboxylic acid methyl ester | 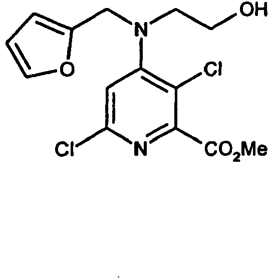 | 7.36 (m, 1H), 7.03 (s, 1H), 6.32 (m, 1H), 6.22 (m, 1H), 4.54 (s, 2H), 3.98 (s, 3H), 3.78 (m, 2H), 3.45 (t, 2H), 1.83 (br. s, 1H) |
| 67-2 | 4-Cyclohexylmethyl amino-3,6-dichloro-pyridine-2-carboxylic acid methyl ester | 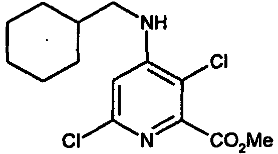 | 6.51 (s, 1H), 5.26 (m, 1H), 3.90 (s, 3H), 3.00 (t, 2H), 1.78-1.55 (m, 6H), 1.28-1.08 (m, 3H), 0.96 (m, 2H) |
| 68-2 (*trans* isomer) | 3,6-Dichloro-pyridine-4-[*trans*-(2-hydroxycyclohexy lmethyl)-amino]-2-carboxylic acid methyl ester | 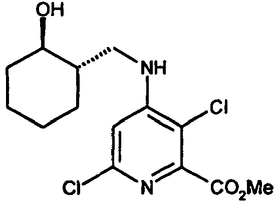 | 6.61 (s, 1H), 6.50 (s, 1H), 3.86 (s, 3H), 3.35 (m, 1H), 3.11 (m, 2H), 1.90 (m, 1H), 1.73-1.52 (m, 4H), 1.30-1.10 (m, 3H), 1.00 (m, 1H) (OH not observed) |
| 69-2 | 4-Cyclooctylmethyl amino-3,6-dichloro-pyridine-2-carboxylic acid methyl ester | 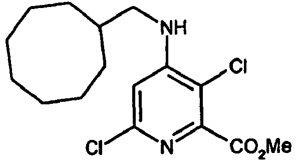 | 6.50 (s, 1H), 5.27 (m, 1H), 3.89 (s, 3H), 2.98 (m, 2H), 2.25 (br. s, 1H), 1.80 (m, 1H), 1.70-1.40 (m, 11H), 1.30 (m, 2H) |
| 70-2 | 3,6-Dichloro-4-(tetra hyd rofu ran-2-ylmethylamino)-pyridine-2-carboxylic acid methyl ester | 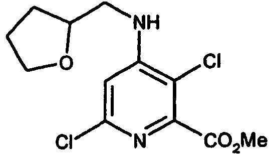 | 6.56 (s, 1H), 5.54 (m, 1H), 4.07 (m, 1H), 3.88 (s, 3H), 3.84 (m, 1H), 3.74 (m, 1H), 3.30 (m, 1H), 3.11 (m, 1H), 2.02 (m, 1H), 1.89 (m, 2H), 1.57 (m, 1H) ppm |
| 71-2 | 3,6-Dichloro-4-[(*N*-ethyl-pyrrolidin-2-ylmethyl)-amino]-pyridine-2-carboxylic acid methyl ester | 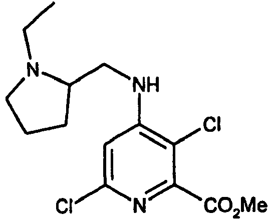 | 6.51 (s, 1H), 6.16 (m, 1H), 3.92 (s, 3H), 3.27-3.07 (m, 3H), 2.73 (m, 2H), 2.23 (m, 2H), 1.92 (m, 1H), 1.73 (m, 2H), 1.60 (m, 1H), 1.07 (t, 3H) |
| 72-2 | 4-[(2-Bromophenylmet hyl)-amino]-3,6-dichloro-pyridine-2-carboxylic acid methyl ester | 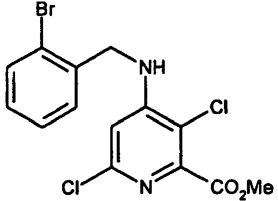 | 7.61 (dd, 1H), 7.34-7.19 (m, 3H), 6.53 (s, 1H), 5.78 (m, 1H), 4.51 (d, 2H), 3.95 (s, 3H) |
| 73-2 | 3,6-Dichloro-4-[(2-methoxyphenylm ethyl)-amino]-pyridine-2-carboxylic acid methyl ester | 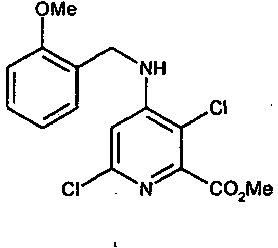 | 7.31 (m, 1H), 7.19 (dd, 1H), 6.94 (m, 2H), 6.65 (s, 1H), 5.78 (m, 1H), 4.42 (d, 2H), 3.94 (s, 3H), 3.88 (s, 3H) |
| 74-2 | 3,6-Dichloro-4-[(2-trifluoromethylphe nylmethyl)-amino]-pyridine-2-carboxylic acid methyl ester | 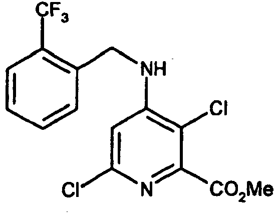 | 7.62 (d, 1H), 7.45 (t, 1H), 7.32 (m, 2H), 6.33 (s, 1H), 6.29 (m, 1H), 4.58 (d, 2H), 3.85 (s, 3H) |
| 75-2 | 3,6-Dichloro-4-(thiophen-2-ylmethylamino)-pyridine-2-carboxylic acid methyl ester | 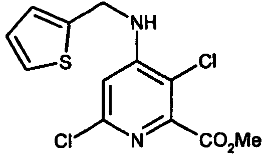 | 7.29 (dd, 1H), 7.04 (m, 1H), 7.01 (m, 1H), 6.67 (s, 1H), 5.68 (m, 1H), 4.62 (d, 2H), 3.96 (s, 3H) |
| 76-2 | 4-[(2-Bromothiophen-3-ylmethyl)-amino]-3,6-dichloro-pyridine-2-carboxylic acid methyl ester | 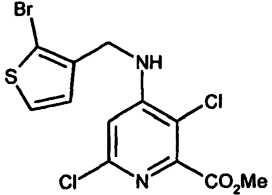 | 7.32 (d, 1H), 6.99 (d, 1H), 6.65 (s, 1H), 5.52 (m, 1H), 4.38 (d, 2H), 3.97 (s, 3H) |
| 77-2 | 3,6-Dichloro-4-[(1,3-dimethylpyrazol-5-ylmethyl)-amino]-pyridine-2-carboxylic acid methyl ester | 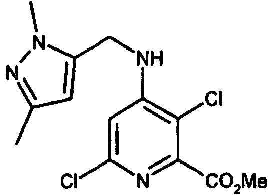 | 6.64 (s, 1H), 6.00 (s, 1H), 5.39 (m, 1H), 4.37 (d, 2H), 3.94 (s, 3H), 3.79 (s, 3H), 2.23 (s, 3H) |
| 78-2 | 3,6-Dichloro-4-(pyridin-3-ylmethylamino)-pyridine-2-carboxylic acid methyl ester | 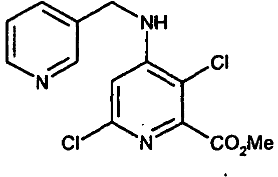 | 8.54 (m, 2H), 7.60 (m, 1H), 7.28 (m, 1H), 6.51 (s, 1H), 5.88 (m, 1H), 4.46 (d, 2H), 3.90 (s, 3H) |
| 79-2 | 3,6-Dichloro-4-[(6-trifluoromethylpyri din-3-ylmethyl)-amino]-pyridine-2-carboxylic acid methyl ester | 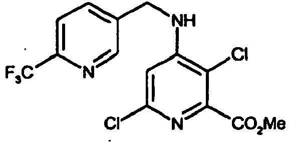 | 8.74 (s, 1H), 7.83 (d, 1H), 7.74 (d, 1H), 6.54 (s, 1H), 5.70 (m, 1H), 4.61 (d, 2H), 3.98 (s, 3H) |
| 80-2 | 3,6-Dichloro-4-(pyridin-4-ylmethylamino)-pyridine-2-carboxylic acid methyl ester | 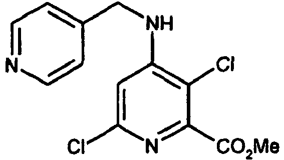 | 8.56 (d, 2H), 7.18 (d, 2H), 6.40 (s, 1H), 6.02 (m, 1H), 4.49 (d, 2H), 3.92 (s, 3H) |
| 81-2 | 3,6-Dichloro-4-[(5-methylpyrazin-2-ylmethyl)-amino]-pyridine-2-carboxylic acid methyl ester | 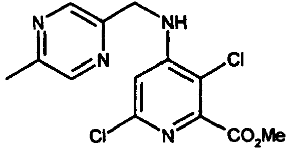 | 8.51 (s, 1H), 8.47 (s, 1H), 6.66 (s, 1H), 6.48 (m, 1H), 4.53 (d, 2H), 3.96 (s, 3H), 2.60 (s, 3H) |

### EXAMPLE 4

### Synthesis of 3-bromo-6-chloro-4-cyclopropylmethylamino-pyridine-2-carboxylic acid methyl ester (Compound 1-17).

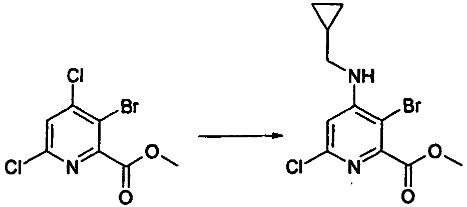

A solution of 3-bromo-4,6-dichloro-pyridine-2-carboxylic acid methyl ester (prepared as described in Example 2) (0.545 g, 1.91 mmol) in N-methylpyrrolidone (2 mL) was added to a mixture of cyclopropylmethylamine (136 mg, 1.91 mmol) and diisopropyethylamine (0.66 ml, 3.82 mmol) and the resulting mixture heated at 80 °C for 20 h. The reaction mixture was cooled to room temperature, water (2 ml) and dichloromethane (2 ml) added and the mixture stirred for 5 min. The phases were separated and the organic phase concentrated *in vacuo.* The residue was purified by reverse phase preparative HPLC, using FractionLynx (X Bridge column, ammonium acetate buffer) to give 3-bromo-6-chloro-4-cyclopropylamino-pyridine-2-carboxylic acid methyl ester. Characterising data for the compound is:
¹H NMR (400 MHz, CDCl₃) δ 6.50 (s, 1H), 5.40 (m, 1H), 4.00 (s, 3H), 3.10 (m, 2H), 1.10 (m, 1H), 0.70 (m, 2H), 0.30 (m, 2H) ppm.

Other compounds made using this general method are listed in Table 5 below. Characteristic data provided is either ¹H-nmr data (400 MHz, CDCl₃) δ_{H} ppm or LCMS [retention time (RT, recorded in minutes) and the molecular ion, typically the cation M+H⁺]. Compounds characterised by HPLC-MS used a Waters 2777 injector with a 1525 micro pump HPLC equipped with a Waters Atlantis dC18 IS column (column length 20 mm, internal diameter of column 3 mm, particle size 3 micron), Waters 2996 photodiode array, Waters 2420 ELSD and Micromass ZQ2000. The analysis was conducted using a three minute run time, according to the following gradient table:

| Time (minutes) | Solvent A (%) | Solvent B (%) | Flow (ml / minute) |
|---|---|---|---|
| 0.00 | 95 | 5 | 1.3 |
| 2.50 | 0 | 100 | 1.3 |
| 2.80 | 0 | 100 | 1.3 |
| 2.90 | 95 | 5 | 1.3 |

| | | | |
|---|---|---|---|
| Solvent A: H₂O with 0.05% TFA Solvent B: CH₃CN with 0.05% TFA | | | |

**TABLE 5 Compounds made according to the method described in Example 4 above.**

| **Compound Number** | **Name** | **Structure** | **Characteristic data** |
|---|---|---|---|
| 2-17 | 3-Bromo-6-chloro-4-phenylmethylami no-pyridine-2-carboxylic acid methyl ester | 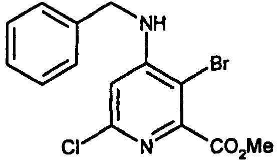 | RT 1.64, MH⁺ 355, 357, 359 |
| 3-17 | 3-Bromo-6-chloro4-[(2-chlorophenylmeth yl)-amino]-pyridine-2-carboxylic acid methyl ester | 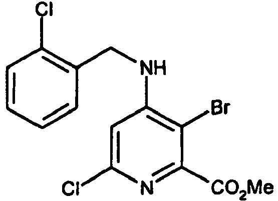 | RT 1.76, MH⁺ 389,391,393 |
| 6-17 | 3-Bromo-6-chloro-4-[(2-fluorophenylmeth yl)-amino]-pyridine-2-carboxylic acid methyl ester | 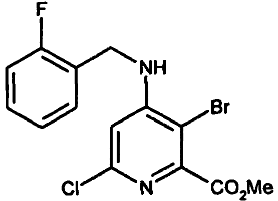 | RT 1.66, MH⁺ 373, 375, 377 |
| 9-17 | 3-Bromo-6-chloro-4-[(2-methylphenylmet hyl)-amino]-pyridine-2-carboxylic acid methyl ester | 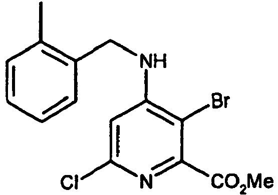 | Used without purification |
| 14-17 | 3-Bromo-6-chloro-4-[(4-dimethylaminoph enylmethyl)-amino]-pyridine-2-carboxylic acid methyl ester | 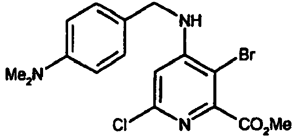 | RT 1.06, MH⁺ 398, 400, 402 |
| 15-17 | 3-Bromo-6-chloro-4-[(2-nitrophenylmethyl )-amino]-pyridine-2-carboxylic acid methyl ester | 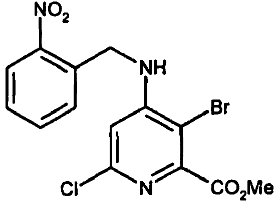 | 8.21 (m, 1H), 7.68 (m, 1H), 7.55 (t, 1H), 7.46 (d, 1H), 6.46 (s, 1H), 5.96 (m, 1H), 4.91 (d, 2H), 3.98 (s, 3H) |
| 21-17 | 3-Bromo-6-chloro-4-[(2,6-dichlorophenylme thyl)-amino]-pyridine-2-carboxylic acid methyl ester | 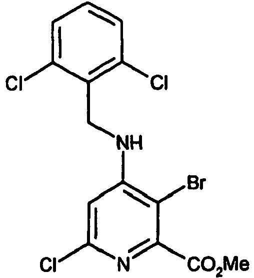 | RT 1.91, MH⁺ 423, 425, 427, 429 |
| 26-17 | 3-Bromo-6-chloro-4-[(furan-2-ylmethyl)-amino]-pyridine-2-carboxylic acid methyl ester | 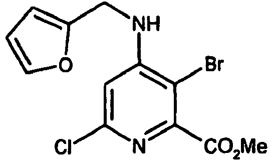 | 7.43-7.42 (m, 1H), 6.67 (s, 1H), 6.39-6.37 (m, 1H), 6.33-6.31 (m, 1H), 5.66-5.60 (m, 1H), 4.44 (d, 2H), 3.97 (s, 3H) |
| 32-17 | 3-Bromo-6-chloro-4-[(3-chloro-pyridin-2-ylmethyl)-amino]-pyridine-2-carboxylic acid methyl ester | 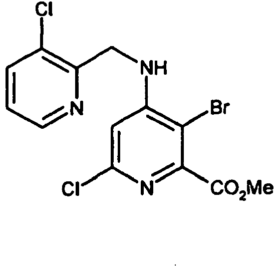 | 8.50 (s, 1H), 7.80 (m, 1H), 7.40-7.20 (m, 2H), 6.70 (s, 1H), 4.50 (m, 2H), 4.00 (s, 3H) |
| 33-17 | 3-Bromo-6-chloro-4-[(3-methyl-pyridin-2-ylmethyl)-amino]-pyridine-2-carboxylic acid methyl ester | 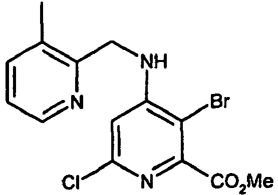 | RT 0.97, MH⁺ 370, 372, 374 |
| 67-17 | 3-Bromo-6-chloro-4-cyclohexylmethyl amino-pyridine-2-carboxylic acid methyl ester | 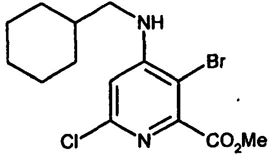 | RT 1.94, MH⁺ 361,363,365 |
| 73-17 | 3-Bromo-6-chloro-4-[(2-methoxyphenylm ethyl)-amino]-pyridine-2-carboxylic acid methyl ester | 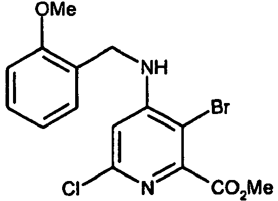 | RT 1.72, MH⁺ 385, 387, 389 |
| 74-17 | 3-Bromo-6-chloro-4-[(2-trifluoromethylphe nylmethyl)-amino]-pyridine-2-carboxylic acid methyl ester | 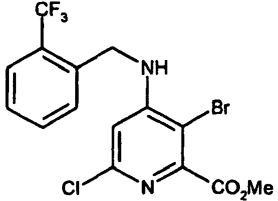 | RT 1.82, MH⁺ 423, 425, 427 |
| 75-17 | 3-Bromo-6-chloro-4-(thiophen-2-ylmethylamino)-pyridine-2-carboxylic acid methyl ester | 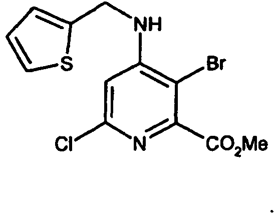 | RT 1.59, MH⁺ 361,363,365 |
| 80-17 | 3-Bromo-6-chloro-4-[(pyridin-4-ylmethyl)-amino]-pyridine-2-carboxylic acid methyl ester | 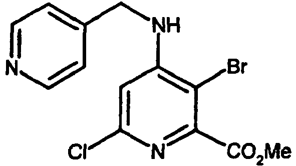 | RT 0.84, MH⁺ 356, 358, 360 |

### EXAMPLE 5

### Synthesis of 3-chloro-6-(4-chloro-2-fluoro-3-methoxy-phenyl)-4-[(furan-2-ylmethyl)-amino]-pyridine-2-carboxylic acid methyl ester (Compound 26-164).

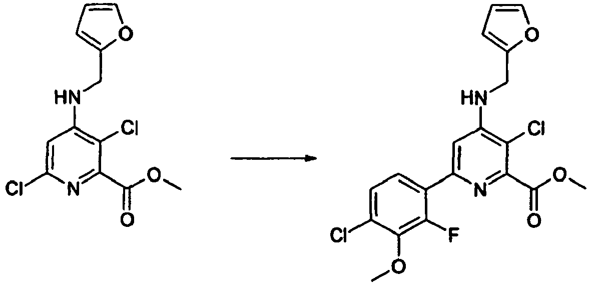

3,6-Dichloro-4-[(furan-2-ylmethyl)-amino]-pyridine-2-carboxylic acid methyl ester (prepared as described in Example 3) (0.500 g, 1.66 mmol), 2-(4-chloro-2-fluoro-3-methoxy-phenyl)-[1,3,2]dioxaborinane (0.446 g, 1.83 mmol), caesium fluoride (0.505 g, 3.32 mmol) and [1,1-bis(diphenylphosphino)-ferrocene]dichloropalladium(II) dichloromethane adduct (0.068 g, 0.08 mmol) were suspended in a degassed mixture of dimethoxyethane (2.5 mL) and water (2.5 mL). After stirring the suspension under an atmosphere of nitrogen for 5 min, the reaction mixture was heated under microwave irradiation to 140 °C for 15 min. After cooling to room temperature, the reaction was poured into water and the aqueous layer was extracted with dichloromethane using a hydrophobic frit to collect the organic layer. The organic layer was evaporated under reduced pressure, and the resulting residue was purified by silica gel chromatography (gradient elution: 0 - 40% ethyl acetate in *iso*-hexane). The resulting material was further purified by reverse phase preparative HPLC, using FractionLynx (XBridge column, 30 x 100 mm, 5 micron particles, 20 mM ammonium acetate buffer) to give 3-chloro-6-(4-chloro-2-fluoro-3-methoxy-phenyl)-4-[(furan-2-ylmethyl)-amino]-pyridine-2-carboxylic acid methyl ester (355 mg, 50%) as a solid.
Characterising data for the compound are as follows:
¹H NMR (400 MHz, CDCl₃) δ 7.63 (t, 1H), 7.40 (m, 1H), 7.24 (m, 1H), 7.16 (d, 1H), 6.35 (m, 1H), 6.31 (m, 1H), 5.50 (m, 1H), 4.50 (d, 2H), 3.98 (s, 6H) ppm.

Further examples of compounds that were prepared using this method are listed below in Table 6.

**TABLE 6 Compounds made according to the method described in Example 5 above.**

| **Compound Number** | **Name** | **Structure** | **¹H NMR (400 MHz, CDCl₃) δ ppm** |
|---|---|---|---|
| 26-245 | 3-Chloro-6-(4-chloro-3-dimethylamino -2-fluoro-phenyl)-4-[(furan-2-ylmethyl)-amino]-pyridine-2-carboxylic acid methyl ester | 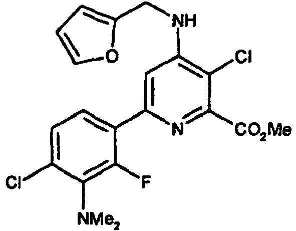 | 7.56 (t, 1H), 7.40 (m, 1H), 7.22 (dd, 1H), 7.12 (d, 1H), 6.36 (m, 1H), 6.32 (m, 1H), 5.48 (m, 1H), 4.50 (d, 2H), 3.98 (s, 3H), 2.90 (s, 6H) |
| 22-164 | 3-Chloro-6-(4-chloro-2-fluoro-3-methoxy-phenyl)-4-[(2,4-dimethoxyphenyl-methyl)-amino]-pyridine-2-carboxylic acid methyl ester | 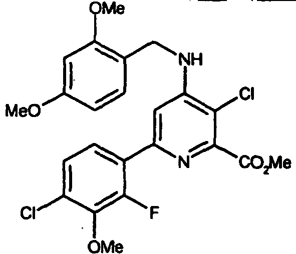 | 7.61 (t, 1H), 7.22 (dd,1H), 7.17 (d, 1H), 7.12 (m, 1H), 6.50 (d, 1H), 6.47 (dd, 1H), 5.55 (m, 1H), 4.41 (d, 2H), 3.98 (s, 6H), 3.86 (s, 3H), 3.81 (s, 3H) |

### EXAMPLES 6 AND 7

### Synthesis of 3-chloro-6-cyclopropyl-4-[(furan-2-ylmethyl)-amino]-pyridine-2-carboxylic acid methyl ester (Compound 26-83) and 6-chloro-3-cyclopropyl-4-[(furan-2-ylmethyl)-amino]-pyridine-2-carboxylic acid methyl ester (Compound 26-7).

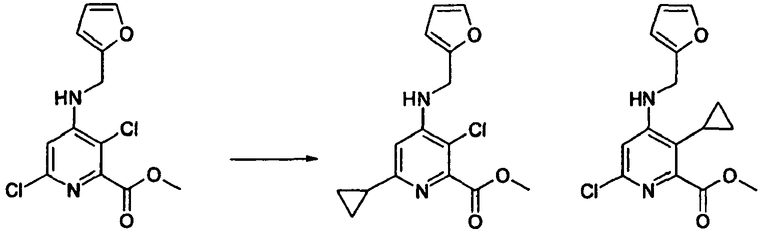

3,6-Dichloro-4-[(furan-2-ylmethyl)-amino]-pyridine-2-carboxylic acid methyl ester (prepared as described in Example 3) (0.301 g, 1.00 mmol), cyclopropyl boronic acid (0.086 g, 1.00 mmol), potassium phosphate (0.637 g, 3.00 mmol), palladium acetate (0.011 g, 0.05 mmol) and tricyclohexylphosphine tetrafluoroborate (0.037 g, 0.10 mmol) were suspended in a degassed mixture of toluene (3.6 mL) and water (0.4 mL). After stirring the suspension under an atmosphere of nitrogen for 5 min, the reaction mixture was heated under microwave irradiation to 140 °C for 45 min. After cooling to room temperature, the reaction mixture was poured into water and the aqueous layer was extracted with dichloromethane using a hydrophobic frit to collect the organic layer. The organic layer was evaporated under reduced pressure, and the resulting material was purified by reverse phase preparative HPLC, using FractionLynx (XBridge column, 30 x 100 mm, 5 micron particles, 20 mM ammonium acetate buffer) to give two regioisomeric products. The two products were separately further purified by silica gel chromatography (gradient elution: 0 - 40% ethyl acetate in iso-hexane) to give 3-chloro-6-cyclopropyl-4-[(furan-2-ylmethyl)-amino]-pyridine-2-carboxylic acid methyl ester (72 mg, 23%) and 6-chloro-3-cyclopropyl-4-[(furan-2-ylmethyl)-amino]-pyridine-2-carboxylic acid methyl ester (30 mg, 10%) as gums.
Characterising data for 3-chloro-6-cyclopropyl-4-[(furan-2-ylmethyl)-amino]-pyridine-2-carboxylic acid methyl ester are as follows:
¹H NMR (400 MHz, CDCl₃) δ 7.40 (m, 1H), 6.43 (s, 1H), 6.35 (m, 1H), 6.27 (m, 1H), 5.27 (m, 1H), 4.41 (d, 2H), 3.95 (s, 3H), 1.95 (m, 1H), 0.95 (m, 4H) ppm.
Characterising data for 6-chloro-3-cyclopropyl-4-[(furan-2-ylmethyl)-amino]-pyridine-2-carboxylic acid methyl ester are as follows:
¹H NMR (400 MHz, CDCl₃) δ 7.40 (m, 1H), 6.60 (s, 1H), 6.36 (m, 1H), 6.28 (m, 1H), 5.52 (m, 1H), 4.41 (d, 2H), 3.94 (s, 3H), 1.60 (m, 1H), 1.02 (m, 2H), 0.42 (m, 2H) ppm.

### EXAMPLE 8

### Synthesis of 6-(4-chloro-2-fluoro-3-methoxy-phenyl)-3-ethenyl-4-[(furan-2-ylmethyl)-amino]-pyridine-2-carboxylic acid methyl ester (Compound 26-174).

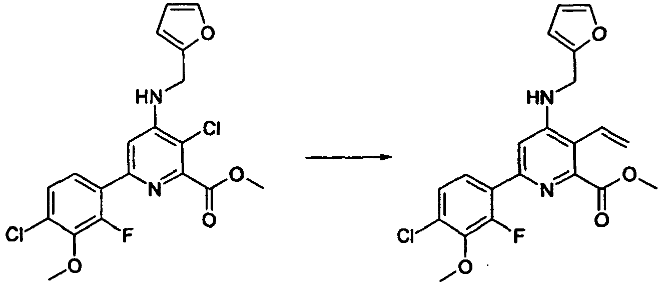

3-Chloro-6-(4-chloro-2-fluoro-3-methoxy-phenyl)-4-[(furan-2-ylmethyl)-amino]-pyridine-2-carboxylic acid methyl ester (prepared as described in Example 5) (0.150 g, 0.35 mmol), 4,4,5,5-tetramethyl-2-vinyl-[1,3,2]dioxaborolane (0.082 g, 0.53 mmol), caesium fluoride (0.107 g, 0.71 mmol), and [1,1-bis(diphenylphosphino)-ferrocene]dichloropalladium(II) dichloromethane adduct (0.029 g, 0.035 mmol) were suspended in a degassed mixture of dimethoxyethane (1.8 mL) and water (1.8 mL). After stirring the suspension under an atmosphere of nitrogen for 5 min, the reaction mixture was heated under microwave irradiation to 160 °C for 30 min. After cooling to room temperature, the reaction mixture was poured into water and the aqueous layer was extracted with dichloromethane using a hydrophobic frit to collect the organic layer. The organic layer was evaporated under reduced pressure, and the resulting material was purified by reverse phase preparative HPLC, using FractionLynx (XBridge column, 30 x 100 mm, 5 micron particles, 20 mM ammonium acetate buffer) to give the desired product. The product was further purified by silica gel chromatography (gradient elution: 0 - 30% ethyl acetate in iso-hexane) to give 6-(4-chloro-2-fluoro-3-methoxy-phenyl)-3-ethenyl-4-[(furan-2-ylmethyl)-amino]-pyridine-2-carboxylic acid methyl ester (13 mg, 9%) as a yellow gum.
Characterising data for the compound are as follows:
¹H NMR (400 MHz, CDCl₃) δ 7.65 (t, 1H), 7.39 (m, 1H), 7.22 (dd, 1H), 7.11 (d, 1H), 6.80 (m, 1H), 6.35 (m, 1H), 6.29 (m, 1H), 5.65 (dd, 1H), 5.50 (dd, 1H), 5.28 (m, 1H) 4.41 (d, 2H), 3.98 (s, 3H), 3.91 (s, 3H) ppm.

Further examples of compounds that were prepared using this method are listed below in Table 7.

**TABLE 7 Compounds made according to the method described in Example 8 above.**

| **Compound Number** | **Name** | **Structure** | **¹H NMR (400 MHz, CDCl₃) δ ppm** |
|---|---|---|---|
| 26-255 | 6-(4-Chloro-3-dimethylamino-2-fluoro-phenyl)-3-ethenyl-4-[(furan-2-ylmethyl)-amino]-pyridine-2-carboxylic acid methyl ester | 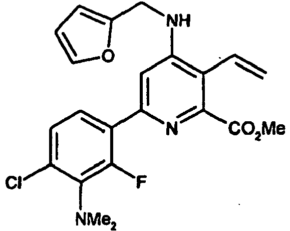 | 7.57 (t, 1H), 7.39 (m, 1H), 7.22 (dd, 1H), 7.06 (d, 1H), 6.80 (m, 1H), 6.35 (m, 1H), 6.28 (m, 1H), 5.65 (dd, 1H), 5.50 (dd, 1H), 5.25 (m, 1H) 4.42 (d, 2H), 3.91 (s, 3H), 2.90 (d, 6H) |

### EXAMPLE 9

### Synthesis of (Z)-6-(4-chloro-2-fluoro-3-methoxy-phonyl)-3-(2-ethoxyethenyl)-4-[(furan-2-ylmethyl)-amino]-pyridine-2-carboxylic acid methyl ester (Compound 26-463).

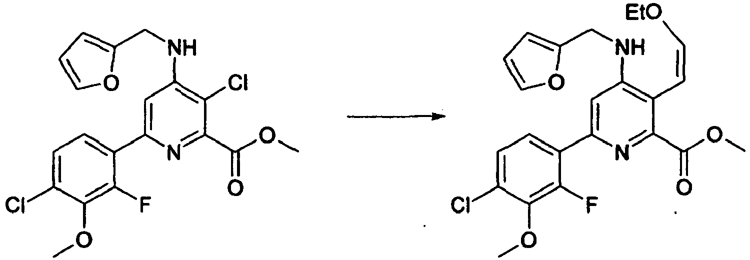

3-Chloro-6-(4-chloro-2-fluoro-3-methoxy-phenyl)-4-[(furan-2-ylmethyl)-amino]-pyridine-2-carboxylic acid methyl ester (prepared as described in Example 5) (0.20 g, 0.47 mmol), (Z)-1-ethoxy-2-(tributylstannyl)-ethene (0.21 g, 0.59 mmol), and bis(tri-t-butylphosphine)palladium (12 mg, 0.024 mmol) were suspended in degassed dimethylformamide (5 ml) and the resulting mixture heated under microwave irradiation to 160 °C for 15 min. After cooling to room temperature, the reaction mixture was poured into water and the aqueous layer was extracted with dichloromethane. The organic extracts were evaporated under reduced pressure, and the residue purified by reverse phase preparative HPLC, using FractionLynx (XBridge column, 30 x 100 mm, 5 micron particles, 20 mM ammonium acetate buffer) to give (Z)-6-(4-chloro-2-fluoro-3-methoxy-phenyl)-3-(2-ethoxyethenyl)-4-[(furan-2-ylmethyl)-amino]-pyridine-2-carboxylic acid methyl ester (23 mg, 11 %) as a solid.
Characterising data for the compound are as follows:
¹H NMR (400 MHz, CD₃OD) δ 7.45-7.40 (m, 2H), 7.28 (dd, 1H), 7.01 (d, 1H), 6.44 (d, 1H), 6.35 (m, 1H), 6.29 (m, 1H), 5.25 (d, 1H), 4.48 (s, 2H), 3.96 (s, 3H), 3.91 (q, 2H), 3.87 (s, 3H), 1.21 (t, 3H) ppm (NH not observed).

### EXAMPLE 10

### Synthesis of 3-bromo-5,6-dichloro-4-[(2-nitrophenyl-methyl)-amino]-pyridine-2-carboxylic acid methyl ester (Compound 15-41)

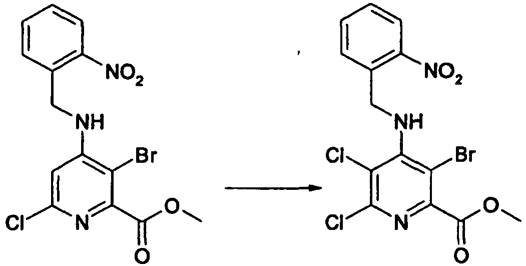

N-Chlorosuccinimide (185 mg, 1.4 mmol) was added to a stirred solution of 3-bromo-6-chloro-4-[(2-nitrophenylmethyl)-amino]-pyridine-2-carboxylic acid methyl ester (prepared as described in Example 4) (500 mg, 1.25 mmol) in acetonitrile (30 ml) and stirring continued at ambient temperature for 3 hours. The reaction mixture was then heated at reflux for 20 hours, with additional N-chlorosuccinimide (185 mg, 1.4 mmol) being added after 6 hours. The reaction mixture was allowed to cool and evaporated under reduced pressure. Water (40 ml) was added to the residue and the resulting mixture extracted with ethyl acetate (3 x 40 ml). The combined organic extracts were dried over magnesium sulphate, filtered and evaporated under reduced pressure to provide a yellow oil that was purified by silica gel chromatography (gradient elution: 0 - 50% ethyl acetate in iso-hexane) to provide 3-bromo-5,6-dichloro-4-[(2-nitrophenyl-methyl)-amino]-pyridine-2-carboxylic acid methyl ester (500 mg, 92%). Characterising data for the compound are as follows:
¹H NMR (400 MHz, CDCl₃) δ 8.10 (m, 1H), 7.60 (m, 1H), 7.50 (m, 2H), 5.90 (m, 1H), 5.20 (m, 2H), 4.00 (s, 3H) ppm.

Further examples of compounds that were prepared using this method are listed below in Table 8.

**TABLE 8 Compounds made according to the method described in Example 10 above.**

| **Compound Number** | **Name** | **Structure** | **¹H NMR (400 MHz, CDCl₃) δ ppm** |
|---|---|---|---|
| 1-41 | 3-Bromo-4-cyclopropylmethyl amino-5,6-dichloro-pyridine-2-carboxylic acid methyl ester | 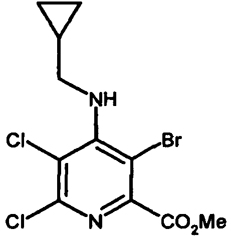 | 5.30 (m, 1H), 3.90 (s, 3H), 3.50 (m, 2H), 1.10 (m, 1H), 0.60 (m, 2H), 0.30 (m, 2H) |
| 32-41 | 3-Bromo-4-[(3-chloro-pyridin-2-ylmethyl)-amino]-5,6-dichloro-pyridine-2-carboxylic acid methyl ester | 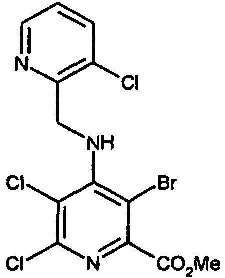 | 8.60 (m, 1H), 7.60 (m, 2H), 5.10 (m, 2H), 4.00 (s, 3H) (NH not observed) |

### EXAMPLE 11

### Synthesis of 3-bromo-6-chloro-5-fluoro-4-[(2-nitrophenyl-methyl)-amino]-pyridine-2-carboxylic acid methyl ester (Compound 15-29)

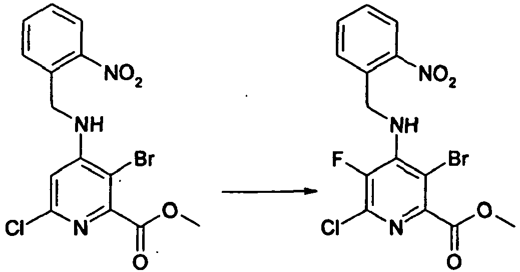

Selectfluor® (660 mg, 1.9 mmol) was added to a stirred solution of 3-bromo-6-chloro-4-[(2-nitrophenylmethyl)-amino]-pyridine-2-carboxylic acid methyl ester (prepared as described in Example 4) (500 mg, 1.25 mmol) in acetonitrile (30 ml) and the mixture heated at reflux for 16 hours. The reaction mixture was allowed to cool and evaporated under reduced pressure. Water (40 ml) was added to the residue and the resulting mixture extracted with ethyl acetate (3 x 40 ml). The combined organic extracts were dried over magnesium sulphate, filtered and evaporated under reduced pressure to provide a yellow oil that was purified by silica gel chromatography (gradient elution: 0 - 50% ethyl acetate in *iso*-hexane) to provide 3-bromo-6-chloro-5-fluoro-4-[(2-nitrophenyl-methyl)-amino]-pyridine-2-carboxylic acid methyl ester (440 mg, 84%). Characterising data for the compound are as follows:
¹H NMR (400 MHz, CDCl₃) δ 8.20 (m, 1H), 7.70 (m, 1H), 7.50 (m, 2H), 6.00 (m, 1H), 5.10 (m, 2H), 4.00 (s, 3H) ppm.

Further examples of compounds that were prepared using this method are listed below in Table 9.

**TABLE 9 Compounds made according to the method described in Example 11 above.**

| **Compound Number** | **Name** | **Structure** | **¹H NMR (400 MHz, CDCl₃) δ ppm** |
|---|---|---|---|
| 1-29 | 3-Bromo-6-chloro-4-cyclopropylmethyl amino-5-fluoro-pyridine-2-carboxylic acid methyl ester | 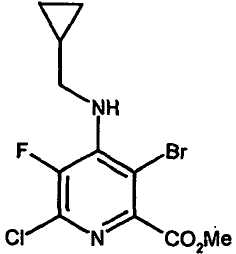 | 5.15 (br s, 1H), 3.96 (s, 3H), 3.43 (m, 2H), 1.13 (m, 1H), 0.62 (m, 2H), 0.30 (m, 2H) |
| 32-29 | 3-Bromo-6-chloro-4-[(3-chloro-pyridin-2-ylmethyl)-amino]-5-fluoro-pyridine-2-carboxylic acid methyl ester | 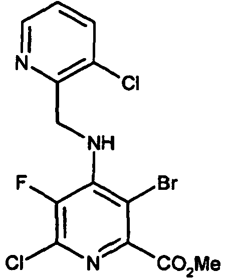 | 8.60 (m, 1H), 7.70 (m, 1H), 7.40 (m, 1H), 7.30 (m, 1H), 5.00 (m, 2H), 4.00 (s, 3H) |

### EXAMPLE 12

### Synthesis of 6-chloro-4-[(furan-2-ylmethyl)-amino]-3-methyl-pyridine-2-carboxylic acid methyl ester (Compound 26-19).

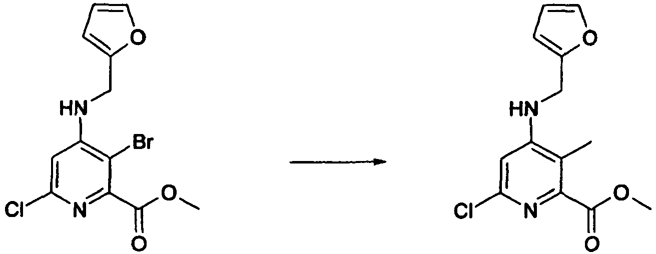

A mixture of 3-bromo-6-chloro-4-[(furan-2-ylmethyl)-amino]-pyridine-2-carboxylic acid methyl ester (prepared as described in Example 4) (187 mg, 0.54 mmol), tetramethyltin (107 mg, 0.59 mmol) and bis(triphenylphosphine)palladium(II) dichloride (19 mg, 0.03 mmol) in dimethylformamide (2 mL) was heated to 160 °C under microwave irradiation for 15 min, then allowed to cool and poured into water. The mixture was extracted with dichloromethane, and the organic extracts concentrated under reduced pressure. The residue was purified by reverse phase preparative HPLC, using FractionLynx (X Bridge column, ammonium acetate buffer) followed by silica gel chromatography (gradient elution: 0 - 60% ethyl acetate in iso-hexane) to give 6-chloro-4-[(furan-2-ylmethyl)-amino]-3-methyl-pyridine-2-carboxylic acid methyl ester (68 mg, 45%) as a white solid.
Characterising data for the compound are as follows:
¹H NMR (400 MHz, CDCl₃) δ 7.41 (d, 1H), 6.65 (s, 1H), 6.37 (m, 1H), 6.30 (d, 1H), 4.73 (m, 1H), 4.39 (d, 2H), 3.93 (s, 3H), 2.24 (s, 3H) ppm.

Further examples of compounds that were prepared using this method are listed below in Table 10.

**TABLE 10 Compounds made according to the method described in Example 12 above.**

| **Compound Number** | **Name** | **Structure** | **¹H NMR (400 MHz, CDCl₃) δ ppm** |
|---|---|---|---|
| 1-31 | 6-Chloro-4-cyclopropylmethyl amino-5-fluoro-3-methyl-pyridine-2-carboxylic acid methyl ester | 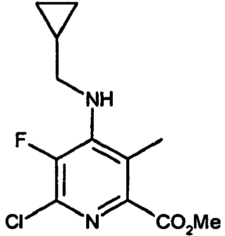 | 3.90 (s, 3H), 3.40 (m, 2H), 2.30 (s, 3H), 1.10 (m, 1H), 0.60 (m, 2H), 0.30 (m, 2H) (NH not observed) |
| 1-43 | 4-Cyclopropylmethy lamino-5,6-dichloro-3-methyl-pyridine-2-carboxylic acid methyl ester | 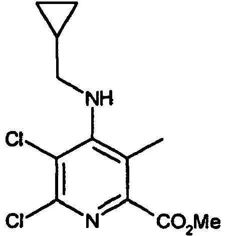 | 4.80 (m, 1H), 3.90 (s, 3H), 3.30 (m, 2H), 2.50 (s, 3H), 1.10 (m, 1H), 0.60 (m, 2H), 0.30 (m, 2H) |
| 15-31 | 6-Chloro-5-fluoro-3-methyl-4-[(2-nitrophenyl-methyl)-amino]-pyridine-2-carboxylic acid methyl ester | 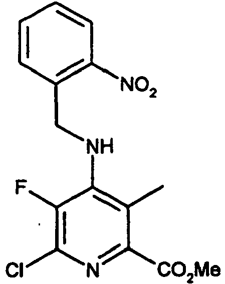 | 8.10 (m, 1H), 7.60 (m, 1H), 7.50 (m, 2H), 5.10 (m 1H), 5.00 (m, 2H), 4.00 (s, 3H), 2.40 (s, 3H) |
| 15-43 | 5,6-Dichloro-3-methyl-4-[(2-nitrophenyl-methyl)-amino]-pyridine-2-carboxylic acid methyl ester | 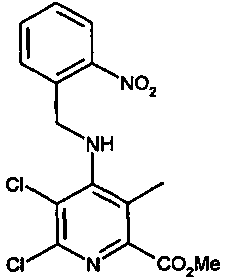 | 8.10 (m, 1H), 7.60 (m, 1H), 7.50 (m, 2H), 5.20 (m, 1H), 4.80 (m, 2H), 3.90 (s, 3H), 2.40 (s, 3H) |
| 15-456 | 5,6-Dichloro-4-[(2-nitrophenyl-methyl)-amino]-3-propyn-1-yl-pyridine-2-carboxylic acid methyl ester | 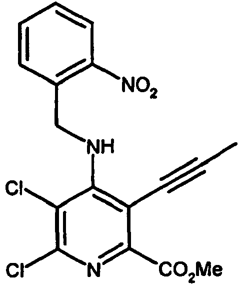 | 8.10 (m, 1H), 7.65 (m, 1H), 7.60 (m, 1H), 7.50 (m, 1H), 6.10 (m, 1H), 5.40 (m, 2H), 3.90 (s, 3H), 1.95 (s, 3H) |
| 26-458 | 6-Chloro-4-[(furan-2-yl-methyl)-amino]-3-phenyl-pyridine-2-carboxylic acid methyl ester | 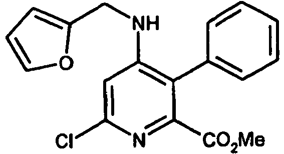 | 7.51-7.41 (m, 3H), 7.34 (m, 1H), 7.25 (m, 2H), 6.72 (s, 1H), 6.31 (m, 1H), 6.17 (m, 1H), 4.68 (m, 1H), 4.30 (d, 2H), 3.63 (s, 3H) |
| 32-43 | 4-[(3-Chloro-pyridin-2-ylmethyl)-amino]-5,6-dichloro-3-methyl-pyridine-2-carboxylic acid methyl ester | 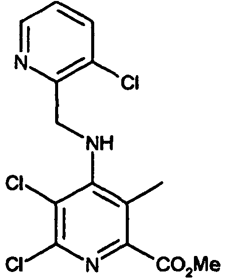 | 8.60 (m, 1H), 7.70 (m, 1H), 7.30 (m, 1H), 7.00 (m, 1H), 4.90 (m, 2H), 3.90 (s, 3H), 2.50 (s, 3H) |
| 32-465 | 5-Chloro-4-[(3-chloro-pyridin-2-ylmethyl)-amino]-3,6-dimethyl-pyridine-2-carboxylic acid methyl ester | 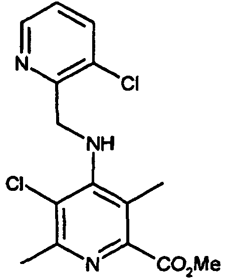 | 8.50 (m, 1H), 7.70 (m, 1H), 7.20 (m, 1H), 6.70 (m, 1H), 4.80 (m, 2H), 4.00 (s, 3H), 2.60 (s, 3H), 2.50 (s, 3H) |

### EXAMPLE 13

### Synthesis of 5,6-dichloro-4-[(2-nitrophenyl-methyl)-amino]-3-trifluoromethyl-pyridine-2-carboxylic acid methyl ester (Compound 15-444)

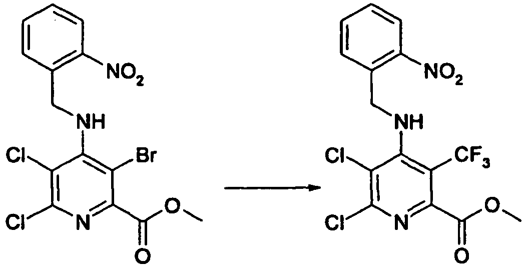

A mixture of of 3-bromo-5,6-dichloro-4-[(2-nitrophenylmethyl)-amino]-pyridine-2-carboxylic acid methyl ester (prepared as described in Example 10) (220 mg, 0.51 mmol), methyl 2,2-difluoro-2-(fluorosulphonyl)-acetate (71 µl, 0.56 mmol)), copper (I) iodide (29 mg, 0.15 mmol) and dimethylformamide (1.5 ml) was heated under microwave irradiation at 150 °C for 1 hour, then cooled and filtered through Celite®. The filtrate was purified by reverse phase preparative HPLC, using FractionLynx (X Bridge column, ammonium acetate buffer) to provide 5,6-dichloro-4-[(2-nitrophenyl-methyl)-amino]-3-trifluoromethyl-pyridine-2-carboxylic acid methyl ester (52 mg, 24%).

The compound was characterised by HPLC-MS using a Waters Acquity HPLC equipped with a Waters Atlantis dC18 column (column length 20 mm, internal diameter of column 3 mm, particle size 3 micron, temperature 40 °C), Waters TW detector, Waters 2777 Sample manager, ESI Corona CAD detector and Micromass ZQ2000 MS. Standard MS conditions are ES+/- switching over mass range 130-700. The analysis is conducted using a two minute run time, according to the following gradient table:

| Time (mins) | Solvent A (%) | Solvent B (%) | Flow (ml / min) |
|---|---|---|---|
| 0.00 | 90.0 | 10.0 | 2.00 |
| 1.50 | 10.0 | 90.0 | 2.00 |
| 1.75 | 10.0 | 90.0 | 2.00 |
| 1.90 | 90.0 | 10.0 | 2.00 |
| 2.00 | 90.0 | 10.0 | 2.00 |

| | | | |
|---|---|---|---|
| Solvent A: H₂O containing 0.1% HCOOH Solvent B: CH₃CN containing 0.1% HCOOH | | | |

LCMS RT 1.10; MH⁺ 424, 426, 428.

Further examples of compounds that were prepared using this method are listed below in Table 11.

**TABLE 11 Compounds made according to the method described in Example 13 above.**

| **Compound Number** | **Name** | **Structure** | **¹H NMR (400 MHz, CDCl₃) δ ppm** |
|---|---|---|---|
| 1-444 | 4-Cyclopropylmethy lamino-5,6-dichloro-3-trifluoromethyl-pyridine-2-carboxylic acid methyl ester | 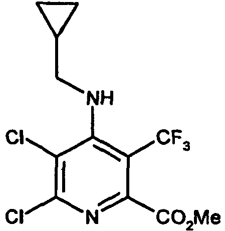 | 5.50 (m, 1H), 3.90 (s, 3H), 3.60 (m, 2H), 1.10 (m, 1H), 0.70 (m, 2H), 0.30 (m, 2H) |
| 26-440 | 6-Chloro-4-[(furan-2-yl-methyl)-amino]-3-trifluoromethyl-pyridine-2-carboxylic acid methyl ester | 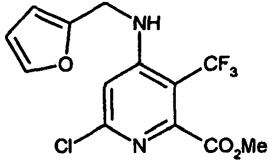 | 7.43 (m, 1H), 6.77 (s, 1H), 6.38 (m, 1H), 6.32 (m, 1H), 5.50 (br. s, 1H), 4.44 (d, 2H), 3.94 (s, 3H) |

### EXAMPLE 14

### Synthesis of 6-chloro-4-cyclopropylmethylamino-3-ethenyl-pyridine-2-carboxylic acid methyl ester (Compound 1-12).

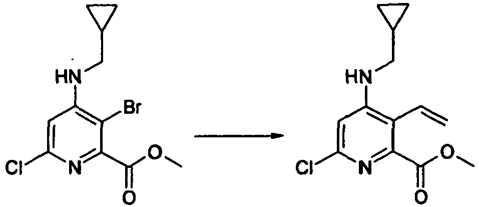

A mixture of 3-bromo-6-chloro-4-cyclopropylmethylamino-pyridine-2-carboxylic acid methyl ester (prepared as described in Example 4) (243 mg, 0.76 mmol), tributyl(vinyl)tin (265 mg, 0.84 mmol, 0.205 mL) and bis(triphenylphosphine)palladium(II) dichloride (27 mg, 0.04 mmol) in dimethylformamide (2 mL) was heated to 160 °C under microwave irradiation for 15 min, then allowed to cool. Brine (2 ml) and dichloromethane (2 ml) were added, the phases separated and the organic phase concentrated under reduced pressure. The residue was purified by reverse phase preparative HPLC, using FractionLynx (X Bridge column, ammonium acetate buffer) followed by silica gel chromatography (gradient elution: 0 - 50% ethyl acetate in iso-hexane) to give 6-chloro-4-cyclopropylmethylamino-3-ethenyl-pyridine-2-carboxylic acid methyl ester (36 mg, 18%).
Characterising data for the compound are as follows: ¹H NMR (400 MHz, CDCl₃) δ 6.75 (m, 1H), 6.52 (s, 1H), 5.67 (dd, 1H), 5.46 (dd, 1H), 5.12 (br. s, 1H), 3.89 (s, 3H), 2.98 (m, 2H), 1.09 (m, 1H), 0.62 (m, 2H), 0.27 (m, 2H) ppm.

Other compounds made using this general method are listed in Table 12 below. Characteristic data provided is either ¹H-nmr data (400 MHz, CDCl₃) δ_{H} ppm or LCMS [retention time (RT, recorded in minutes) and the molecular ion, typically the cation M+H⁺]. Compounds characterised by HPLC-MS used a Waters Acquity HPLC equipped with a Waters Atlantis dC18 column (column length 20 mm, internal diameter of column 3 mm, particle size 3 micron, temperature 40 °C), Waters TUV detector, Waters 2777 Sample manager, ESI Corona CAD detector and Micromass ZQ2000 MS. Standard MS conditions are ES+/- switching over mass range 130-700. The analysis is conducted using a two minute run time, according to the following gradient table:

| Time (mins) | Solvent A (%) | Solvent B (%) | Flow (ml / min) |
|---|---|---|---|
| 0.00 | 90.0 | 10.0 | 2.00 |
| 1.50 | 10.0 | 90.0 | 2.00 |
| 1.75 | 10.0 | 90.0 | 2.00 |
| 1.90 | 90.0 | 10.0 | 2.00 |
| 2.00 | 90.0 | 10.0 | 2.00 |

| | | | |
|---|---|---|---|
| Solvent A: H₂O containing 0.1 % HCOOH Solvent B: CH₃CN containing 0.1% HCOOH | | | |

**Table 12 - Compounds made according to the general method described in Example 14.**

| **Compoun d No.** | **Name** | **Structure** | **Characteristic data** |
|---|---|---|---|
| 1-27 | 6-Chloro-4-cyclopropylme thylamino-3-ethenyl-5-fluoro-pyridine-2-carboxylic acid methyl ester | 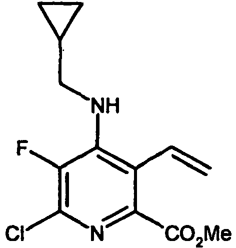 | 6.80 (m, 1H), 5.80 (m, 1H), 5.50 (m, 1H), 4.80 (m, 1H), 3.90 (s, 3H), 3.40 (m, 2H), 1.10 (m, 1H), 0.60 (m, 2H), 0.30 (m, 2H) |
| 1-39 | 4-Cyclopropylm ethylamino-5,6-dichloro-3-ethenyl-pyridine-2-carboxylic acid methyl ester | 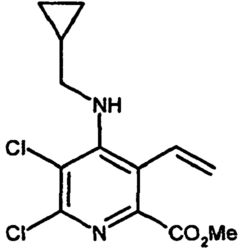 | 6.90 (m, 1H), 5.60 (m, 1H), 5.60 (m, 1H), 5.10 (m, 1H), 3.90 (s, 3H), 3.40 (m, 2H), 1.10 (m, 1H), 0.60 (m, 2H), 0.30 (m, 2H) |
| 2-12 | 6-Chloro-3-ethenyl-4-phenylmethyla mino-pyridine-2-carboxylic acid methyl ester | 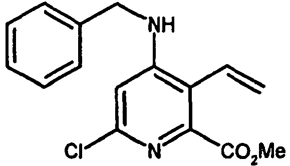 | 7.40-7.25 (m, 5H), 6.76 (m, 1H), 6.56 (s, 1H), 5.64 (dd, 1H), 5.47 (dd, 1H), 5.39 (m, 1H), 4.36 (d, 2H), 3.89 (s, 3H) |
| 3-12 | 6-Chloro-4-[(2-chlorophenylm ethyl)-amino]-3-ethenyl-pyridine-2-carboxylic acid methyl ester | 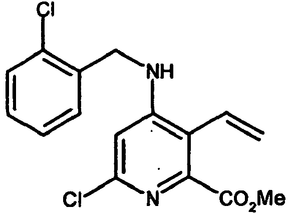 | 7.43 (m, 1H), 7.28 (m, 3H), 6.79 (m, 1H), 6.54 (s, 1H), 5.68 (dd, 1H), 5.50 (dd, 1H), 5.48 (m, 1H), 4.47 (d, 2H), 3.91 (s, 3H) |
| 6-12 | 6-Chloro-3-ethenyl-4-[(2-fluorophenylm ethyl)-amino]-pyridine-2-carboxylic acid methyl ester | 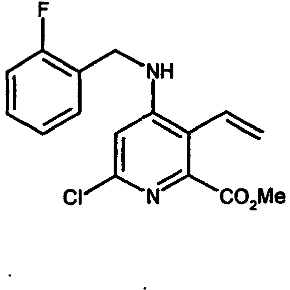 | 7.33 (m, 1H), 7.25 (m, 1H), 7.15 (m, 2H), 6.77 (m, 1H), 6.59 (s, 1H), 5.67 (dd, 1H), 5.48 (dd, 1H), 5.41 (m, 1H), 4.43 (d, 2H), 3.90 (s, 3H) |
| 9-12 | 6-Chloro-3-ethenyl-4-[(2-methylphenyl methyl)-amino]-pyridine-2-carboxylic acid methyl ester | 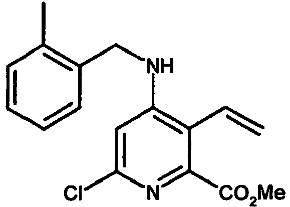 | 7.28-7.18 (m, 4H), 6.74 (m, 1H), 6.58 (s, 1H), 5.62 (dd, 1H), 5.44 (dd, 1H), 5.15 (m, 1H), 4.29 (d, 2H), 3.90 (s, 3H), 2.34 (s, 3H) |
| 14-12 | 6-Chloro-4-[(4-dimethylamino phenylmethyl)-amino]-3-ethenyl-pyridine-2-carboxylic acid methyl ester | 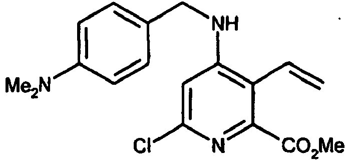 | 7.16 (d, 2H), 6.72 (m, 3H), 6.61 (s, 1H), 5.60 (dd, 1H), 5.45 (dd, 1H), 5.22 (m, 1H), 4.21 (d, 2H), 3.89 (s, 3H), 2.97 (s, 6H) |
| 15-12 | 6-Chloro-3-ethenyl-4-[(2-nitrophenylmet hyl)-amino]-pyridine-2-carboxylic acid methyl ester | 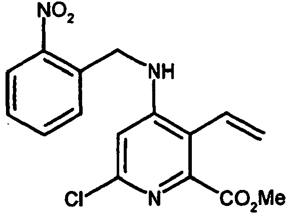 | 8.14 (dd, 1H), 7.68 (m, 1H), 7.53 (m, 2H), 6.73 (m, 1H), 6.48 (s, 1H), 5.64 (dd, 1H), 5.50 (dd, 1H), 4.85 (s, 2H), 3.86 (s, 3H) (NH not observed) (CD₃OD) |
| 15-27 | 6-Chloro-3-ethenyl-5-fluoro-4-[(2-nitrophenylmet hyl)-amino]-pyridine-2-carboxylic acid methyl ester | 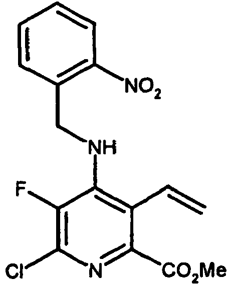 | 8.10 (m, 1H), 7.60 (m, 1H), 7.50 (m, 2H), 6.70 (m, 1H), 5.70 (m, 2H), 5.40 (m, 1H), 4.90 (m, 2H), 3.90 (s, 3H) |
| 15-39 | 5,6-Dichloro-3-ethenyl-4-[(2-nitrophenylmet hyl)-amino]-pyridine-2-carboxylic acid methyl ester | 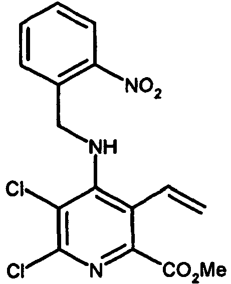 | 8.10 (m, 1H), 7.60 (m, 1H), 7.50-7.40 (m, 2H), 6.70 (m, 1H), 5.30 (m, 1H), 5.50-5.30 (m, 2H), 5.00 (m, 2H), 3.90 (s, 3H) |
| 21-12 | 6-Chloro-4-[(2,6-dichlorophenyl methyl)-amino]-3-ethenyl-pyridine-2-carboxylic acid methyl ester | 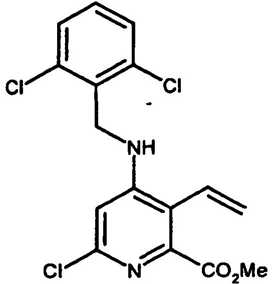 | 7.37 (m, 2H), 7.25 (m, 1H), 6.88 (s, 1H), 6.73 (m, 1H), 5.62 (d, 1H), 5.42 (d, 1H), 5.40 (m, 1H), 4.61 (d, 2H), 3.88 (s, 3H) |
| 26-12 | 6-Chloro-3-ethenyl-4-[(furan-2-ylmethyl)-amino]-pyridine-2-carboxylic acid methyl ester | 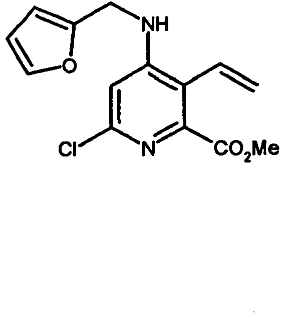 | 7.40 (d, 1H), 6.77 (m, 1H), 6.67 (s, 1H), 6.37 (m, 1H), 6.27 (d, 1H), 5.67 (dd, 1H), 5.47 (dd, 1H), 5.37 (m, 1H), 4.36 (d, 2H), 3.90 (s, 3H) |
| 26-21 (mix of E and Z isomers) | 6-Chloro-4-[(furan-2-ylmethyl)-amino]-3-(propen-1-yl)-pyridine-2-carboxylic acid methyl ester | 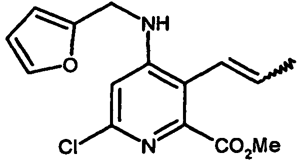 | 7.38 (m, 1 H, isomers A+B), 6.65 (s, 0.75H, isomer A), 6.62 (s, 0.25H, isomer B), 6.38-6.23 (m, 3H, isomers A+B), 6.08-6.00 (m, 0.75H, isomer A), 5.92-5.83 (m, 0.25H, isomer B), 5.27 (m, 0.25H, isomer B), 5.15 (m, 0.75H, isomer A), 4.36 (m, 2H, isomers A+B), 3.87 (s, 3H, isomers A+B), 1.90 (dd, 0.75H, isomer B), 1.49 (dd, 2.25H, isomer A) |
| 32-12 | 6-Chloro-4-[(3-chloro-pyridin-2-ylmethyl)-amino]-3-ethenyl-pyridine-2-carboxylic acid methyl ester | 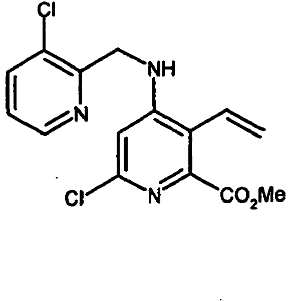 | 8.50 (d, 1H), 7.75 (d, 1H), 7.27 (m, 1H), 7.06 (m, 1H), 6.85 (m, 1H), 6.69 (m, 1H), 5.76 (dd, 1H), 5.60 (dd, 1H), 4.45 (d, 2H), 3.91 (s, 3H) |
| 32-39 | 4-[(3-Chloro-pyridin-2-ylmethyl)-amino]-5,6-dichloro-3-ethenyl-pyridine-2-carboxylic acid methyl ester | 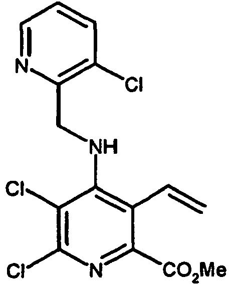 | RT 1.12; MH⁺ 372,374 |
| 32-467 | 4-[(3-Chloro-pyridin-2-ylmethyl)-amino]-3,6-(di-ethenyl)-5-fluoro-pyridine-2-carboxylic acid methyl ester | 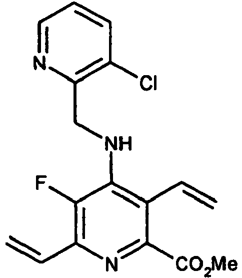 | 8.50 (m, 1 H), 7.70 (m, 2H), 7.00-6.70 (m, 2H), 6.70 (m, 1H), 6.40 (m, 1H), 5.80 (m, 1H), 5.60 (m, 2H), 4.80 (m, 2H), 3.90 (s, 3H) |
| 33-12 | 6-Chloro-3-ethenyl-4-[(3-methyl-pyridin-2-ylmethyl)-amino]-pyridine-2-carboxylic acid methyl ester | 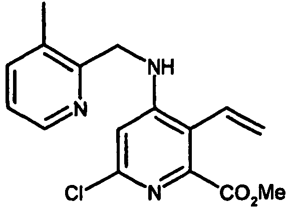 | 8.44 (m, 1H), 7.53 (d, 1H), 7.42 (br. s, 1H), 7.20 (m, 1H), 6.87 (m, 1H), 6.65 (s, 1H), 5.77 (dd, 1H), 5.62 (dd, 1H), 4.29 (d, 2H), 3.92 (s, 3H), 2.35 (s, 3H) |
| 67-12 | 6-Chloro-4-cyclohexylmet hylamino-3-ethenyl-pyridine-2-carboxylic acid methyl ester | 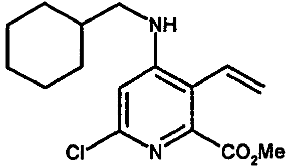 | 6.74 (m, 1H), 6.53 (s, 1H), 5.66 (d, 1H), 5.43 (d, 1H), 5.08 (m, 1H), 3.89 (s, 3H), 2.97 (t, 2H), 1.82-1.68 (m, 4H), 1.62-1.54 (m, 2H), 1.34-1.13 (m, 3H), 1.05-0.93 (m, 1H), 0.90-0.82 (m, 1H) |
| 73-12 | 6-Chloro-3-ethenyl-4-[(2-methoxypheny Imethyl)-amino]-pyridine-2-carboxylic acid methyl ester | 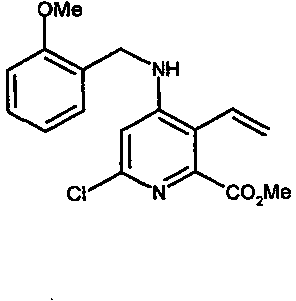 | 7.31 (m, 1H), 7.19 (dd, 1H), 6.94 (m, 2H), 6.75 (m, 1H), 6.63 (s, 1H), 5.64 (dd, 1H), 5.54 (m, 1H), 5.45 (dd, 1H), 4.35 (d, 2H), 3.89 (s, 3H), 3.88 (s, 3H) |
| 74-12 | 6-Chloro-3-ethenyl-4-[(2-trifluoromethyl phenylmethyl)-amino]-pyridine-2-carboxylic acid methyl ester | 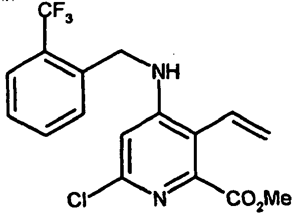 | 7.73 (d, 1H), 7.55 (m, 1H), 7.45-7.37 (m, 2H), 6.79 (m, 1H), 6.48 (s, 1H), 5.68 (dd, 1H), 5.49 (dd, 1H), 5.47 (m, 1H), 4.59 (d, 2H), 3.90 (s, 3H) |
| 75-12 | 6-Chloro-3-ethenyl-4-(thiophen-2-ylmethylamino )-pyridine-2-carboxylic acid methyl ester | 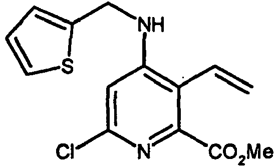 | 7.27 (m, 1H), 7.01 (m, 2H), 6.74 (m, 1H), 6.64 (s, 1H), 5.65 (dd, 1H), 5.47 (dd, 1H), 5.40 (m, 1H), 4.54 (d, 2H), 3.89 (s, 3H) |

### EXAMPLE 15

### Synthesis of 6-chloro-3-ethenyl-4-[(furan-2-ylmethyl)-amino]-pyridine-2-carboxylic acid (Compound 26-11).

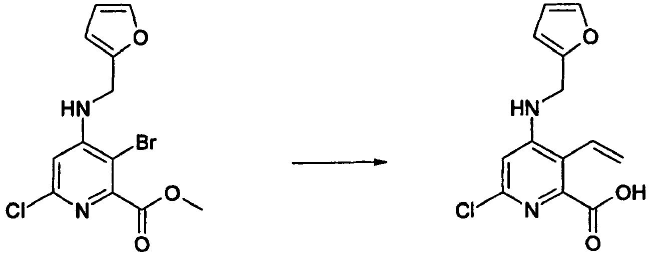

A mixture of 3-bromo-6-chloro-4-cyclopropylmethylamino-pyridine-2-carboxylic acid methyl ester (prepared as described in Example 4) (200 mg, 0.58 mmol), tributyl(vinyl)tin (202 mg, 0.64 mmol) and bis(triphenylphosphine)palladium(II) dichloride (20 mg, 0.03 mmol) in dimethylformamide (2 ml) was heated to 160 °C under microwave irradiation for 15 min, then allowed to cool. Water (2 ml) and dichloromethane (2 ml) were added, the phases separated and the organic phase concentrated under reduced pressure. The residue was dissolved in methanol (10 ml) and aqueous sodium hydroxide (2N; 5 ml) added. The resulting solution was stirred at ambient temperature for 8 hours, then acidified by the addition of hydrochloric acid (1 N) and extracted with dichloromethane. The combined organic extracts were evaporated under reduced pressure and the resulting yellow oil was purified by reverse phase preparative HPLC, using FractionLynx (X Bridge column, ammonium acetate buffer) to give 6-chloro-3-ethenyl-4-[(furan-2-ylmethyl)-amino]- pyridine-2-carboxylic acid (30 mg, 19%) as a white solid.
Characterising data for the compound are as follows: ¹H NMR (400 MHz, CDCl₃) δ 7.40 (br. s, 1H), 7.37 (s, 1H), 6.79 (m, 1H), 6.46 (s, 1H), 6.33 (m, 1H), 6.24 (m, 1H), 5.55 (m, 2H), 5.40 (d, 1H), 4.28 (d, 2H) ppm.

### EXAMPLE 16 Pre-emergence biological efficacy

Seeds of *Alopecurus myosuroides* (ALOMY), *Setaria faberi* (SETFA), *Echinochloa crus-galli* (ECHCG), *Solanum nigrum* (SOLNI), *Amaranthus retroflexus* (AMARE) and *Ipomea hederaceae* (IPOHE) were sown in standard soil in pots. After cultivation for one day under controlled conditions in a glasshouse (at 24/16°C, day/night; 14 hours light; 65 % humidity), the plants were sprayed with an aqueous spray solution derived from the formulation of the technical active ingredient in acetone / water (50:50) solution containing 0.5% Tween 20 (polyoxyethylene sorbitan monolaurate, CAS RN 9005-64-5) to give a final dose of 1000 or 250 g/ha of test compound.

The test plants were then grown under controlled conditions in the glasshouse (at 24/16°C, day/night; 14 hours light; 65 % humidity) and watered twice daily. After 13 days the test was evaluated (100 = total damage to plant; 0 = no damage to plant). Results are shown below in Table 13.

**TABLE 13 Percentage damage caused to weed species by compounds of the invention when applied pre-emergence.**

| **Compound Number** | **Rate (g/ha)** | **Species** | | | | | |
|---|---|---|---|---|---|---|---|
| | | **SOLNI** | **AMARE** | **SETFA** | **ALOMY** | **ECHCG** | **IPOHE** |
| 1-2 | 1,000 | 100 | 100 | 90 | 80 | 90 | 100 |
| 1-12 | 1,000 | 100 | 100 | 90 | 80 | 90 | 90 |
| 2-2 | 1,000 | 100 | 100 | 80 | 60 | 30 | 90 |
| 2-12 | 1,000 | 100 | 100 | 80 | 80 | 30 | 80 |
| 3-12 | 1,000 | 90 | 100 | 10 | 20 | 0 | 50 |
| 5-2 | 1,000 | 100 | 100 | 50 | 20 | 10 | 90 |
| 6-2 | 1,000 | 100 | 100 | 80 | 70 | 60 | 90 |
| 6-12 | 1,000 | 100 | 100 | 40 | 40 | 30 | 60 |
| 7-2 | 1,000 | 100 | 100 | 80 | 50 | 50 | 100 |
| 9-12 | 1,000 | 100 | 90 | 0 | 10 | 90 | 60 |
| 14-12 | 1,000 | 100 | 100 | 80 | 80 | 30 | 100 |
| 15-2 | 1,000 | 100 | 100 | 90 | 90 | 80 | 100 |
| 15-12 | 1,000 | 100 | 100 | 80 | 70 | 90 | 100 |
| 15-17 | 1,000 | 90 | 100 | 60 | 40 | 20 | 90 |
| 15-27 | 1,000 | 100 | 100 | 90 | 90 | 90 | 90 |
| 15-29 | 1,000 | 90 | 90 | 40 | 90 | 70 | 100 |
| 15-31 | 1,000 | 90 | 100 | 20 | 20 | 20 | 70 |
| 15-39 | 1,000 | 100 | 100 | 40 | 30 | 80 | 90 |
| 15-41 | 1,000 | 80 | 100 | 30 | 20 | 40 | 100 |
| 15-43 | 1,000 | 70 | 90 | 0 | 0 | 0 | 50 |
| 15-444 | 1,000 | 30 | 30 | 0 | 0 | 0 | 50 |
| 21-12 | 1,000 | 2- | 100 | 0 | 0 | 0 | 30 |
| 22-164 | 1,000 | 80 | 80 | 10 | 20 | 60 | 70 |
| 24-2 | 1,000 | 100 | 100 | 70 | 10 | 0 | 80 |
| 26-2 | 1,000 | 100 | 100 | 90 | 70 | 30 | 90 |
| 26-7 | 1,000 | 80 | 60 | 10 | 10 | 10 | 70 |
| 26-11 | 1,000 | 100 | 100 | 100 | 100 | 90 | 100 |
| 26-12 | 1,000 | 100 | 100 | 100 | 90 | 90 | 90 |
| 26-17 | 1,000 | 100 | 100 | 80 | 50 | 50 | 100 |
| 26-19 | 1,000 | 100 | 100 | 70 | 70 | 0 | 40 |
| 26-83 | 1,000 | 100 | 100 | 20 | 40 | 20 | 100 |
| 26-164 | 1,000 | 100 | 100 | 90 | 50 | 90 | 100 |
| 26-174 | 1,000 | 90 | 80 | 10 | 20 | 30 | 90 |
| 26-245 | 1,000 | 60 | 100 | 30 | 10 | 30 | 60 |
| 26-255 | 1,000 | 70 | 30 | 10 | 10 | 20 | 10 |
| 26-463 | 1,000 | 10 | 0 | 10 | 10 | 10 | 0 |
| 31-2 | 1,000 | 100 | 100 | 80 | 70 | 60 | 100 |
| 32-12 | 1,000 | 100 | 100 | 80 | 70 | 50 | 70 |
| 32-17 | 1,000 | 80 | 90 | 0 | 10 | 0 | 80 |
| 32-29 | 250 | 80 | 90 | 0 | 10 | 10 | 70 |
| 32-39 | 1,000 | 60 | 100 | 0 | 0 | 0 | 50 |
| 32-41 | 1,000 | 80 | 90 | 0 | 0 | 0 | 80 |
| 33-2 | 1,000 | 100 | 100 | 80 | 60 | 50 | 100 |
| 33-12 | 1,000 | 100 | 100 | 80 | 70 | 80 | 100 |
| 66-2 | 1,000 | 100 | 100 | 70 | 80 | 50 | 100 |
| 67-2 | 1,000 | 90 | 100 | 60 | 20 | 10 | 80 |
| 67-12 | 1,000 | 80 | 100 | 0 | 10 | 10 | 60 |
| 68-2 (*trans*) | 1,000 | 100 | 100 | 80 | 70 | 0 | 90 |
| 69-2 | 1,000 | 100 | 90 | 10 | 20 | 10 | 80 |
| 70-2 | 1,000 | 100 | 100 | 80 | 30 | 20 | 70 |
| 71-2 | 1,000 | 100 | 100 | 80 | 70 | 20 | 100 |
| 73-2 | 1,000 | 100 | 100 | 80 | 50 | 20 | 100 |
| 72-2 | 1,000 | 100 | 90 | 30 | 30 | 10 | 80 |
| 73-12 | 1,000 | 100 | 100 | 80 | 70 | 20 | 70 |
| 74-2 | 1,000 | 90 | 90 | 10 | 0 | 0 | 70 |
| 74-12 | 1,000 | 0 | 0 | 0 | 0 | 0 | 0 |
| 75-2 | 1,000 | 100 | 100 | 80 | 70 | 60 | 90 |
| 75-12 | 1,000 | 100 | 100 | 70 | 80 | 60 | 80 |
| 77-2 | 1,000 | 100 | 100 | 70 | 60 | 20 | 90 |
| 78-2 | 1,000 | 100 | 100 | 50 | 40 | 20 | 90 |
| 80-2 | 1,000 | 100 | 100 | 80 | 70 | 20 | 90 |
| 81-2 | 1,000 | 100 | 100 | 70 | 70 | 70 | 90 |

### EXAMPLE 17 Post-emergence biological efficacy

Seeds of *Alopecurus myosuroides* (ALOMY), *Setaria faberi* (SETFA), *Echinochloa crus-galli* (ECHCG), *Solanum nigrum* (SOLNI), *Amaranthus retroflexus* (AMARE) and *Ipomea hederaceae* (IPOHE) were sown in standard soil in pots. After cultivation for 8 days under controlled conditions in a glasshouse (at 24/16°C, day/night; 14 hours light; 65 % humidity), the plants were sprayed with an aqueous spray solution derived from the formulation of the technical active ingredient in acetone / water (50:50) solution containing 0.5% Tween 20 (polyoxyethylene sorbitan monolaurate, CAS RN 9005-64-5) to give a final dose of 1000 or 250 g/ha of test compound.

The test plants were then grown on under controlled conditions in a glasshouse (at 24/16°C, day/night; 14 hours light; 65 % humidity) and watered twice daily. After 13 days the test was evaluated (100 = total damage to plant; 0 = no damage to plant). Results are shown below in Table 14.

**TABLE 14 Percentage damage caused to weed species by compounds of the invention when applied post-emergence**

| **Compound Number** | **Rate (g/ha)** | **Species** | | | | | |
|---|---|---|---|---|---|---|---|
| | | **SOLNI** | **AMARE** | **SETFA** | **ALOMY** | **ECHCG** | **IPOHE** |
| 1-2 | 1,000 | 90 | 100 | 90 | 90 | 70 | 80 |
| 1-12 | 1,000 | 90 | 100 | 90 | 70 | 80 | 70 |
| 2-2 | 1,000 | 100 | 100 | 80 | 50 | 60 | 70 |
| 2-12 | 1,000 | 100 | 100 | 80 | 60 | 50 | 70 |
| 3-12 | 1,000 | 90 | 100 | 10 | 0 | 20 | 40 |
| 5-2 | 1,000 | 100 | 100 | 70 | 50 | 50 | 70 |
| 6-2 | 1,000 | 100 | 100 | 90 | 60 | 40 | 70 |
| 6-12 | 1,000 | 90 | 100 | 80 | 20 | 20 | 70 |
| 7-2 | 1,000 | 100 | 100 | 90 | 50 | 50 | 80 |
| 9-12 | 1,000 | 80 | 100 | 50 | 40 | 50 | 40 |
| 14-12 | 1,000 | 90 | 100 | 80 | 60 | 50 | 50 |
| 15-2 | 1,000 | 100 | 100 | 90 | 70 | 70 | 90 |
| 15-12 | 1,000 | 90 | 100 | 100 | 80 | 70 | 60 |
| 15-17 | 1,000 | 90 | 80 | 80 | 50 | 30 | 80 |
| 15-27 | 1,000 | 100 | 100 | 90 | 70 | 70 | 90 |
| 15-29 | 1,000 | 100 | 100 | 80 | 50 | 50 | 70 |
| 15-31 | 1,000 | 100 | 100 | 80 | 20 | 50 | 70 |
| 15-39 | 1,000 | 100 | 100 | 80 | 60 | 60 | 90 |
| 15-41 | 1,000 | 100 | 100 | 70 | 10 | 0 | 80 |
| 15-43 | 1,000 | 100 | 100 | 40 | 40 | 10 | 70 |
| 15-444 | 1,000 | 90 | 100 | 40 | 10 | 50 | 50 |
| 21-12 | 1,000 | 80 | 100 | 0 | 20 | 10 | 30 |
| 22-164 | 1,000 | 90 | 100 | 60 | 0 | 60 | 70 |
| 24-2 | 1,000 | 100 | 100 | 80 | 50 | 20 | 70 |
| 26-2 | 1,000 | 100 | 100 | 90 | 40 | 30 | 60 |
| 26-7 | 1,000 | 70 | 40 | 0 | 0 | 0 | 30 |
| 26-11 | 1,000 | 100 | 100 | 90 | 80 | 80 | 80 |
| 26-12 | 1,000 | 100 | 100 | 90 | 80 | 80 | 70 |
| 26-17 | 1,000 | 90 | 100 | 80 | 70 | 60 | 60 |
| 26-19 | 1,000 | 90 | 100 | 70 | 60 | 30 | 40 |
| 26-83 | 1,000 | 90 | 100 | 0 | 0 | 0 | 100 |
| 26-164 | 1,000 | 100 | 100 | 90 | 70 | 70 | 90 |
| 26-174 | 1,000 | 100 | 100 | 0 | 10 | 20 | 50 |
| 26-245 | 1,000 | 90 | 100 | 60 | 40 | 50 | 60 |
| 26-255 | 1,000 | 70 | 20 | 0 | 0 | 0 | 10 |
| 26-463 | 1,000 | 60 | 20 | 0 | 0 | 0 | 40 |
| 31-2 | 1,000 | 100 | 100 | 80 | 50 | 20 | 80 |
| 32-12 | 1,000 | 90 | 100 | 60 | 50 | 40 | 50 |
| 32-17 | 1,000 | 90 | 80 | 50 | 20 | 20 | 50 |
| 32-29 | 250 | 80 | 70 | 60 | 20 | 50 | 60 |
| 32-39 | 1,000 | 90 | 100 | 50 | 10 | 20 | 40 |
| 32-41 | 1,000 | 90 | 90 | 60 | 20 | 20 | 70 |
| 33-2 | 1,000 | 100 | 100 | 70 | 60 | 60 | 80 |
| 33-12 | 1,000 | 90 | 100 | 90 | 80 | 80 | 70 |
| 66-2 | 1,000 | 80 | 100 | 0 | 20 | 20 | 60 |
| 67-2 | 1,000 | 100 | 100 | 20 | 30 | 20 | 70 |
| 67-12 | 1,000 | 80 | 90 | 20 | 10 | 20 | 60 |
| 68-2 (*trans*) | 1,000 | 100 | 100 | 70 | 40 | 20 | 70 |
| 69-2 | 1,000 | 90 | 100 | 0 | 10 | 0 | 70 |
| 70-2 | 1,000 | 100 | 100 | 60 | 40 | 30 | 70 |
| 71-2 | 1,000 | 100 | 100 | 50 | 20 | 20 | 60 |
| 72-2 | 1,000 | 100 | 100 | 70 | 20 | 10 | 70 |
| 73-2 | 1,000 | 90 | 100 | 80 | 40 | 40 | 70 |
| 73-12 | 1,000 | 90 | 100 | 80 | 20 | 20 | 50 |
| 74-2 | 1,000 | 100 | 100 | 0 | 10 | 0 | 60 |
| 74-12 | 1,000 | 70 | 100 | 10 | 10 | 20 | 10 |
| 75-2 | 1,000 | 100 | 100 | 80 | 60 | 70 | 70 |
| 75-12 | 1,000 | 100 | 100 | 80 | 50 | 70 | 50 |
| 77-2 | 1,000 | 100 | 100 | 80 | 50 | 30 | 80 |
| 78-2 | 1,000 | 100 | 100 | 50 | 20 | 20 | 70 |
| 80-2 | 1,000 | 100 | 90 | 50 | 40 | 60 | 70 |
| 81-2 | 1,000 | 100 | 100 | 80 | 40 | 40 | 60 |

## Claims

1. A compound having the formula (I): 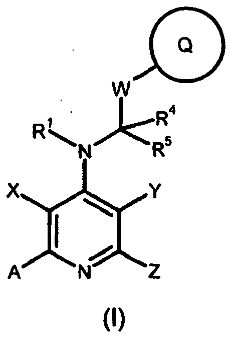 or a salt or N-oxide thereof,
wherein:
A is halogen, C1-C6 alkyl optionally substituted by 1 to 3 groups R², C1-C6 haloalkyl optionally substituted by 1 to 3 groups R², C2-C6 alkenyl optionally substituted by 1 to 3 groups R², C3-C8 cycloalkyl optionally substituted by 1 to 3 groups R², C1-C6 alkylthio optionally substituted by 1 to 3 groups R², C6-C10 aryl optionally substituted by 1 to 3 groups R³, a mono- or bicyclic heteroaryl group having 3 to 10 ring atoms and at least one ring atom which is nitrogen, oxygen or sulfur optionally substituted by 1 to 3 groups R³;
R¹ is hydrogen, C1-C6 alkyl optionally substituted by 1 to 3 groups R², C2-C6 alkenyl optionally substituted by 1 to 3 groups R², C2-C6 alkynyl optionally substituted by 1 to 3 groups R², C3-C8 cycloalkyl optionally substituted by 1 to 3 groups R², C1-C6 acyl optionally substituted by 1 to 3 groups R², C6-C10 aryl optionally substituted by 1 to 3 groups R³, a mono- or bicyclic heteroaryl group having 3 to 10 ring atoms and at least one ring atom which is nitrogen, oxygen or sulfur optionally substituted by 1 to 3 groups R³, C1-C6 alkylsulphonyl optionally substituted by 1 to 3 groups R², C2-C7 alkoxycarbonyl optionally substituted by 1 to 3 groups R², aminocarbonyl, C1-C6 alkylaminocarbonyl optionally substituted by 1 to 3 groups R², di C1-C6 alkylaminocarbonyl optionally substituted by 1 to 3 groups R²_{;}
R⁴ is hydrogen, cyano, nitro, C1-C6 alkyl optionally substituted by 1 to 3 groups R², C1-C6 haloalkyl optionally substituted by 1 to 3 groups R², C3-C6 cycloalkyl optionally substituted by 1 to 3 groups R², C2-C6 alkenyl optionally substituted by 1 to 3 groups R², C6-C10 aryl optionally substituted by 1 to 3 groups R³, a mono- or bicyclic heteroaryl group having 3 to 10 ring atoms and at least one ring atom which is nitrogen, oxygen or sulfur optionally substituted by 1 to 3 groups R³, C1-C6 acyl optionally substituted by 1 to 3 groups R², C1-C6 alkoxycarbonyl optionally substituted by 1 to 3 groups R², carboxy, aminocarbonyl, C1-C6 alkylaminocarbonyl optionally substituted by 1 to 3 groups R², di C1-C6 alkylaminocarbonyl optionally substituted by 1 to 3 groups R², or diC1-C6 alkylphosphonyl optionally substituted by 1 to 3 groups R²;
R⁵ is hydrogen, C1-C6 alkyl optionally substituted by 1 to 3 groups R², or C1-C6 haloalkyl;
W is a direct bond or a linker group of the formula -(C1-C3 alkylene)ₛ-L-(C1-C3 alkylene)ᵣ wherein each alkylene group is optionally substituted by up to 3 groups R², s and t may each be independently 0 or 1, and L is a direct single, double or triple bond, -S(O)ᵤ- wherein u is 0, 1 or 2, -N(R¹¹)- wherein R¹¹ is H or C1-C6 alkyl, -O- or -C(O)O-;
Q is a 3-10 membered ring system optionally containing up to 4 heteroatoms independently selected from O, N or S, the ring system optionally substituted by up to three substituents R³;
X is hydrogen, halogen, cyano, nitro, hydroxyl, C1-C6 alkyl optionally substituted by 1 to 3 groups R², C1-C6 haloalkyl optionally substituted by 1 to 3 groups R², C2-C6 alkenyl optionally substituted by 1 to 3 groups R², C2-C6 alkynyl optionally substituted by 1 to 3 groups R², C1-C6 haloalkoxy, C3-C8 cycloalkyl optionally substituted by 1 to 3 groups R², C6-C10 aryl optionally substituted by 1 to 3 groups R³, a mono- or bicyclic heteroaryl group having 3 to 10 ring atoms and at least one ring atom which is nitrogen, oxygen or sulfur optionally substituted by 1 to 3 groups R³, C1-C6 alkoxy optionally substituted by 1 to 3 groups R², amino, C1-C6 alkylamino optionally substituted by 1 to 3 groups R², di(C1-C6 alkyl)amino optionally substituted by 1 to 3 groups R², C1-C6 alkylthio optionally substituted by 1 to 3 groups R², C1-C6 alkylsulphinyl optionally substituted by 1 to 3 groups R², C1-C6 alkylsulphonyl optionally substituted by 1 to 3 groups R², di(C1-C6 alkyl) phosphonyl, tri(C1-C6 alkyl)silyl;
Y is halogen, cyano, nitro, hydroxyl, C1-C6 alkyl optionally substituted by 1 to 3 groups R², C2-C6 alkenyl optionally substituted by 1 to 3 groups R², C2-C6 alkynyl optionally substituted by 1 to 3 groups R², C1-C6 haloalkyl optionally substituted by 1 to 3 groups R², C1-C6 haloalkoxy, C1-C6 alkoxy optionally substituted by 1 to 3 groups R², C3-C8 cycloalkyl optionally substituted by 1 to 3 groups R², C3-C8 cycloalkoxy optionally substituted by 1 to 3 groups R², C6-C10 aryl optionally substituted by 1 to 3 groups R³, a mono- or bicyclic heteroaryl group having 3 to 10 ring atoms and at least one ring atom which is nitrogen, oxygen or sulfur optionally substituted by 1 to 3 groups R³, amino, C1-C6 alkylamino, di(C1-C6 alkyl)amino, C1-C6 alkylthio, C1-C6 alkylsulphinyl, C1-C6 alkylsulphonyl, di(C1-C6 alkyl) phosphonyl or tri(C1-C6 alkyl)silyl;
Z is C(O)R^{6,} C(S)R⁶, or C(=NR⁷)R⁸;
each R² is independently halogen, hydroxyl, amino, C1-C3 alkylamino, di (C1-C3) alkylamino, carboxy, cyano, C1-C3 alkyl, C1-C3 haloalkyl, C3-C6 cycloalkyl, C1-C3 alkoxy, C1-C3 haloalkoxy, C1-C3 alkylthio, C1-C3 alkylsulphonyl, C2-C6 carboxyalkyl, carboxy, C2-C6 alkoxycarbonyl, C2-C7 alkylcarbonyloxy, phenyl, or phenoxy;
each R³ is independently halogen, hydroxyl, nitro, amino, thiol, cyano, C1-C3 alkyl, C1-C3 haloalkyl, C1-C3 alkoxy, C1-C3 haloalkoxy, C1-C3 alkylthio, C1-C3 haloalkylthio, C2-C6 carboxyalkyl, C2-C6 alkoxycarbonyl, C2-C7 alkylcarbonyloxy, phenyl, phenoxy, C1-C3 alkylamino, or di(C1-C3 alkyl)amino;
R⁶ is hydrogen, hydroxyl, C1-C10 alkoxy optionally substituted by C1-C6 alkoxy or phenyl, C3-C8 cycloalkoxy optionally substituted by C1-C6 alkoxy or phenyl, C1-C6 haloalkoxy, C2-C6 alkenyloxy, C1-C6 alkylthio, amino, C1-C6 alkylamino, or di(C1-C6 alkyl)amino;
R⁷ is hydrogen, C1-C6 alkyl, C1-C6 alkoxy, C3-C8 cycloalkoxy, amino, C1-C6 alkylamino, or di(C1-C6 alkyl)amino;
R⁸ is hydrogen, C1-C6 alkoxy, C3-C8 cycloalkoxy, C1-C6 alkylthio, amino, C1-C6 alkylamino, or di(C1-C6 alkyl)amino.

2. A compound according to claim 1 wherein A is halogen, phenyl optionally substituted by 1 to 3 groups R³, or C3-C6 cycloalkyl optionally substituted by 1 to 3 groups R².

3. A compound according to claim 1 or 2 wherein R¹ is hydrogen or C1-C6 alkyl.

4. A compound according to any preceding claim wherein X is hydrogen, halogen, C1-C6 alkyl, C1-C6 haloalkyl, C1-C3 alkoxy(C1-C6)alkyl, or C3-C6 cycloalkyl.

5. A compound according to any preceding claim wherein Y is halogen, C1-C3 alkyl, C1-C3 haloalkyl, C1-C2 alkoxy(C1-C2)alkyl, cyclopropyl, C2-C4 alkenyl, or C2-C4 alkynyl.

6. A compound according to any preceding claim wherein R⁴ is hydrogen. C1-C6 alkyl, C1-C6 haloalkyl, C1-C6 hydroxyalkyl, C1-C3 alkoxy(C1-C6)alkyl, C2-C6 alkoxycarbonyl or carboxyl.

7. A compound according to any preceding claim wherein R⁵ is hydrogen or C1-C6 alkyl.

8. A compound according to any preceding claim wherein W is a direct bond or a methylene group.

9. A compound according to any preceding claim, wherein Q is phenyl, pyridyl, furyl, thiophenyl, oxazolyl, thiazolyl, each optionally substituted by 1 or 2 groups R³, or C3-C8 cycloalkyl optionally substituted by 1 or 2 groups R³
wherein
each R³ is independently halogen, nitro, cyano, C1-C2 alkyl, C1-C2 haloalkyl, C1-C2 alkoxy, C1-C2 haloalkoxy or C2-C3 alkoxycarbonyl, amino or di(C1-C2alkyl)amino.

10. A compound according to any preceding claim, wherein X is hydrogen, halogen, C1-C2 alkyl, C1-C2 haloalkyl, C1-C2 alkoxy(C1-C2)alkyl, or C3-C6 cycloalkyl.

11. A compound according to any preceding claim, wherein Y is halogen, C1-C2 alkyl, C1-C2 haloalkyl, C1-C2 alkoxy(C1-C2)alkyl, or C2-4 alkenyl.

12. A compound according to any preceding claim wherein Z is C(O)R⁶, wherein R⁶ is hydroxyl, C1-C6 alkoxy, phenyl(C1-C2)alkoxy, or (C1-C3)alkoxy(C1-C6)alkoxy.

13. A compound according to claim 1, wherein A is halogen, R¹ is hydrogen, X is hydrogen or halogen, Y is halogen, methyl or vinyl, Z is C(O)R⁶, wherein R⁶ is hydroxyl or C1-C6 alkoxy, R⁴ and R⁵ are both hydrogen, W is a direct bond and Q is a phenyl, furanyl, pyridyl or cyclopropyl ring optionally substituted by up to three substituents R³, wherein each R³ is independently halogen, hydroxyl, nitro, amino, C1-C3 alkyl, C1-C3 haloalkyl, C1-C3 alkoxy, C1-C3 haloalkoxy, C2-C6 alkoxycarbonyl, C2-C7 alkylcarbonyloxy, C1-C3 alkylamino, or di(C1-C3 alkyl)amino.

14. A herbicidal composition comprising a compound as defined in any one of claims 1 to 13 together with at least one agriculturally acceptable adjuvant or diluent.

15. A composition according to claim 14 which comprises a further herbicide in addition to the compound of formula (I).

16. A composition according to claim 14 or 15 which comprises a safener.

17. Use of a compound as defined in any one of claims 1 to 13 or a composition as defined in any one of claims 14 to 16 as a herbicide.

18. A method of controlling weeds in crops of useful plants, comprising applying to said weeds or to the locus of said weeds, or to said useful crop plants, a compound as defined in any one of claims 1 to 13 or a composition as claimed in any one of claims 14 to 16.

## Patentansprüche

1. Verbindung der Formel (I): 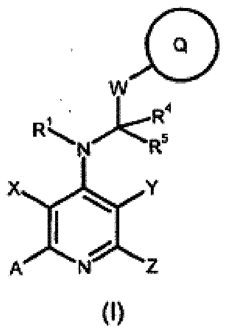 oder ein Salz oder N-Oxid davon,
mit den folgenden Bedeutungen:
A bedeutet Halogen, C1-C6-Alkyl, das gegebenenfalls durch 1 bis 3 Gruppen R² substituiert ist, C1-C6-Halogenalkyl, das gegebenenfalls durch 1 bis 3 Gruppen R² substituiert ist, C2-C6-Alkenyl, das gegebenenfalls durch 1 bis 3 Gruppen R² substituiert ist, C3-C8-Cycloalkyl, das gegebenenfalls durch 1 bis 3 Gruppen R² substituiert ist, C1-C6-Alkylthio, das gegebenenfalls durch 1 bis 3 Gruppen R² substituiert ist, C6-C10-Aryl, das gegebenenfalls durch 1 bis 3 Gruppen R³ substituiert ist, mono- oder bicyclische Heteroarylgruppe mit 3 bis 10 Ringatomen und mindestens einem Ringatom, bei dem es sich um Stickstoff, Sauerstoff oder Schwefel handelt und das gegebenenfalls durch 1 bis 3 Gruppen R³ substituiert ist;
R¹ bedeutet Wasserstoff, C1-C6-Alkyl, das gegebenenfalls durch 1 bis 3 Gruppen R² substituiert ist, C2-C6-Alkenyl, das gegebenenfalls durch 1 bis 3 Gruppen R² substituiert ist, C2-C6-Alkinyl, das gegebenenfalls durch 1 bis 3 Gruppen R² substituiert ist, C3-C8-Cycloalkyl, das gegebenenfalls durch 1 bis 3 Gruppen R² substituiert ist, C1-C6-Acyl das gegebenenfalls durch 1 bis 3 Gruppen R² substituiert ist, C6-C10-Aryl, das gegebenenfalls durch 1 bis 3 Gruppen R³ substituiert ist, mono- oder bicyclische Heteroarylgruppe mit 3 bis 10 Ringatomen und mindestens einem Ringatom, bei dem es sich um Stickstoff, Sauerstoff oder Schwefel handelt, das gegebenenfalls durch 1 bis 3 Gruppen R³ substituiert ist, C1-C6-Alkylsulfonyl, das gegebenenfalls durch 1 bis 3 Gruppen R² substituiert ist, C2-C7-Alkoxycarbonyl, das gegebenenfalls durch 1 bis 3 Gruppen R² substituiert ist, Aminocarbonyl, C1-C6-Alkylaminocarbonyl, das gegebenenfalls durch 1 bis 3 Gruppen R² substituiert ist, Di-C1-C6-alkylaminocarbonyl, das gegebenenfalls durch 1 bis 3 Gruppen R² substituiert ist;
R⁴ bedeutet Wasserstoff, Cyano, Nitro, C1-C6-Alkyl, das gegebenenfalls durch 1 bis 3 Gruppen R² substituiert ist, C1-C6-Halogenalkyl, das gegebenenfalls durch 1 bis 3 Gruppen R² substituiert ist, C3-C6-Cycloalkyl, das gegebenenfalls durch 1 bis 3 Gruppen R² substituiert ist, C2-C6-Alkenyl, das gegebenenfalls durch 1 bis 3 Gruppen R² substituiert ist, C6-C10-Aryl, das gegebenenfalls durch 1 bis 3 Gruppen R³ substituiert ist, mono- oder bicyclische Heteroarylgruppe mit 3 bis 10 Ringatomen und mindestens einem Ringatom, bei dem es sich um Stickstoff, Sauerstoff oder Schwefel handelt, das gegebenenfalls durch 1 bis 3 Gruppen R³ substituiert ist, C1-C6-Acyl, das gegebenenfalls durch 1 bis 3 Gruppen R² substituiert ist, C1-C6-Alkoxycarbonyl, das gegebenenfalls durch 1 bis 3 Gruppen R² substituiert ist, Carboxyl, Aminocarbonyl, C1-C6-Alkylaminocarbonyl, das gegebenenfalls durch 1 bis 3 Gruppen R² substituiert ist, Di-C1-C6-alkylaminocarbonyl, das gegebenenfalls durch 1 bis 3 Gruppen R² substituiert ist, oder Di-C1-C6-Alkylphosphonyl, das gegebenenfalls durch 1 bis 3 Gruppen R² substituiert ist;
R⁵ bedeutet Wasserstoff, C1-C6-Alkyl, das gegebenenfalls durch 1 bis 3 Gruppen R² substituiert ist oder C1-C6-Halogenalkyl;
W bedeutet eine direkte Bindung oder eine Linkergruppe der Formel -(C1-C3-Alkylen)ₛ-L-(C1-C3-Alkylen)ₜ-, wobei jede Alkylengruppe gegebenenfalls durch bis zu 3 Gruppen R² substituiert ist, s und t jeweils unabhängig 0 oder 1 bedeuten können und L eine direkte Einfach-, Doppel- oder Dreifachbindung bedeutet, -S(O)ᵤ-, worin u 0, 1 oder 2 bedeutet, -N(R¹¹)-, worin R¹¹ H oder C1-C6-Alkyl bedeutet, -0- oder -C(O)O- bedeutet;
Q bedeutet ein 3-10-gliedriges Ringsystem, das gegebenenfalls bis zu 4 Heteroatome, die unabhängig aus der Reihe 0, N oder S ausgewählt sind, enthält, wobei das Ringsystem gegebenenfalls durch bis zu drei Substituenten R³ substituiert ist;
X bedeutet Wasserstoff, Halogen, Cyano, Nitro, Hydroxyl, C1-C6-Alkyl, das gegebenenfalls durch 1 bis 3 Gruppen R² substituiert ist, C1-C6-Halogenalkyl, das gegebenenfalls durch 1 bis 3 Gruppen R² substituiert ist, C2-C6-Alkenyl, das gegebenenfalls durch 1 bis 3 Gruppen R² substituiert ist, C2-C6-Alkinyl, das gegebenenfalls durch 1 bis 3 Gruppen R² substituiert ist, C1-C6-Halogenalkoxy, C3-C8-Cycloalkyl, das gegebenenfalls durch 1 bis 3 Gruppen R² substituiert ist, C6-C10-Aryl, das gegebenenfalls durch 1 bis 3 Gruppen R³ substituiert ist, eine mono- oder bicyclische Heteroarylgruppe mit 3 bis 10 Ringatomen und mindestens **einem Ringatom**, bei dem es sich um Stickstoff, Sauerstoff oder Schwefel handelt, das gegebenenfalls durch 1 bis 3 Gruppen R³ substituiert ist, C1-C6-Alkoxy, das gegebenenfalls durch 1 bis 3 Gruppen R² substituiert ist, Amino, C1-C6-Alkylamino, das gegebenenfalls durch 1 bis 3 Gruppen R² substituiert ist, Di-(C1-C6-alkyl)amino, das gegebenenfalls durch 1 bis 3 Gruppen R² substituiert ist, C1-C6-Alkylthio, das gegebenenfalls durch 1 bis 3 Gruppen R² substituiert ist, C1-C6-Alkylsulfinyl, das gegebenenfalls durch 1 bis 3 Gruppen R² substituiert ist, C1-C6-Alkylsulfonyl, das gegebenenfalls durch 1 bis 3 Gruppen R² substituiert ist, Di-(C1-C6-alkyl)phosphonyl, Tri-(C1-C6-alkyl)silyl;
Y bedeutet Halogen, Cyano, Nitro, Hydroxyl, C1-C6-Alkyl, das gegebenenfalls durch 1 bis 3 Gruppen R² substituiert ist, C2-C6-Alkenyl, das gegebenenfalls durch 1 bis 3 Gruppen R² substituiert ist, C2-C6-Alkinyl, das gegebenenfalls durch 1 bis 3 Gruppen R² substituiert ist, C1-C6-Halogenalkyl, das gegebenenfalls durch 1 bis 3 Gruppen R² substituiert ist, C1-C6-Halogenalkoxy, C1-C6-Alkoxy, das gegebenenfalls durch 1 bis 3 Gruppen R² substituiert ist, C3-C8-Cycloalkyl, das gegebenenfalls durch 1 bis 3 Gruppen R² substituiert ist, C3-C8-Cycloalkoxy, das gegebenenfalls durch 1 bis 3 Gruppen R² substituiert ist, C6-C10-Aryl, das gegebenenfalls durch 1 bis 3 Gruppen R³ substituiert ist, eine mono- oder bicyclische Heteroarylgruppe mit 3 bis 10 Ringatomen und mindestens einem Ringatom, bei dem es sich um Stickstoff, Sauerstoff oder Schwefel handelt, das gegebenenfalls durch 1 bis 3 Gruppen R³ substituiert ist, Amino, C1-C6-Alkylamino, Di-(C1-C6-alkyl)amino, C1-C6-Alkylthio, C1-C6-Alkylsulfinyl, C1-C6-Alkylsulfonyl, Di-(C1-C6-alkyl)phosphonyl oder Tri-(C1-C6-alkyl)silyl;
Z bedeutet C(O)R⁶, C(S)R⁶ oder C(=NR⁷)R⁸;
R² bedeutet jeweils unabhängig Halogen, Hydroxyl, Amino, C1-C3-Alkylamino, Di-(C1-C3)-Alkylamino, Carboxyl, Cyano, C1-C3-Alkyl, C1-C3-Halogenalkyl, C3-C6-Cycloalkyl, C1-C3-Alkoxy, C1-C3-Halogenalkoxy, C1-C3-Alkylthio, C1-C3-Alkylsulfonyl, C2-C6-Carboxyalkyl, Carboxyl, C2-C6-Alkoxycarbonyl, C2-C7-Alkylcarbonyloxy, Phenyl oder Phenoxy;
R³ bedeutet jeweils unabhängig Halogen, Hydroxyl, Nitro, Amino, Thiol, Cyano, C1-C3-Alkyl, C1-C3-Halogenalkyl, C1-C3-Alkoxy, C1-C3-Halogenalkoxy, C1-C3-Alkylthio, C1-C3-Halogenalkylthio, C2-C6-Carboxyalkyl, C2-C6-Alkoxycarbonyl, C2-C7-Alkylcarbonyloxy, Phenyl, Phenoxy, C1-C3-Alkylamino oder Di-(C1-C3-alkyl)amino;
R⁶ bedeutet Wasserstoff, Hydroxyl, C1-C10-Alkoxy, das gegebenenfalls durch C1-C6-Alkoxy oder Phenyl substituiert ist, C3-C8-Cycloalkoxy, das gegebenenfalls durch C1-C6-Alkoxy oder Phenyl substituiert ist, C1-C6-Halogenalkoxy, C2-C6-Alkenyloxy, C1-C6-Alkylthio, Amino, C1-C6-Alkylamino oder Di-(C1-C6-alkyl)amino;
R⁷ bedeutet Wasserstoff, C1-C6-Alkyl, C1-C6-Alkoxy, C3-C8-Cycloalkoxy, Amino, C1-C6-Alkylamino oder Di-(C1-C6-alkyl)amino;
R⁸ bedeutet Wasserstoff, C1-C6-Alkoxy, C3-C8-Cycloalkoxy, C1-C6-Alkylthio, Amino, C1-C6-Alkylamino oder Di-(C1-C6-alkyl)amino.

2. Verbindung nach Anspruch 1, wobei A Halogen, Phenyl, das gegebenenfalls durch 1 bis 3 Gruppen R³ substituiert ist, oder C3-C6-Cycloalkyl, das gegebenenfalls durch 1 bis 3 Gruppen R² substituiert ist, bedeutet.

3. Verbindung nach Anspruch 1 oder 2, wobei R¹ Wasserstoff oder C1-C6-Alkyl bedeutet.

4. Verbindung nach einem vorhergehenden Anspruch, wobei X Wasserstoff, Halogen, C1-C6-Alkyl, C1-C6-Halogenalkyl, C1-C3-Alkoxy-(C1-C6)-alkyl oder C3-C6-Cycloalkyl bedeutet.

5. Verbindung nach einem vorhergehenden Anspruch, wobei Y Halogen, C1-C3-Alkyl, C1-C3-Halogenalkyl, C1-C2-Alkoxy-(C1-C2)-alkyl, Cyclopropyl, C2-C4-Alkenyl oder C2-C4-Alkinyl bedeutet.

6. Verbindung nach einem vorhergehenden Anspruch, wobei R⁴ Wasserstoff, C1-C6-Alkyl, C1-C6-Halogenalkyl, C1-C6-Hydroxyalkyl, C1-C3-Alkoxy-(C1-C6)-alkyl, C2-C6-Alkoxycarbonyl oder Carboxyl bedeutet.

7. Verbindung nach einem vorhergehenden Anspruch, wobei R⁵ Wasserstoff oder C1-C6-Alkyl bedeutet.

8. Verbindung nach einem vorhergehenden Anspruch, wobei W eine direkte Bindung oder eine Methylengruppe bedeutet.

9. Verbindung nach einem vorhergehenden Anspruch, wobei Q Phenyl, Pyridyl, Furyl, Thiophenyl, Oxazolyl, Thiazolyl, das jeweils gegebenenfalls durch 1 oder 2 Gruppen R³ substituiert ist, oder C3-C8-Cycloalkyl, das gegebenenfalls durch 1 oder 2 Gruppen R³ substituiert ist, bedeutet,
wobei
R³ jeweils unabhängig Halogen, Nitro, Cyano, C1-C2-Alkyl, C1-C2-Halogenalkyl, C1-C2-Alkoxy, C1-C2-Halogenalkoxy oder C2-C3-Alkoxycarbonyl, Amino oder Di-(C1-C2-alkyl)amino bedeutet.

10. Verbindung nach einem vorhergehenden Anspruch, wobei X Wasserstoff, Halogen, C1-C2-Alkyl, C1-C2-Halogenalkyl, C1-C2-Alkoxy-(C1-C2)-alkyl oder C3-C6-Cycloalkyl bedeutet.

11. Verbindung nach einem vorhergehenden Anspruch, wobei Y Halogen, C1-C2-Alkyl, C1-C2-Halogenalkyl, C1-C2-Alkoxy-(C1-C2)-alkyl oder C2-4-Alkenyl bedeutet.

12. Verbindung nach einem vorhergehenden Anspruch, wobei Z C(O)R⁶ bedeutet, wobei R⁶ Hydroxyl, C1-C6-Alkoxy, Phenyl-(C1-C2)-alkoxy oder (C1-C3)-Alkoxy-(C1-C6)-alkoxy bedeutet.

13. Verbindung nach Anspruch 1, wobei A Halogen bedeutet, R¹ Wasserstoff bedeutet, X Wasserstoff oder Halogen bedeutet, Y Halogen, Methyl oder Vinyl bedeutet, Z C(O)R⁶ bedeutet, wobei R⁶ Hydroxyl oder C1-C6-Alkoxy bedeutet, R⁴ und R⁵ beide Wasserstoff bedeuten, W eine direkte Bindung bedeutet und Q einen Phenyl-, Furanyl-, Pyridyl- oder Cyclopropylring, der gegebenenfalls durch bis zu drei Substituenten R³ substituiert ist, bedeutet, wobei R³ jeweils unabhängig Halogen, Hydroxyl, Nitro, Amino, C1-C3-Alkyl, C1-C3-Halogenalkyl, C1-C3-Alkoxy, C1-C3-Halogenalkoxy, C2-C6-Alkoxycarbonyl, C 2-C7-Alkylcarbonyloxy, C1-C3-Alkylamino oder Di-(C1-C3-alkyl)amino bedeutet.

14. Herbizide Zusammensetzung, die eine Verbindung wie in einem der Ansprüche 1 bis 13 definiert gemeinsam mit mindestens einem landwirtschaftlich unbedenklichen Hilfsmittel oder Verdünnungsmittel umfasst.

15. Zusammensetzung nach Anspruch 14, die zusätzlich zu der Verbindung der Formel (I) ein weiteres Herbizid umfasst.

16. Zusammensetzung nach Anspruch 14 oder 15, die einen Safener umfasst.

17. Verwendung einer wie in einem der Ansprüche 1 bis 13 definierten Verbindung oder einer wie in einem der Ansprüche 14 bis 16 definierten Zusammensetzung als Herbizid.

18. Verfahren zum Bekämpfen von Unkräutern in Nutzpflanzenkulturen, wobei man eine wie in einem der Ansprüche 1 bis 13 definierte Verbindung oder eine wie in einem der Ansprüche 14 bis 16 beanspruchte Zusammensetzung auf die Unkräuter oder den Standort der Unkräuter oder die Nutzpflanzen appliziert.

## Revendications

1. Composé répondant à la formule (I) : 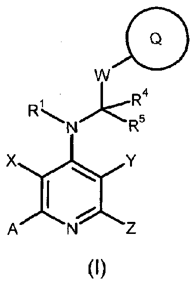 ou un sel ou N-oxyde de celui-ci,
dans laquelle
A est halogène, C1-C6 alkyle éventuellement substitué par de 1 à 3 groupements R², C1-C6 halogénoalkyle éventuellement substitué par de 1 à 3 groupements R², C2-C6 alcényle éventuellement substitué par de 1 à 3 groupements R², C3-C8 cycloalkyle éventuellement substitué par de 1 à 3 groupements R², C1-C6 alkylthio éventuellement substitué par de 1 à 3 groupements R², C6-C10 aryle éventuellement substitué par de 1 à 3 groupements R³, un groupement hétéroaryle mono- ou bicyclique ayant de 3 à 10 atomes de cycle et au moins un atome de cycle qui est l'azote, l'oxygène ou le soufre éventuellement substitué par de 1 à 3 groupements R³ ;
R¹ est hydrogène, C1-C6 alkyle éventuellement substitué par de 1 à 3 groupements R², C2-C6 alcényle éventuellement substitué par de 1 à 3 groupements R², C2-C6 alcynyle éventuellement substitué par de 1 à 3 groupements R², C3-C8 cycloalkyle éventuellement substitué par de 1 à 3 groupements R², C1-C6 acyle éventuellement substitué par de 1 à 3 groupements R², C6-C10 aryle éventuellement substitué par de 1 à 3 groupements R³, un groupement hétéroaryle mono- ou bicyclique ayant de 3 à 10 atomes de cycle et au moins un atome de cycle qui est l'azote, l'oxygène ou le soufre éventuellement substitué par de 1 à 3 groupements R³, C1-C6 alkylsulfonyle éventuellement substitué par de 1 à 3 groupements R², C2-C7 alcoxycarbonyle éventuellement substitué par de 1 à 3 groupements R², aminocarbonyle, C1-C6 alkylaminocarbonyle éventuellement substitué par de 1 à 3 groupements R², di-C1-C6 alkylaminocarbonyle éventuellement substitué par de 1 à 3 groupements R² ;
R⁴ est hydrogène, cyano, nitro, C1-C6 alkyle éventuellement substitué par de 1 à 3 groupements R², C1-C6 halogénoalkyle éventuellement substitué par de 1 à 3 groupements R², C3-C6 cycloalkyle éventuellement substitué par de 1 à 3 groupements R², C2-C6 alcényle éventuellement substitué par de 1 à 3 groupements R², C6-C10 aryle éventuellement substitué par de 1 à 3 groupements R³, un groupement hétéroaryle mono- ou bicyclique ayant de 3 à 10 atomes de cycle et au moins un atome de cycle qui est l'azote, l'oxygène ou le soufre éventuellement substitué par de 1 à 3 groupements R³, C1-C6 acyle éventuellement substitué par de 1 à 3 groupements R², C1-C6 alcoxycarbonyle éventuellement substitué par de 1 à 3 groupements R², carboxy, aminocarbonyle, C1-C6 alkylaminocarbonyle éventuellement substitué par de 1 à 3 groupements R², di-C1-C6 alkylaminocarbonyle éventuellement substitué par de 1 à 3 groupements R², ou di-C1-C6 alkylphosphonyle éventuellement substitué par de 1 à 3 groupements R² ;
R⁵ est hydrogène, C1-C6 alkyle éventuellement substitué par de 1 à 3 groupements R², ou C1-C6 halogénoalkyle ;
W est une liaison directe ou un groupement de liaison de formule -(C1-C3 alkylène)ₛ-L-(C1-C3 alkylène)ₜ- où chaque groupement alkylène est éventuellement substitué par jusqu'à 3 groupements R², s et t peuvent valoir chacun indépendamment 0 ou 1, et L est une liaison directe simple, double ou triple, -S(O)ᵤ- où u vaut 0, 1 ou 2, -N (R¹¹) - où R¹¹ est H ou C1-C6 alkyle, -0- ou -C(O)O- ;
Q est un noyau de 3-10 chaînons contenant éventuellement jusqu'à 4 hétéroatomes choisis indépendamment parmi 0, N ou S, le noyau étant éventuellement substitué par jusqu'à trois substituants R³ ;
X est hydrogène, halogène, cyano, nitro, hydroxyle, C1-C6 alkyle éventuellement substitué par de 1 à 3 groupements R², C1-C6 halogénoalkyle éventuellement substitué par de 1 à 3 groupements R², C2-C6 alcényle éventuellement substitué par de 1 à 3 groupements R², C2-C6 alcynyle éventuellement substitué par de 1 à 3 groupements R², C1-C6 halogénoalcoxy, C3-C8 cycloalkyle éventuellement substitué par de 1 à 3 groupements R², C6-C10 aryle éventuellement substitué par de 1 à 3 groupements R³, un groupement hétéroaryle mono- ou bicyclique ayant de 3 à 10 atomes de cycle et au moins un atome de cycle qui est l'azote, l'oxygène ou le soufre éventuellement substitué par de 1 à 3 groupements R³, C1-C6 alcoxy éventuellement substitué par de 1 à 3 groupements R², amino, C1-C6 alkylamino éventuellement substitué par de 1 à 3 groupements R², di(C1-C6 alkyl)amino éventuellement substitué par de 1 à 3 groupements R², C1-C6 alkylthio éventuellement substitué par de 1 à 3 groupements R², C1-C6 alkylsulfinyle éventuellement substitué par de 1 à 3 groupements R², C1-C6 alkylsulfonyle éventuellement substitué par de 1 à 3 groupements R², di(C1-C6 alkyl)phosphonyle, tri(C1-C6 alkyl)silyle ;
Y est halogène, cyano, nitro, hydroxyle, C1-C6 alkyle éventuellement substitué par de 1 à 3 groupements R², C2-C6 alcényle éventuellement substitué par de 1 à 3 groupements R², C2-C6 alcynyle éventuellement substitué par de 1 à 3 groupements R², C1-C6 halogénoalkyle éventuellement substitué par de 1 à 3 groupements R², C1-C6 halogénoalcoxy, C1-C6 alcoxy éventuellement substitué par de 1 à 3 groupements R², C3-C8 cycloalkyle éventuellement substitué par de 1 à 3 groupements R², C3-C8 cycloalcoxy éventuellement substitué par de 1 à 3 groupements R², C6-C10 aryle éventuellement substitué par de 1 à 3 groupements R³, un groupement hétéroaryle mono- ou bicyclique ayant de 3 à 10 atomes de cycle et au moins un atome de cycle qui est l'azote, l'oxygène ou le soufre éventuellement substitué par de 1 à 3 groupements R³, amino, C1-C6 alkylamino, di(C1-C6 alkyl)amino, C1-C6 alkylthio, C1-C6 alkylsulfinyle, C1-C6 alkylsulfonyle, di(C1-C6 alkyl)phosphonyle ou tri(C1-C6 alkyl)silyle ;
Z est C(O)R⁶ C(S)R⁶, ou C(=NR⁷)R⁸ ;
chaque R² est indépendamment halogène, hydroxyle, amino, C1-C3 alkylamino, di(C1-C3)alkylamino, carboxy, cyano, C1-C3 alkyle, C1-C3 halogénoalkyle, C3-C6 cycloalkyle, C1-C3 alcoxy, C1-C3 halogénoalcoxy, C1-C3 alkylthio, C1-C3 alkylsulfonyle, C2-C6 carboxyalkyle, carboxy, C2-C6 alcoxycarbonyle, C2-C7 alkylcarbonyloxy, phényle, ou phénoxy ;
chaque R³ est indépendamment halogène, hydroxyle, nitro, amino, thiol, cyano, C1-C3 alkyle, C1-C3 halogénoalkyle, C1-C3 alcoxy, C1-C3 halogénoalcoxy, C1-C3 alkylthio, C1-C3 halogénoalkylthio, C2-C6 carboxyalkyle, C2-C6 alcoxycarbonyle, C2-C7 alkylcarbonyloxy, phényle, phénoxy, C1-C3 alkylamino, ou di(C1-C3 alkyl)amino ;
R⁶ est hydrogène, hydroxyle, C1-C10 alcoxy éventuellement substitué par C1-C6 alcoxy ou phényle, C3-C8 cycloalcoxy éventuellement substitué par C1-C6 alcoxy ou phényle, C1-C6 halogénoalcoxy, C2-C6 alcényloxy, C1-C6 alkylthio, amino, C1-C6 alkylamino, ou di(C1-C6 alkyl)amino ;
R⁷ est hydrogène, C1-C6 alkyle, C1-C6 alcoxy, C3-C8 cycloalcoxy, amino, C1-C6 alkylamino, ou di(C1-C6 alkyl)amino ;
R⁸ est hydrogène, C1-C6 alcoxy, C3-C8 cycloalcoxy, C1-C6 alkylthio, amino, C1-C6 alkylamino, ou di(C1-C6 alkyl)amino.

2. Composé selon la revendication 1, dans lequel A est halogène, phényle éventuellement substitué par de 1 à 3 groupements R³, ou C3-C6 cycloalkyle éventuellement substitué par de 1 à 3 groupements R².

3. Composé selon la revendication 1 ou 2, dans lequel R¹ est hydrogène ou C1-C6 alkyle.

4. Composé selon l'une quelconque des revendications précédentes, dans lequel X est hydrogène, halogène, C1-C6 alkyle, C1-C6 halogénoalkyle, C1-C3 alcoxy(C1-C6)alkyle, ou C3-C6 cycloalkyle.

5. Composé selon l'une quelconque des revendications précédentes, dans lequel Y est halogène, C1-C3 alkyle, C1-C3 halogénoalkyle, C1-C2 alcoxy(C1-C2)alkyle, cyclopropyle, C2-C4 alcényle, ou C2-C4 alcynyle.

6. Composé selon l'une quelconque des revendications précédentes, dans lequel R⁴ est hydrogène, C1-C6 alkyle, C1-C6 halogénoalkyle, C1-C6 hydroxyalkyle, C1-C3 alcoxy(C1-C6)alkyle, C2-C6 alcoxycarbonyle ou carboxyle.

7. Composé selon l'une quelconque des revendications précédentes, dans lequel R⁵ est hydrogène ou C1-C6 alkyle.

8. Composé selon l'une quelconque des revendications précédentes, dans lequel W est une liaison directe ou un groupement méthylène.

9. Composé selon l'une quelconque des revendications précédentes, dans lequel Q est phényle, pyridyle, furyle, thiophényle, oxazolyle, thiazolyle, chacun étant éventuellement substitué par 1 ou 2 groupements R³, ou C3-C8 cycloalkyle éventuellement substitué par 1 ou 2 groupements R³ où
chaque R³ est indépendamment halogène, nitro, cyano, C1-C2 alkyle, C1-C2 halogénoalkyle, C1-C2 alcoxy, C1-C2 halogénoalcoxy ou C2-C3 alcoxycarbonyle, amino ou di(C1-C2alkyl)amino.

10. Composé selon l'une quelconque des revendications précédentes, dans lequel X est hydrogène, halogène, C1-C2 alkyle, C1-C2 halogénoalkyle, C1-C2 alcoxy(C1-C2)alkyle, ou C3-C6 cycloalkyle.

11. Composé selon l'une quelconque des revendications précédentes, dans lequel Y est halogène, C1-C2 alkyle, C1-C2 halogénoalkyle, C1-C2 alcoxy(C1-C2)alkyle, ou C2-4 alcényle.

12. Composé selon l'une quelconque des revendications précédentes, dans lequel Z est C(O)R ⁶ où R⁶ est hydroxyle, C1-C6 alcoxy, phényl(C1-C2)alcoxy, ou (C1-C3)alcoxy(C1-C6)alcoxy.

13. Composé selon la revendication 1, dans lequel A est halogène, R¹ est hydrogène, X est hydrogène ou halogène, Y est halogène, méthyle ou vinyle, Z est C(O)R⁶, où R⁶ est hydroxyle ou C1-C6 alcoxy, R⁴ et R⁵ sont tous deux hydrogène, W est une liaison directe et Q est un cycle phényle, furanyle, pyridyle ou cyclopropyle éventuellement substitué par jusqu'à trois substituants R³, où chaque R³ est indépendamment halogène, hydroxyle, nitro, amino, C1-C3 alkyle, C1-C3 halogénoalkyle, C1-C3 alcoxy, C1-C3 halogénoalcoxy, C2-C6 alcoxycarbonyle, C2-C7 alkylcarbonyloxy, C1-C3 alkylamino, ou di(C1-C3 alkyl)amino.

14. Composition herbicide comprenant un composé tel que défini selon l'une quelconque des revendications 1 à 13, conjointement avec au moins un adjuvant ou diluant acceptable sur le plan agricole.

15. Composition selon la revendication 14, comprenant un autre herbicide en plus du composé de formule (I).

16. Composition selon la revendication 14 ou 15, comprenant un agent phytoprotecteur.

17. Utilisation d'un composé tel que défini selon l'une quelconque des revendications 1 à 13, ou d'une composition telle que définie selon l'une quelconque des revendications 14 à 16, comme herbicide.

18. Méthode de contrôle d'adventices dans des cultures de plantes utiles, comprenant l'application auxdites adventices, ou au lieu où lesdites adventices se développent, ou auxdites plantes de culture utiles, d'un composé tel que défini selon l'une quelconque des revendications 1 à 13, ou d'une composition telle que définie selon l'une quelconque des revendications 14 à 16.
